# EUROPEAN PATENT APPLICATION

(11) **EP 4 177 263 A1**
(43) Date of publication of application: **10.05.2023**
(21) Application number: 21836893.4
(22) Date of filing: 06.07.2021
(51) Int. Cl.: C07K 14/605, A61K 38/26, A61P 3/10, A61P 3/06, A61P 3/04, C07K 1/00

(54) **NOVEL POLYPEPTIDE AND THERAPEUTIC USE THEREOF**

(30) Priority: 06.07.2020 CN 202010643549
(71) Applicant: Vitalixir (Beijing) Co., Ltd, Beijing 102200 (CN)
(72) Inventor: HAN, Jie, Beijing 102200 (CN); SHEN, Zhichao, Beijing 102200 (CN)
(74) Representative: Elzaburu S.L.P.
(86) International application number: PCT/CN2021/104805
(87) International publication number: WO 2022/007805

(57) **Abstract**

The invention relates to the field of polypeptide chemistry, in particular, the invention relates to a polypeptide compound and its use in medicine.

## Description

### TECHNICAL FIELD

The invention relates to a peptide compound and its use in medical treatment.

### BACKGROUND OF THE INVENTION

Metabolic syndrome is a combination of multiple medical disorders that increase the risk of developing type 2 diabetes, atherosclerotic vascular disease, heart disease, and stroke. The medical parameters that define metabolic syndrome include diabetes mellitus, impaired glucose tolerance, increased fasting blood glucose, insulin resistance, central obesity, hypertension, elevated total cholesterol and triglycerides, elevated low-density cholesterol, and reduced high-density cholesterol. Diabetes includes type 1 diabetes, type 2 diabetes, and gestational diabetes. According to data from the World Health Organization (WHO), the prevalence of diabetes in developed countries is 5% to 10%. The number of people with diabetes in the world is expected to approximately double between 2000 and 2030. More than 50% of all individuals with diabetes in the world are undiagnosed, and there are more people with prediabetes than people with diabetes. For example, people with diabetes in China has reached 114 million, and another 500 million have impaired glucose tolerance and glucose metabolism, and on their way to become diabetic. More than half of the patients do not know they are sick. The great harm of diabetes mainly lies in serious complications and high mortality. The data show that diabetes is the leading cause of lower limb amputation and adult blindness.

Obesity is a medical condition and accumulation of excess body fat may have an adverse effect on health and life expectancy. Due to its prevalence in adults and children it has become one of the leading preventable causes of death in modern world. It increases the likelihood of various other diseases, including heart disease, type 2 diabetes, obstructive sleep apnea, certain types of cancer, and osteoarthritis. It is most commonly caused by a combination of excess food intake, reduced energy expenditure, and genetic susceptibility. With the same body mass index (BMI) Asians have higher visceral fat content than Caucasians and tend to have more severe insulin resistance than Caucasians. The insulin sensitivity of Asian type 2 diabetic patients with normal body weight decreases significantly in comparison to those non-diabetic people. Excess fat causes insulin resistance and β-cell damage, destroying blood glucose regulation. Various metabolic abnormalities associated with obesity significantly increase the risk of cardiovascular disease. According to clinical statistics, more than 70% of patients with type 2 diabetes are overweight. Therefore, reducing the weight and body fat of diabetic patients is an important approach to effectively control or even reverse diabetes progression. Existing small molecule weight-loss drugs have very serious side effects. Glucagon-like peptide-1 (GLP-1) receptor agonists can control blood glucose by promoting insulin secretion, increasing insulin sensitivity, and reducing the release of glucagon. Therefore, GLP-1 drugs are suitable for the treatment of metabolic diseases, especially diabetes. GLP-1 receptor agonists (e.g. exenatide, liraglutide) have shown weight-reducing effects in animal experiments and clinical trials with relatively mild side effects. Liraglutide has been approved for the treatment of diabetes and obesity in the United States, becoming the only drug for these two indications.

However, existing GLP-1 drugs have gastrointestinal side effects. These side effects affect the patient's medication compliance and reduce drug usage as well as user base. According to physiological mechanisms of GLP-1 receptors GLP-1 drugs have a slow effect on weight loss. Clinical treatment of obesity requires the use of higher doses than diabetes treatment, which leads to more severe gastrointestinal side effects. Most patients lose an average of less than 5% body weight, and their body weight rebounds significantly after stopping the medication.

Therefore, there is a clinical need for drugs that can lower blood glucose, decrease blood lipids and body fat content, and reduce body weight simultaneously.

People with diabetes are at higher risk of developing cardiovascular disease. Therefore, people with diabetes need to strictly control blood lipid. Clinical studies have shown that long-term use of statins may increase the risk of users getting diabetes. Statin and fibrate drugs have obvious side effects, and they are intolerant to many patients. There is no therapeutic drug with ideal curative effect for non-alcoholic fatty liver disease. The peptide compounds of the present invention can not only lower blood glucose but also significantly reduce triglyceride and total cholesterol, especially low-density lipoproteins (LDL). They are expected to remedy hyperglycemia and hyperlipidemia at the same time and are expected to have obvious benefits to the user's cardiovascular health. The peptide compounds of the present invention are more suitable for diabetic patients or pre-diabetic people than statins. The peptide compounds of the present invention provide new options for the treatment of these diseases. The peptides of the present invention are suitable for various diseases caused by abnormal lipid metabolism, including hyperlipidemia and non-alcoholic fatty liver disease. The peptides of the present invention can also be used for diseases such as hypertension, arteriosclerosis, coronary heart disease, peripheral artery disease, stroke, or any combination of these diseases.

The majority of patients with diabetes are middle-aged and elderly people who need to take multiple drugs. This inevitably involves compatibility and matching between drugs. In addition to drug interactions and more toxic side effects resulting from multiple drugs, different drugs have different pharmacokinetics and different dose frequency, which also increases the distress for patients. Therefore, the peptides of the present invention are not only beneficial to enhance the curative effects, mitigate toxicity and side effects, but are also convenient for the patients, thereby improving the therapeutic effects.

The peptides of the present invention can also be used for diabetes treatment like GLP-1 drugs. As these peptides reduce body weight and body fat mass and improve insulin resistance, they should not only have an excellent effect on hyperglycemia but also be particularly effective for a large percentage of diabetic patients who develop diabetes as a result of their excess weight. Although many overweight or obese people are not medically classified as diabetic they have pre-diabetic symptoms such as glucose intolerance and postprandial hyperglycemia. The peptides of the present invention are also suitable for people with pre-diabetes.

### SUMMARY OF THE INVENTION

In one aspect, the invention relates to a peptide compound of formula (I) or a pharmaceutically acceptable salt or solvate thereof,

Y-aib-E-G-T-F-T-S-D-X1-S-X11-X2-X12-E-X3-X4-A-X5-X6-X13-F-X7-X8-W-L-X9-X14-G-X10 (I)

Wherein X1 is an amino acid residue selected from L, K, C, or Y, or formula(II), or formula(III); X2 is an amino acid residue selected from Q, A, aib, or Y; X3 is an amino acid residue selected from E, C, or K, or formula(II), or formula(III); X4 is an amino acid residue selected from E, C, or K, or formula(II), or formula(III); X5 is an amino acid residue selected from V or A; X6 is an amino acid residue selected from K, R, C, or Q, or formula(II), or formula(III); X7 is an amino acid residue selected from I or V; X8 is an amino acid residue selected from E, Q, N, K, C, or A, or formula(II), or formula(III); X9 is an amino acid residue selected from I, L, C, or K, or formula(II), or formula(III); X10 is absent or is GPSSGAPPP, GPPSGAPPP,GPSSGKPPP, GPSSGEPPP, GPSSaibAPPP, GPSSGAPP, GPSSGAP, GPSSGA, GPSSG, GPSS, GPS, GP, or G; X11 is an amino acid residue of I, C, S, or K, or formula(II), or formula(III); X12 is an amino acid residue of L, C, or K, or formula(II), or formula(III); X13 is an amino acid residue of L, E, or D; X14 is an amino acid residue of A, C, K, or R, or formula(II), or formula(III); said structure of formula(II) or formula(III) is as follows: 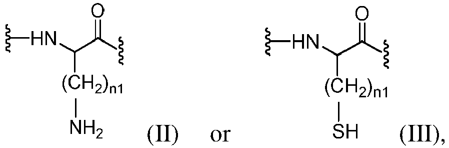
wherein the wavy line indicates the attachment point to an adjacent group, n1 is an integer of 1-7,
optionally, an amino acid residue of formula(II) containing a side chain amino group is modified at its side chain amino group with a long-acting group, an amino acid residue of formula(III) containing a side chain mercapto group is modified at its side chain mercapto group with a long-acting group; preferably, formula(II) is a lysine residue , formula(III) is a cysteine residue.

Optionally, one or two amino acids selected from S or an amino acid whose side chain contains an amino group or a mercapto group are added to the C-terminus of X10, and the carboxyl group of the C-terminal amino acid is optionally amidated to a C-terminal amide, said amino acid residue containing an amino group or a mercapto group has the structure of formula(II) or (III); when formula (II) or (III) is a C-terminal amino acid, its carboxyl part is COOH or CONH₂, preferably, formula(II) is a lysine residue, and formula (III) is a cysteine residue ,
preferably, the long-acting group has the structure of formula (IV):

   01-02-03-04-05-06-07-08- (IV),
wherein O1 represents the structure of formula (V) or (VI): 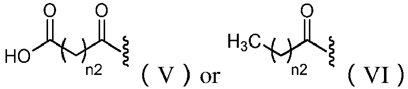
wherein n2 is an integer of 6-24, preferably 10-24, further preferably 16-22;
wherein the wavy line indicates the attachment point to the amino group of an adjacent group, and O2-O3-O4-O5-O6-O7-O8- represents a linker, wherein each of O2 to O8 is independently selected from any one of the following amino acid residues or long chain structures: α-Glu, γ-Glu, α-Asp, β-Asp, α-hGlu, δ-hGlu, Gly, Ala, β-Ala, GABA or PEG2, or one or more residues of O2 to O8 are absent, provided that at least two residues of O2 to O8 are present, preferably, O2 to O8 contain at least one negatively charged moiety.

A peptide compound according to any one of the preceding aspects, wherein O2-O3-O4-O5-O6-O7-O8- represents a linker selected from the group consisting of yGlu-PEG2-γGlu-,γGlu-PEG2-2×γGlu-,γGlu-PEG2-,γGlu-2×PEG2-,γGlu-3×PEG2-,γGlu-PEG2-γGlu-PEG2-,yGlu-2×PEG2-yGlu-, γGlu-2×PEG2-2×γGlu-,2×γGlu-,2×γGlu-PEG2-,2×γGlu-PEG2-γGlu-,2×γGlu-PEG2-γGlu-PEG2-, 2×γGlu-2×PEG2-,2×γGlu-2PEG2-γGlu-,2×γGlu-2×PEG2-2×γGlu-.

A peptide compound according to any one of the preceding aspects, in some embodiments, wherein O2-O3-O4-O5-O6-O7-O8- represents a linker selected from the group consisting of yGlu-PEG2-, γGlu-2×PEG2-, γGlu-3×PEG2-, O1 represents the structure of formula (V) or (VI).

A peptide compound according to any one of the preceding aspects, in some embodiments, wherein O2-O3-O4-O5-O6-O7-O8- represents a linker selected from the group consisting of yGlu-PEG2-, γGlu-2×PEG2-, γGlu-3×PEG2-, O1 represents the structure of formula (V).

A peptide compound according to any one of the preceding aspects, in some embodiments, wherein O2-O3-O4-O5-O6-O7-O8- represents a linker selected from the group consisting of γGlu-2×PEG2-, and γGlu-3×PEG2 -, O1 represents the structure of formula (V).

A peptide compound according to any one of the preceding aspects, in some embodiments, wherein O2-O3-O4-O5-O6-O7-O8- represents the linker γGlu-2×PEG2-, and O1 represents the structure of formula (V).

A peptide compound according to any one of the preceding aspects, in some embodiments, wherein O2-O3-O4-O5-O6-O7-O8- represents the linker γGlu-2×PEG2-, and O1 represents the structure of formula (V), wherein n2 is an integer of 16-22.

In some embodiments, the side chain amino group of lysine is linked to a long-acting group, in the present patent application, said molecular structure is abbreviated as K(x18) and has the structure of formula(VII):

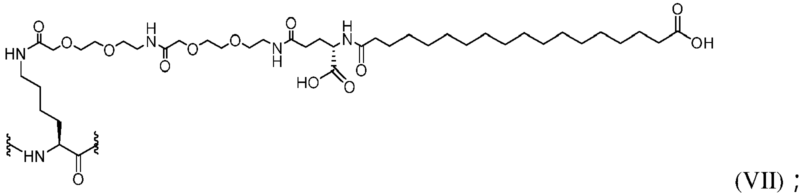

In some embodiments, the side chain amino group of lysine is linked to a long-acting group, in the present patent application, said molecular structure is abbreviated as K(x20) and has the structure of formula(VIII): 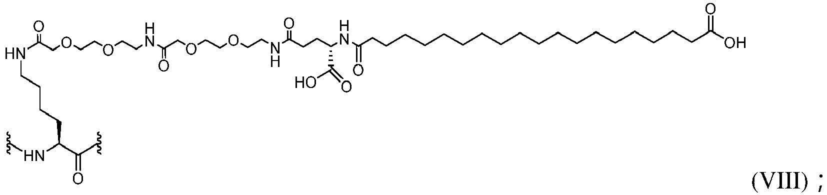 wherein the wavy line indicates the attachment point to an adjacent amino acid residue.

Optionally, an amino acid added to the C-terminus of X10 contains a side chain thiol group which is modified with a long-acting group of formula (IV), optionally, a reactive group capable of reacting with said thiol group to form a covalent bond can be added between the side chain thiol group and a long-acting group as needed.

In some embodiments, the connection relationship between the side chain thiol group of side chain thiol-containing amino acid and the long-acting group is: side chain thiol group of the amino acid containing a side chain thiol group-thiol reactive group--optional linking group L- long-acting group.

In some embodiments, the side chain thiol group of an amino acid containing a side chain thiol group reacts with a Michael acceptor (e.g. maleimide or vinyl sulfone) or a thiol reactive group (e.g. iodoacetic acid or bromoacetic acid) ) to be connected to one end of an linking group L after the reaction, preferably, the other end of said linking group L is further connected to a long-acting group of formula (IV) via a covalent bond.

In some embodiments, the linking group L is a long chain formed by -(CH₂)ₙ₃- and - (CH₂CH₂O)ₙ₄- which are arranged and combined according to structural requirements, and are linked together by covalent bonds; or one end or both ends of -(CH₂)ₙ₃-, -(CH₂CH₂O)ₙ₄-optionally contain an amino group or a carboxyl group, and are connected by amide bonds to form a long chain, for example, the linking group L is selected from -NH-(CH₂)ₙ₅-(CH₂CH₂O)ₙ₆-(CH₂)ₙ₇-, -NH-(CH₂)ₙ₅-(CH₂CH₂O)ₙ₆-(CH₂)ₙ₇-NH-, -NH-(CH₂)ₙ₅-(CH₂CH₂O)ₙ₆-(CH₂)ₙ₇-CO-, -NH-(CH₂)ₙ₅-(CH₂CH₂O)ₙ₆-(CH₂)ₙ₇-NHCO-(CH₂)ₙ₈-, -NH-(CH₂)ₙ₅-(CH₂CH₂O)ₙ₆-(CH₂)ₙ₇-NHCO-(CH₂)ₙ₈-NH-, or any combination thereof, wherein n3, n4, n5, n6, n7, n8 are each an integer of 0 to 10, for example, 0, 1, 2, 3, 4, 5, 6, 7, 8, 9, 10.

In some embodiments, L is -NH-CH₂-(CH₂CH₂O)₃-(CH₂)₃-NH-.

In some embodiments, non-limiting illustrative examples of connection of a Michael acceptor or thiol reactive group to a linking group L include

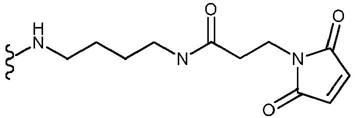

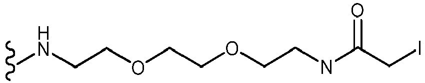

The structures after reaction of the above exemplary Michael acceptor or thiol reactive group with the side chain thiol group of an amino acid containing a side chain thiol group are as follows: 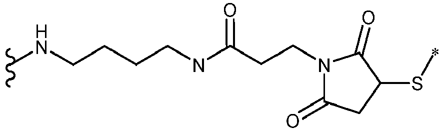 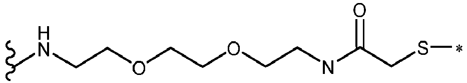 wherein the wavy line is the point of attachment to an long-acting group of formula (IV), for example, to O8. ^{∗} is the connection point between the side chain thiol group of an amino acid containing a side chain thiol group and the other parts of the amino acid.

Optionally, any one of the amino acids in the peptide fragments represented by X10 may be substituted with an amino acid containing an amino group or a thiol group on its side chain, and said amino acid has the structure of formula (II) or formula (III). Optionally, an amino acid containing a side chain amino group is modified at its side chain amino group with a long-acting group, preferably, the long-acting group has the structure of formula (IV); optionally, an amino acid containing a side chain thiol group is modified at its side chain thiol group with a long-acting group, preferably, the long-acting group has the structure of formula (IV). Optionally, a reactive group capable of reacting with the thiol group to form a covalent bond can be added between the side chain thiol group and an long-acting group as needed.

In some embodiments, the side chain thiol group of cysteine is linked to a long-acting group, in the present patent application, said molecular structure is abbreviated as C(x18) and has the structure of formula(IX):

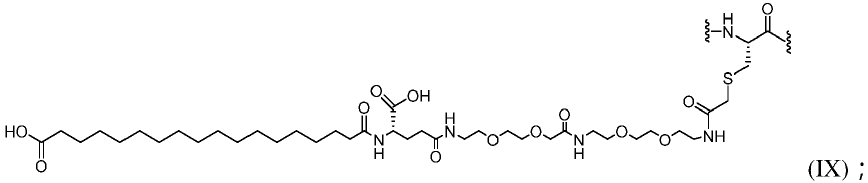

In some embodiments, the side chain thiol group of cysteine is linked to a long-acting group, in the present patent application, said molecular structure is abbreviated as C(x20) and has the structure of formula(X): 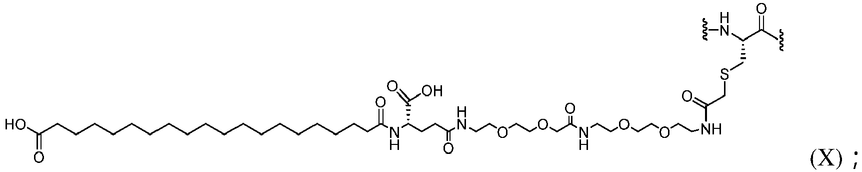 wherein the wavy line represents the connection point to the adjacent structure.

Optionally, the peptide fragments GPPSGAPPP, GPSSGKPPP, GPSSGEPPP, GPSSaibAPPP represented by X10 can be reduced by 1, 2, 3, 4, 5, 6, 7, 8 amino acid residues from the C-terminus of the fragments to the N-terminus of the fragments.

According to a peptide compound of any of the preceding aspects, in some embodiments, X1 is L. According to a peptide compound of any of the preceding aspects, in some embodiments, X1 is Y. According to a peptide compound of any of the preceding aspects, in some embodiments, X1 is K. According to a peptide compound of any of the preceding aspects, in some embodiments, X1 is C.

According to a peptide compound of any of the preceding aspects, in some embodiments, X2 is Q. According to a peptide compound of any of the preceding aspects, in some embodiments, X2 is Y. According to a peptide compound of any of the preceding aspects, in some embodiments, X2 is A. According to a peptide compound of any of the preceding aspects, in some embodiments, X2 is Aib.

According to a peptide compound of any of the preceding aspects, in some embodiments, X3 is E. According to a peptide compound of any of the preceding aspects, in some embodiments, X3 is K. According to a peptide compound of any of the preceding aspects, in some embodiments, X3 is C.

According to a peptide compound of any of the preceding aspects, in some embodiments, X4 is E. According to a peptide compound of any of the preceding aspects, in some embodiments, X4 is K. According to a peptide compound of any of the preceding aspects, in some embodiments, X4 is C.

According to a peptide compound of any of the preceding aspects, in some embodiments, X5 is V. According to a peptide compound of any of the preceding aspects, in some embodiments, X5 is A.

According to a peptide compound of any of the preceding aspects, in some embodiments, X6 is K. According to a peptide compound of any of the preceding aspects, in some embodiments, X6 is R. According to a peptide compound of any of the preceding aspects, in some embodiments, X6 is Q. According to a peptide compound of any of the preceding aspects, in some embodiments, X6 is C.

According to a peptide compound of any of the preceding aspects, in some embodiments, X7 is I. According to a peptide compound of any of the preceding aspects, in some embodiments, X7 is V.

According to a peptide compound of any of the preceding aspects, in some embodiments, X8 is E. According to a peptide compound of any of the preceding aspects, in some embodiments, X8 is N. According to a peptide compound of any of the preceding aspects, in some embodiments, X8 is Q. According to a peptide compound of any of the preceding aspects, in some embodiments, X8 is A. According to a peptide compound of any of the preceding aspects, in some embodiments, X8 is K. According to a peptide compound of any of the preceding aspects, in some embodiments, X8 is C.

According to a peptide compound of any of the preceding aspects, in some embodiments, X9 is I. According to a peptide compound of any of the preceding aspects, in some embodiments, X9 is L. According to a peptide compound of any of the preceding aspects, in some embodiments, X9 is K. According to a peptide compound of any of the preceding aspects, in some embodiments, X9 is Aib. According to a peptide compound of any of the preceding aspects, in some embodiments, X9 is V.

According to a peptide compound of any of the preceding aspects, in some embodiments, X10 is absent. According to a peptide compound of any of the preceding aspects, in some embodiments, X10 is GPSSGAPPPSK. According to a peptide compound of any of the preceding aspects, in some embodiments, X10 is GPSSGAPPPSC. According to a peptide compound of any of the preceding aspects, in some embodiments, X10 is GPSSGAPPPS. According to a peptide compound of any of the preceding aspects, in some embodiments, X10 is GPSSGAPPPK. According to a peptide compound of any of the preceding aspects, in some embodiments, X10 is GPSSGAPPPC. According to a peptide compound of any of the preceding aspects, in some embodiments, X10 is GPSSGAPPP. According to a peptide compound of any of the preceding aspects, in some embodiments, X10 is GPSSGAPP. According to a peptide compound of any of the preceding aspects, in some embodiments, X10 is GPSSGAP. According to a peptide compound of any of the preceding aspects, in some embodiments, X10 is GPSSGA. According to a peptide compound of any of the preceding aspects, in some embodiments, X10 is GPSSG. According to a peptide compound of any of the preceding aspects, in some embodiments, X10 is GPSS. According to a peptide compound of any of the preceding aspects, in some embodiments, X10 is GPS. According to a peptide compound of any of the preceding aspects, in some embodiments, X10 is GP. According to a peptide compound of any of the preceding aspects, in some embodiments, X10 is G.

According to a peptide compound of any of the preceding aspects, in some embodiments, X1 is L, and X10 is GPSSGAPPPS. According to a peptide compound of any of the preceding aspects, in some embodiments, X1 is L, and X10 is GPSSGAPPPK. According to a peptide compound of any of the preceding aspects, in some embodiments, X1 is L, and X10 is GPSSGAPPPC. According to a peptide compound of any of the preceding aspects, in some embodiments, X1 is L, and X10 is GPSSGAPPP. According to a peptide compound of any of the preceding aspects, in some embodiments, X1 is L, and X10 is GPSSGAPP. According to a peptide compound of any of the preceding aspects, in some embodiments, X1 is L, and X10 is GPSSGAP. According to a peptide compound of any of the preceding aspects, in some embodiments, X1 is L, and X10 is GPSSGA. According to a peptide compound of any of the preceding aspects, in some embodiments, X1 is L, and X10 is GPSSG. According to a peptide compound of any of the preceding aspects, in some embodiments, X1 is L, and X10 is GPSS. According to a peptide compound of any of the preceding aspects, in some embodiments, X1 is L, and X10 is GPS. According to a peptide compound of any of the preceding aspects, in some embodiments, X1 is L, and X10 is GP. According to a peptide compound of any of the preceding aspects, in some embodiments, X1 is L, and X10 is G.

According to a peptide compound of any of the preceding aspects, in some embodiments, X2 is Q and X10 is GPSSGAPPPS.

According to a peptide compound of any of the preceding aspects, in some embodiments, X2 is Q and X10 is GPSSGAPPPK.

According to a peptide compound of any of the preceding aspects, in some embodiments, X2 is Q and X10 is GPSSGAPPPC.

According to a peptide compound of any of the preceding aspects, in some embodiments, X2 is A and X10 is GPSSGAPPPS.

According to a peptide compound of any of the preceding aspects, in some embodiments, X2 is A and X10 is GPSSGAPPPK.

According to a peptide compound of any of the preceding aspects, in some embodiments, X2 is A and X10 is GPSSGAPPPC.

According to a peptide compound of any of the preceding aspects, in some embodiments, X2 is aib and X10 is GPSSGAPPPS.

According to a peptide compound of any of the preceding aspects, in some embodiments, X2 is aib and X10 is GPSSGAPPPK.

According to a peptide compound of any of the preceding aspects, in some embodiments, X2 is aib and X10 is GPSSGAPPPC.

According to a peptide compound of any of the preceding aspects, in some embodiments, X2 is Q and X10 is GPSSGAPPP.

According to a peptide compound of any of the preceding aspects, in some embodiments, X2 is Q and X10 is GPSSGAPP.

According to a peptide compound of any of the preceding aspects, in some embodiments, X2 is Q and X10 is GPSSGAP.

According to a peptide compound of any of the preceding aspects, in some embodiments, X2 is Q and X10 is GPSSGA.

According to a peptide compound of any of the preceding aspects, in some embodiments, X2 is Q and X10 is GPSSG.

According to a peptide compound of any of the preceding aspects, in some embodiments, X2 is Q and X10 is GPSS.

According to a peptide compound of any of the preceding aspects, in some embodiments, X2 is Q and X10 is GPS.

According to a peptide compound of any of the preceding aspects, in some embodiments, X2 is Q and X10 is GP.

According to a peptide compound of any of the preceding aspects, in some embodiments, X2 is Q and X10 is G.

According to a peptide compound of any of the preceding aspects, in some embodiments, X2 is A and X10 is GPSSGAPPP.

According to a peptide compound of any of the preceding aspects, in some embodiments, X2 is A and X10 is GPSSGAPP.

According to a peptide compound of any of the preceding aspects, in some embodiments, X2 is A and X10 is GPSSGAP.

According to a peptide compound of any of the preceding aspects, in some embodiments, X2 is A and X10 is GPSSGA.

According to a peptide compound of any of the preceding aspects, in some embodiments, X2 is A and X10 is GPSSG.

According to a peptide compound of any of the preceding aspects, in some embodiments, X2 is A and X10 is GPSS.

According to a peptide compound of any of the preceding aspects, in some embodiments, X2 is A and X10 is GPS.

According to a peptide compound of any of the preceding aspects, in some embodiments, X2 is A and X10 is GP.

According to a peptide compound of any of the preceding aspects, in some embodiments, X2 is A and X10 is G.

According to a peptide compound of any of the preceding aspects, in some embodiments, X2 is A and X9 is K.

According to a peptide compound of any of the preceding aspects, in some embodiments, X2 is aib and X10 is GPSSGAPPP.

According to a peptide compound of any of the preceding aspects, in some embodiments, X2 is aib and X10 is GPSSGAPP.

According to a peptide compound of any of the preceding aspects, in some embodiments, X2 is aib and X10 is GPSSGAP.

According to a peptide compound of any of the preceding aspects, in some embodiments, X2 is aib and X10 is GPSSGA.

According to a peptide compound of any of the preceding aspects, in some embodiments, X2 is aib and X10 is GPSSG.

According to a peptide compound of any of the preceding aspects, in some embodiments, X2 is aib and X10 is GPSS.

According to a peptide compound of any of the preceding aspects, in some embodiments, X2 is aib and X10 is GPS.

According to a peptide compound of any of the preceding aspects, in some embodiments, X2 is aib and X10 is GP.

According to a peptide compound of any of the preceding aspects, in some embodiments, X2 is aib and X10 is G.

According to a peptide compound of any of the preceding aspects, in some embodiments, X2 is Aib and X9 is K.

According to a peptide compound of any of the preceding aspects, in some embodiments, X2 is Y and X10 is G.

According to a peptide compound of any of the preceding aspects, in some embodiments, X1 is L and X2 is Q.

According to a peptide compound of any of the preceding aspects, in some embodiments, X1 is L and X2 is A.

According to a peptide compound of any of the preceding aspects, in some embodiments, X1 is L and X2 is aib.

According to a peptide compound of any of the preceding aspects, in some embodiments, X1 is L, X2 is Q, and X10 is GPSSGAPPPS. According to a peptide compound of any of the preceding aspects, in some embodiments, X1 is L, X2 is Q, and X10 is GPSSGAPPPK. According to a peptide compound of any of the preceding aspects, in some embodiments, X1 is L, X2 is Q, and X10 is GPSSGAPPPC. According to a peptide compound of any of the preceding aspects, in some embodiments, X1 is L, X2 is Q, and X10 is GPSSGAPPP. According to a peptide compound of any of the preceding aspects, in some embodiments, X1 is L, X2 is Q, and X10 is GPSSGAPP. According to a peptide compound of any of the preceding aspects, in some embodiments, X1 is L, X2 is Q, and X10 is GPSSGAP. According to a peptide compound of any of the preceding aspects, in some embodiments, X1 is L, X2 is Q, and X10 is GPSSGA. According to a peptide compound of any of the preceding aspects, in some embodiments, X1 is L, X2 is Q, and X10 is GPSSG. According to a peptide compound of any of the preceding aspects, in some embodiments, X1 is L, X2 is Q, and X10 is GPSS. According to a peptide compound of any of the preceding aspects, in some embodiments, X1 is L, X2 is Q, and X10 is GPS. According to a peptide compound of any of the preceding aspects, in some embodiments, X1 is L, X2 is Q, and X10 is GP. According to a peptide compound of any of the preceding aspects, in some embodiments, X1 is L, X2 is Q, and X10 is G.

According to a peptide compound of any of the preceding aspects, in some embodiments, X1 is L, X2 is A, and X10 is GPSSGAPPPS. According to a peptide compound of any of the preceding aspects, in some embodiments, X1 is L, X2 is A, and X10 is GPSSGAPPPK. According to a peptide compound of any of the preceding aspects, in some embodiments, X1 is L, X2 is A, and X10 is GPSSGAPPPC. According to a peptide compound of any of the preceding aspects, in some embodiments, X1 is L, X2 is A, and X10 is GPSSGAPPP. According to a peptide compound of any of the preceding aspects, in some embodiments, X1 is L, X2 is A, and X10 is GPSSGAPP. According to a peptide compound of any of the preceding aspects, in some embodiments, X1 is L, X2 is A, and X10 is GPSSGAP. According to a peptide compound of any of the preceding aspects, in some embodiments, X1 is L, X2 is A, and X10 is GPSSGA. According to a peptide compound of any of the preceding aspects, in some embodiments, X1 is L, X2 is A, and X10 is GPSSG. According to a peptide compound of any of the preceding aspects, in some embodiments, X1 is L, X2 is A, and X10 is GPSS. According to a peptide compound of any of the preceding aspects, in some embodiments, X1 is L, X2 is A, and X10 is GPS. According to a peptide compound of any of the preceding aspects, in some embodiments, X1 is L, X2 is A, and X10 is GP. According to a peptide compound of any of the preceding aspects, in some embodiments, X1 is L, X2 is A, and X10 is G.

According to a peptide compound of any of the preceding aspects, in some embodiments, X1 is L, X2 is aib, and X10 is GPSSGAPPPS. According to a peptide compound of any of the preceding aspects, in some embodiments, X1 is L, X2 is aib, and X10 is GPSSGAPPPK. According to a peptide compound of any of the preceding aspects, in some embodiments, X1 is L, X2 is aib, and X10 is GPSSGAPPPC. According to a peptide compound of any of the preceding aspects, in some embodiments, X1 is L, X2 is aib, and X10 is GPSSGAPPP. According to a peptide compound of any of the preceding aspects, in some embodiments, X1 is L, X2 is aib, and X10 is GPSSGAPP. According to a peptide compound of any of the preceding aspects, in some embodiments, X1 is L, X2 is aib, and X10 is GPSSGAP. According to a peptide compound of any of the preceding aspects, in some embodiments, X1 is L, X2 is aib, and X10 is GPSSGA. According to a peptide compound of any of the preceding aspects, in some embodiments, X1 is L, X2 is aib, and X10 is GPSSG. According to a peptide compound of any of the preceding aspects, in some embodiments, X1 is L, X2 is aib, and X10 is GPSS. According to a peptide compound of any of the preceding aspects, in some embodiments, X1 is L, X2 is aib, and X10 is GPS. According to a peptide compound of any of the preceding aspects, in some embodiments, X1 is L, X2 is aib, and X10 is GP. According to a peptide compound of any of the preceding aspects, in some embodiments, X1 is L, X2 is aib, and X10 is G.

According to a peptide compound of any of the preceding aspects, in some embodiments, X1 is L, X2 is A, and X9 is K.

According to a peptide compound of any of the preceding aspects, in some embodiments, X1 is L, X2 is aib, and X9 is K.

According to a peptide compound of any of the preceding aspects, in some embodiments, X1 is L, X2 is Y, and X9 is K.

According to a peptide compound of any of the preceding aspects, in some embodiments, X1 is L, X2 is Q, X5 is V, and X10 is GPSSGAPPPS. According to a peptide compound of any of the preceding aspects, in some embodiments, X1 is L, X2 is Q, X5 is V, and X10 is GPSSGAPPPK. According to a peptide compound of any of the preceding aspects, in some embodiments, X1 is L, X2 is Q, X5 is V, and X10 is GPSSGAPPPC.

According to a peptide compound of any of the preceding aspects, in some embodiments, X1 is L, X2 is A, X5 is V, and X10 is GPSSGAPPPS. According to a peptide compound of any of the preceding aspects, in some embodiments, X1 is L, X2 is A, X5 is V, and X10 is GPSSGAPPPK. According to a peptide compound of any of the preceding aspects, in some embodiments, X1 is L, X2 is A, X5 is V, and X10 is GPSSGAPPPC.

According to a peptide compound of any of the preceding aspects, in some embodiments, X1 is L, X2 is aib, X5 is V, and X10 is GPSSGAPPPS. According to a peptide compound of any of the preceding aspects, in some embodiments, X1 is L, X2 is aib, X5 is V, and X10 is GPSSGAPPPK. According to a peptide compound of any of the preceding aspects, in some embodiments, X1 is L, X2 is aib, X5 is V, and X10 is GPSSGAPPPC.

According to a peptide compound of any of the preceding aspects, in some embodiments, X1 is L, X2 is Q, X3 is K, X5 is V, and X10 is GPSSGAPPPS. According to a peptide compound of any of the preceding aspects, in some embodiments, X1 is L, X2 is Q, X3 is K, X5 is V, and X10 is GPSSGAPPPK. According to a peptide compound of any of the preceding aspects, in some embodiments, X1 is L, X2 is Q, X3 is K, X5 is V, and X10 is GPSSGAPPPC.

According to a peptide compound of any of the preceding aspects, in some embodiments, X1 is L, X2 is A, X3 is K, X5 is V, and X10 is GPSSGAPPPS. According to a peptide compound of any of the preceding aspects, in some embodiments, X1 is L, X2 is A, X3 is K, X5 is V, and X10 is GPSSGAPPPK. According to a peptide compound of any of the preceding aspects, in some embodiments, X1 is L, X2 is A, X3 is K, X5 is V, and X10 is GPSSGAPPPC.

According to a peptide compound of any of the preceding aspects, in some embodiments, X1 is L, X2 is aib, X3 is K, X5 is V, and X10 is GPSSGAPPPS. According to a peptide compound of any of the preceding aspects, in some embodiments, X1 is L, X2 is aib, X3 is K, X5 is V, and X10 is GPSSGAPPPK. According to a peptide compound of any of the preceding aspects, in some embodiments, X1 is L, X2 is aib, X3 is K, X5 is V, and X10 is GPSSGAPPPC.

According to a peptide compound of any of the preceding aspects, in some embodiments, X1 is L, X2 is Q, X3 is K, X5 is V, X7 is I, and X10 is GPSSGAPPPS. According to a peptide compound of any of the preceding aspects, in some embodiments, X1 is L, X2 is Q, X3 is K, X5 is V, X7 is I, and X10 is GPSSGAPPPK. According to a peptide compound of any of the preceding aspects, in some embodiments, X1 is L, X2 is Q, X3 is K, X5 is V, X7 is I, and X10 is GPSSGAPPPC.

According to a peptide compound of any of the preceding aspects, in some embodiments, X1 is L, X2 is A, X3 is K, X5 is V, X7 is I, and X10 is GPSSGAPPPS. According to a peptide compound of any of the preceding aspects, in some embodiments, X1 is L, X2 is A, X3 is K, X5 is V, X7 is I, and X10 is GPSSGAPPPK. According to a peptide compound of any of the preceding aspects, in some embodiments, X1 is L, X2 is A, X3 is K, X5 is V, X7 is I, and X10 is GPSSGAPPPC.

According to a peptide compound of any of the preceding aspects, in some embodiments, X1 is L, X2 is aib, X3 is K, X5 is V, X7 is I, and X10 is GPSSGAPPPS. According to a peptide compound of any of the preceding aspects, in some embodiments, X1 is L, X2 is aib, X3 is K, X5 is V, X7 is I, and X10 is GPSSGAPPPK. According to a peptide compound of any of the preceding aspects, in some embodiments, X1 is L, X2 is aib, X3 is K, X5 is V, X7 is I, and X10 is GPSSGAPPPC.

According to a peptide compound of any of the preceding aspects, in some embodiments, X2 is A, X5 is V. According to a peptide compound of any of the preceding aspects, in some embodiments, X2 is aib, X5 is V. According to a peptide compound of any of the preceding aspects, in some embodiments, X1 is L, X2 is A, X5 is V. According to a peptide compound of any of the preceding aspects, in some embodiments, X1 is L, X2 is aib, X5 is V. According to a peptide compound of any of the preceding aspects, in some embodiments, X1 is L, X2 is A, X3 is K, X5 is V. According to a peptide compound of any of the preceding aspects, in some embodiments, X1 is L, X2 is aib, X3 is K, X5 is V.

According to a peptide compound of any of the preceding aspects, in some embodiments, X1 is L, X2 is A, X3 is K, X5 is V, X7 is I. According to a peptide compound of any of the preceding aspects, in some embodiments, X1 is L, X2 is aib, X3 is K, X5 is V, X7 is I.

According to a peptide compound of any of the preceding aspects, in some embodiments, X2 is Q, X9 is L. According to a peptide compound of any of the preceding aspects, in some embodiments, X2 is Q, X9 is L, and X10 is GPSSGAPPPS. According to a peptide compound of any of the preceding aspects, in some embodiments, X2 is Q, X9 is L, and X10 is GPSSGAPPPK. According to a peptide compound of any of the preceding aspects, in some embodiments, X2 is Q, X9 is L, and X10 is GPSSGAPPPC.

According to a peptide compound of any of the preceding aspects, in some embodiments, X2 is A, X9 is L. According to a peptide compound of any of the preceding aspects, in some embodiments, X2 is A, X9 is L, and X10 is GPSSGAPPPS. According to a peptide compound of any of the preceding aspects, in some embodiments, X2 is A, X9 is L, and X10 is GPSSGAPPPK. According to a peptide compound of any of the preceding aspects, in some embodiments, X2 is A, X9 is L, and X10 is GPSSGAPPPC.

According to a peptide compound of any of the preceding aspects, in some embodiments, X2 is aib, X9 is L. According to a peptide compound of any of the preceding aspects, in some embodiments, X2 is aib, X9 is L, and X10 is GPSSGAPPPS. According to a peptide compound of any of the preceding aspects, in some embodiments, X2 is aib, X9 is L, and X10 is GPSSGAPPPK. According to a peptide compound of any of the preceding aspects, in some embodiments, X2 is aib, X9 is L, and X10 is GPSSGAPPPC.

According to a peptide compound of any of the preceding aspects, in some embodiments, X1 is L, X2 is Q, X9 is L, and X10 is GPSSGAPPPS. According to a peptide compound of any of the preceding aspects, in some embodiments, X1 is L, X2 is Q, X9 is L, and X10 is GPSSGAPPPK. According to a peptide compound of any of the preceding aspects, in some embodiments, X1 is L, X2 is Q, X9 is L, and X10 is GPSSGAPPPC.

According to a peptide compound of any of the preceding aspects, in some embodiments, X1 is L, X2 is A, X9 is L, and X10 is GPSSGAPPPS. According to a peptide compound of any of the preceding aspects, in some embodiments, X1 is L, X2 is A, X9 is L, and X10 is GPSSGAPPPK. According to a peptide compound of any of the preceding aspects, in some embodiments, X1 is L, X2 is A, X9 is L, and X10 is GPSSGAPPPC.

According to a peptide compound of any of the preceding aspects, in some embodiments, X1 is L, X2 is aib, X9 is L, and X10 is GPSSGAPPPS. According to a peptide compound of any of the preceding aspects, in some embodiments, X1 is L, X2 is aib, X9 is L, and X10 is GPSSGAPPPK. According to a peptide compound of any of the preceding aspects, in some embodiments, X1 is L, X2 is aib, X9 is L, and X10 is GPSSGAPPPC.

According to a peptide compound of any of the preceding aspects, in some embodiments, X1 is L, X2 is Q, X5 is V, X9 is L, and X10 is GPSSGAPPPS. According to a peptide compound of any of the preceding aspects, in some embodiments, X1 is L, X2 is Q, X5 is V, X9 is L, and X10 is GPSSGAPPPK. According to a peptide compound of any of the preceding aspects, in some embodiments, X1 is L, X2 is Q, X5 is V, X9 is L, and X10 is GPSSGAPPPC.

According to a peptide compound of any of the preceding aspects, in some embodiments, X1 is L, X2 is A, X5 is V, X9 is L, and X10 is GPSSGAPPPS. According to a peptide compound of any of the preceding aspects, in some embodiments, X1 is L, X2 is A, X5 is V, X9 is L, and X10 is GPSSGAPPPK. According to a peptide compound of any of the preceding aspects, in some embodiments, X1 is L, X2 is A, X5 is V, X9 is L, and X10 is GPSSGAPPPC.

According to a peptide compound of any of the preceding aspects, in some embodiments, X1 is L, X2 is aib, X5 is V, X9 is L, and X10 is GPSSGAPPPS. According to a peptide compound of any of the preceding aspects, in some embodiments, X1 is L, X2 is aib, X5 is V, X9 is L, and X10 is GPSSGAPPPK. According to a peptide compound of any of the preceding aspects, in some embodiments, X1 is L, X2 is aib, X5 is V, X9 is L, and X10 is GPSSGAPPPC

According to a peptide compound of any of the preceding aspects, in some embodiments, X1 is L, X2 is Q, X3 is K, X5 is V, X9 is L, and X10 is GPSSGAPPPS. According to a peptide compound of any of the preceding aspects, in some embodiments, X1 is L, X2 is Q, X3 is K, X5 is V, X9 is L, and X10 is GPSSGAPPPK. According to a peptide compound of any of the preceding aspects, in some embodiments, X1 is L, X2 is Q, X3 is K, X5 is V, X9 is L, and X10 is GPSSGAPPPC.

According to a peptide compound of any of the preceding aspects, in some embodiments, X1 is L, X2 is A, X3 is K, X5 is V, X9 is L, and X10 is GPSSGAPPPS. According to a peptide compound of any of the preceding aspects, in some embodiments, X1 is L, X2 is A, X3 is K, X5 is V, X9 is L, and X10 is GPSSGAPPPK. According to a peptide compound of any of the preceding aspects, in some embodiments, X1 is L, X2 is A, X3 is K, X5 is V, X9 is L, and X10 is GPSSGAPPPC.

According to a peptide compound of any of the preceding aspects, in some embodiments, X1 is L, X2 is aib, X3 is K, X5 is V, X9 is L, and X10 is GPSSGAPPPS. According to a peptide compound of any of the preceding aspects, in some embodiments, X1 is L, X2 is aib, X3 is K, X5 is V, X9 is L, and X10 is GPSSGAPPPK. According to a peptide compound of any of the preceding aspects, in some embodiments, X1 is L, X2 is aib, X3 is K, X5 is V, X9 is L, and X10 is GPSSGAPPPC.

According to a peptide compound of any of the preceding aspects, in some embodiments, X1 is L, X2 is Q, X3 is K, X5 is V, X7 is I, X9 is L, and X10 is GPSSGAPPPS. According to a peptide compound of any of the preceding aspects, in some embodiments, X1 is L, X2 is Q, X3 is K, X5 is V, X7 is I, X9 is L, and X10 is GPSSGAPPPK. According to a peptide compound of any of the preceding aspects, in some embodiments, X1 is L, X2 is Q, X3 is K, X5 is V, X7 is I, X9 is L, and X10 is GPSSGAPPPC.

According to a peptide compound of any of the preceding aspects, in some embodiments, X1 is L, X2 is A, X3 is K, X5 is V, X7 is I, X9 is L, and X10 is GPSSGAPPPS. According to a peptide compound of any of the preceding aspects, in some embodiments, X1 is L, X2 is A, X3 is K, X5 is V, X7 is I, X9 is L, and X10 is GPSSGAPPPK. According to a peptide compound of any of the preceding aspects, in some embodiments, X1 is L, X2 is A, X3 is K, X5 is V, X7 is I, X9 is L, and X10 is GPSSGAPPPC.

According to a peptide compound of any of the preceding aspects, in some embodiments, X1 is L, X2 is aib, X3 is K, X5 is V, X7 is I, X9 is L, and X10 is GPSSGAPPPS. According to a peptide compound of any of the preceding aspects, in some embodiments, X1 is L, X2 is aib, X3 is K, X5 is V, X7 is I, X9 is L, and X10 is GPSSGAPPPK. According to a peptide compound of any of the preceding aspects, in some embodiments, X1 is L, X2 is aib, X3 is K, X5 is V, X7 is I, X9 is L, and X10 is GPSSGAPPPC.

According to a peptide compound of any of the preceding aspects, in some embodiments, X1 is L, X2 is Q, X3 is K, X5 is V, X7 is V, X9 is L, and X10 is GPSSGAPPPS. According to a peptide compound of any of the preceding aspects, in some embodiments, X1 is L, X2 is Q, X3 is K, X5 is V, X7 is V, X9 is L, and X10 is GPSSGAPPPK. According to a peptide compound of any of the preceding aspects, in some embodiments, X1 is L, X2 is Q, X3 is K, X5 is V, X7 is V, X9 is L, and X10 is GPSSGAPPPC.

According to a peptide compound of any of the preceding aspects, in some embodiments, X1 is L, X2 is A, X3 is K, X5 is V, X7 is V, X9 is L, and X10 is GPSSGAPPPS. According to a peptide compound of any of the preceding aspects, in some embodiments, X1 is L, X2 is A, X3 is K, X5 is V, X7 is V, X9 is L, and X10 is GPSSGAPPPK. According to a peptide compound of any of the preceding aspects, in some embodiments, X1 is L, X2 is A, X3 is K, X5 is V, X7 is V, X9 is L, and X10 is GPSSGAPPPC.

According to a peptide compound of any of the preceding aspects, in some embodiments, X1 is L, X2 is aib, X3 is K, X5 is V, X7 is I, X9 is L, and X10 is GPSSGAPPPS. According to a peptide compound of any of the preceding aspects, in some embodiments, X1 is L, X2 is aib, X3 is K, X5 is V, X7 is V, X9 is L, and X10 is GPSSGAPPPK. According to a peptide compound of any of the preceding aspects, in some embodiments, X1 is L, X2 is aib, X3 is K, X5 is V, X7 is V, X9 is L, and X10 is GPSSGAPPPC.

According to a peptide compound of any of the preceding aspects, in some embodiments, X1 is Y and X2 is Q. According to a peptide compound of any of the preceding aspects, in some embodiments, X1 is Y and X2 is A. According to a peptide compound of any of the preceding aspects, in some embodiments, X1 is Y and X2 is Aib. According to a peptide compound of any of the preceding aspects, in some embodiments, X1 is Y and X2 is Y.

According to a peptide compound of any of the preceding aspects, in some embodiments, X1 is Y, X3 is K, and X9 is L. According to a peptide compound of any of the preceding aspects, in some embodiments, X1 is Y, X2 is Y, X3 is K, and X9 is L. According to a peptide compound of any of the preceding aspects, in some embodiments, X1 is Y, X2 is Y, X3 is K, X5 is V, and X9 is L. According to a peptide compound of any of the preceding aspects, in some embodiments, X1 is Y, X2 is Y, X3 is K, X5 is V, X7 is V, and X9 is L. According to a peptide compound of any of the preceding aspects, in some embodiments, X1 is Y, X2 is Y, X3 is K, X5 is V, X7 is V, X8 is N, and X9 is L. According to a peptide compound of any of the preceding aspects, in some embodiments, X1 is Y, X2 is Y, X3 is K, X5 is V, X7 is V, X8 is N, and X9 is I. A peptide compound according to any of the preceding aspects, in some embodiments, X1 is Y, X2 is Y, X3 is K, X5 is V, X7 is I, X8 is E, and X9 is L.

According to a peptide compound of any of the preceding aspects, in some embodiments, X1 is L, and X9 is I.

According to a peptide compound of any of the preceding aspects, in some embodiments, X1 is L and X9 is L. According to a peptide compound of any of the preceding aspects, in some embodiments, X1 is L, X2 is Q, and X9 is L. According to a peptide compound of any of the preceding aspects, in some embodiments, X1 is L, X2 is Q, X5 is V, and X9 is L. According to a peptide compound of any of the preceding aspects, in some embodiments, X1 is L, X2 is Q, X3 is K, X5 is V, and X9 is L. According to a peptide compound of any of the preceding aspects, in some embodiments, X1 is L, X2 is Q, X3 is K, X5 is V, X7 is I, X8 is E, and X9 is L.

According to a peptide compound of any of the preceding aspects, in some embodiments, X1 is L and X2 is A. According to a peptide compound of any of the preceding aspects, in some embodiments, X1 is L, X2 is A, and X9 is L. According to a peptide compound of any of the preceding aspects, in some embodiments, X1 is L, X2 is A, X5 is V, and X9 is L. According to a peptide compound of any of the preceding aspects, in some embodiments, X1 is L, X2 is A, X3 is K, X5 is V, and X9 is L. According to a peptide compound of any of the preceding aspects, in some embodiments, X1 is L, X2 is A, X3 is K, X5 is V, X7 is I, X8 is E, and X9 is L.

According to a peptide compound of any of the preceding aspects, in some embodiments, X1 is L and X2 is aib. According to a peptide compound of any of the preceding aspects, in some embodiments, X1 is L, X2 is aib, and X9 is L. According to a peptide compound of any of the preceding aspects, in some embodiments, X1 is L, X2 is aib, X5 is V, and X9 is L. According to a peptide compound of any of the preceding aspects, in some embodiments, X1 is L, X2 is aib, X3 is K, X5 is V, and X9 is L. According to a peptide compound of any of the preceding aspects, in some embodiments, X1 is L, X2 is aib, X3 is K, X5 is V, X7 is I, X8 is E, and X9 is L.

According to a peptide compound of any of the preceding aspects, in some embodiments, X1 is L, X4 is E, X9 is I or L, X11 is I, X12 is L, X14 is A. According to a peptide compound of any of the preceding aspects, in some embodiments, X1 is L, X4 is E, X5 is V, X9 is I or L, X11 is I, X12 is L, X14 is A. According to a peptide compound of any of the preceding aspects, in some embodiments, X1 is L, X3 is K, X4 is E, X5 is V, X9 is I or L, X11 is I, X12 is L, X14 is A. According to a peptide compound of any of the preceding aspects, in some embodiments, X1 is L, X2 is A or aib, X3 is K, X4 is E, X5 is V, X9 is I or L, X11 is I, X12 is L, X14 is A. According to a peptide compound of any of the preceding aspects, in some embodiments, X1 is L, X2 is A or aib, X3 is K, X4 is E, X5 is V, X7 is I, X9 is I or L, X11 is I, X12 is L, X14 is A. According to a peptide compound of any of the preceding aspects, in some embodiments, X1 is L, X2 is A or aib, X3 is K, X4 is E, X5 is V, X6 is R, X7 is I, X9 is I or L, X11 is I, X12 is L, X13 is L, X14 is A. According to a peptide compound of any of the preceding aspects, in some embodiments, X1 is L, X2 is A or aib, X3 is K, X4 is E, X5 is V, X6 is R, X7 is I, X8 is E, X9 is I or L, X11 is I, X12 is L, X13 is L, X14 is A. According to a peptide compound of any of the preceding aspects, in some embodiments, X1 is L, X3 is K, X4 is E, X5 is V, X6 is R, X7 is I, X8 is E, X9 is I or L, X11 is I, X12 is L, X13 is L, X14 is A.

According to a peptide compound of any of the preceding aspects, in some embodiments, X1 is L, X2 is A or aib, X3 is K, X4 is E, X5 is V, X6 is R, X7 is I, X8 is E, X9 is I or L, X11 is I, X12 is L, X13 is L, X14 is A, X10 is selected from GPSSGAPPP, GPPSGAPPP, GPSSGKPPP, GPSSGEPPP, GPSSaibAPPP, GPSSGAPP, GPSSGAP, GPSSGA, GPSSG, GPSS, GPS, GP, or G, or one or two amino acids selected from S or an amino acid whose side chain cotains an amino group or a mercapto group are added to the C-terminus of X10, and the carboxyl group of the C-terminal amino acid is optionally amidated to a C-terminal amide, said amino acid residue containing an amino group or a mercapto group has the structure of formula(II) or (III); when formula(II) or formula(III) is a C-terminal amino acid, its carboxyl part is COOH or CONH₂, preferably, formula(II) is a lysine residue and formula (III) is a cysteine residue.

According to a peptide compound of any of the preceding aspects, in some embodiments, X1 is L, X2 is Y. According to a peptide compound of any of the preceding aspects, in some embodiments, X1 is L, X2 is Y, X5 is V. According to a peptide compound of any of the preceding aspects, in some embodiments, X1 is L, X2 is Y, X3 is K, X5 is V.

According to a peptide compound of any of the preceding aspects, in some embodiments, X1 is L, X2 is A, aib or Y, X4 is E, X11 is I or S. According to a peptide compound of any of the preceding aspects, in some embodiments, X1 is L, X2 is A, aib or Y, X4 is E, X11 is I. According to a peptide compound of any of the preceding aspects, in some embodiments, X1 is L, X2 is A, aib or Y, X3 is K, X4 is E, X11 is I.

According to a peptide compound of any of the preceding aspects, in some embodiments, X2 is Q, X9 is K, the sequence formed by X10 and C-terminus amino acids is not GPSSGAPPPS or GPSSGAPPPSK.

According to a peptide compound of any of the preceding aspects, in some embodiments, wherein at least one of X1, X3, X4, X6, X8, X9, X11, X12, and X14 is formula(II) or formula(III). According to a peptide compound of any of the preceding aspects, in some embodiments, wherein at least one of X1, X3, X4, X6, X8, X9, X11, X12, and X14 is formula(II) or formula(III), and at least one formula (II) or (III) has the side chain which is linked to a long-acting group or a modifying group. According to a peptide compound of any of the preceding aspects, in some embodiments, wherein at least one of X1, X4, X8, X11, X12, and X14 is formula(II) or formula(III).

According to a peptide compound of any of the preceding aspects, in some embodiments, wherein at least one of X1, X3, X4, X6, X8, X9, X11, X12, X14 is formula(II) or formula(III), and X10 and the sequence composed of X10 and C-terminal amino acids do not contain formula(II) or formula(III).

According to a peptide compound of any of the preceding aspects, in some embodiments, wherein at least one of X1, X3, X4, X6, X8, X9, X11, X12, and X14 is formula(II) or formula(III), and X10 and the sequence composed of X10 and C-terminal amino acids are not GPSSGAPPPK, GPSSGAPPPC, GPSSGAPPPSC, or GPSSGAPPPSK.

According to a peptide compound of any of the preceding aspects, in some embodiments, X2 is an amino acid residue selected from A, aib, or Y, X12 is formula(II) or formula(III).

According to a peptide compound of any of the preceding aspects, in some embodiments, X1 is formula(II) or formula(III ).

According to a peptide compound of any of the preceding aspects, in some embodiments, X1 is K(x18).

According to a peptide compound of any of the preceding aspects, in some embodiments, X1 is K(x20).

According to a peptide compound of any of the preceding aspects, in some embodiments, X3 is formula(II) or formula(III).

According to a peptide compound of any of the preceding aspects, in some embodiments, X3 is K(x18).

According to a peptide compound of any of the preceding aspects, in some embodiments, X3 is K(x20).

According to a peptide compound of any of the preceding aspects, in some embodiments, X4 is formula(II) or formula(III).

According to a peptide compound of any of the preceding aspects, in some embodiments, X4 is K(x18).

According to a peptide compound of any of the preceding aspects, in some embodiments, X4 is K(x20).

According to a peptide compound of any of the preceding aspects, in some embodiments, X6 is formula(II) or formula(III).

According to a peptide compound of any of the preceding aspects, in some embodiments, X6 is K(x18).

According to a peptide compound of any of the preceding aspects, in some embodiments, X6 is K(x20).

According to a peptide compound of any of the preceding aspects, in some embodiments, X8 is formula(II) or formula(III).

According to a peptide compound of any of the preceding aspects, in some embodiments, X8 is K(x18).

According to a peptide compound of any of the preceding aspects, in some embodiments, X8 is K(x20).

According to a peptide compound of any of the preceding aspects, in some embodiments, X9 is formula(II) or formula(III).

According to a peptide compound of any of the preceding aspects, in some embodiments, X9 is K(x18).

According to a peptide compound of any of the preceding aspects, in some embodiments, X9 is K(x20).

According to a peptide compound of any of the preceding aspects, in some embodiments, X11 is formula(II) or formula(III).

According to a peptide compound of any of the preceding aspects, in some embodiments, X11 is K(x18).

According to a peptide compound of any of the preceding aspects, in some embodiments, X11 is K(x20).

According to a peptide compound of any of the preceding aspects, in some embodiments, X12 is formula(II) or formula(III).

According to a peptide compound of any of the preceding aspects, in some embodiments, X12 is K(x18).

According to a peptide compound of any of the preceding aspects, in some embodiments, X12 is K(x20).

According to a peptide compound of any of the preceding aspects, in some embodiments, X14 is formula(II) or formula(III).

According to a peptide compound of any of the preceding aspects, in some embodiments, X14 is K(x18).

According to a peptide compound of any of the preceding aspects, in some embodiments, X14 is K(x20).

The peptide compounds according to any of any of the preceding aspects, wherein the peptide compounds are not peptide compounds described in PCT application number PCT/CN2020/070697.

The peptide compounds according to any of the preceding aspects, wherein the peptide compounds are selected from: 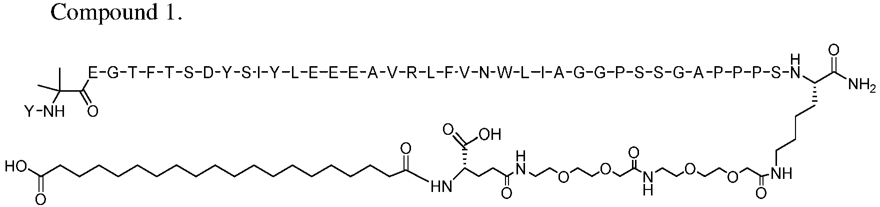 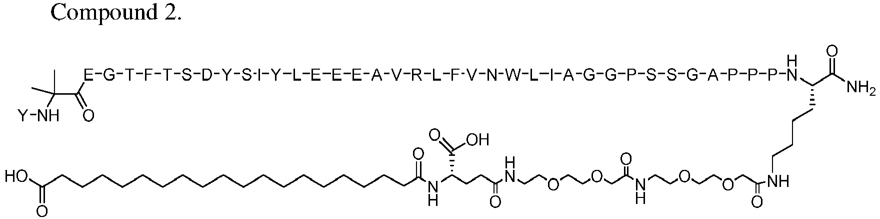 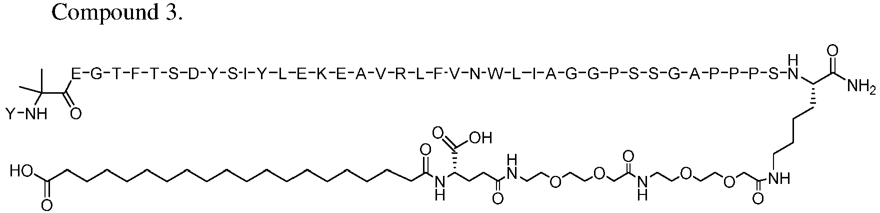 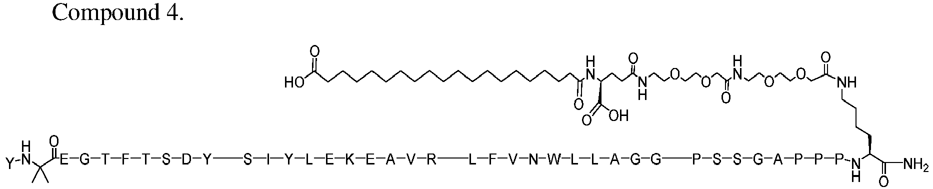 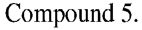 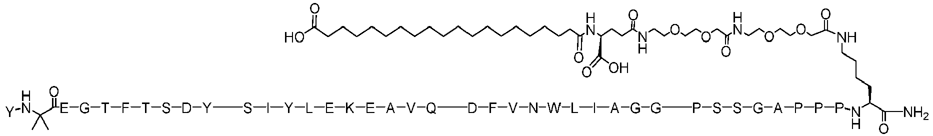 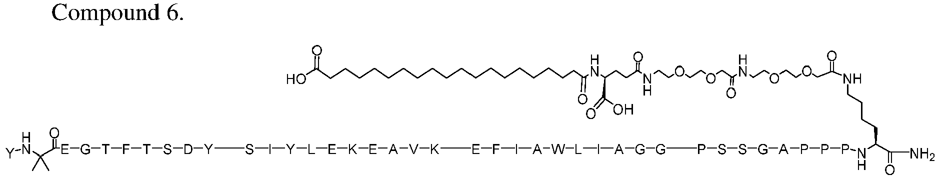 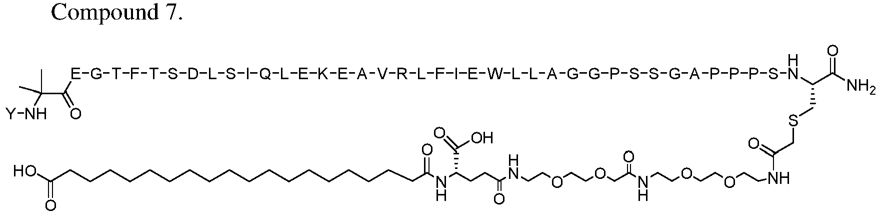 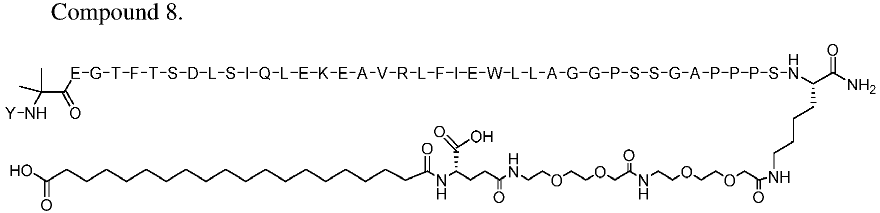 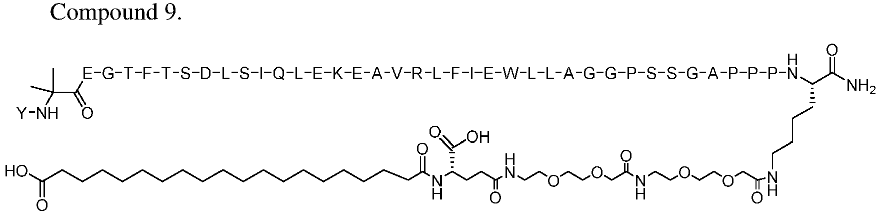 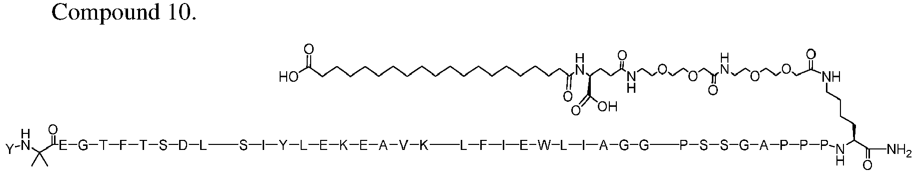 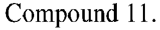 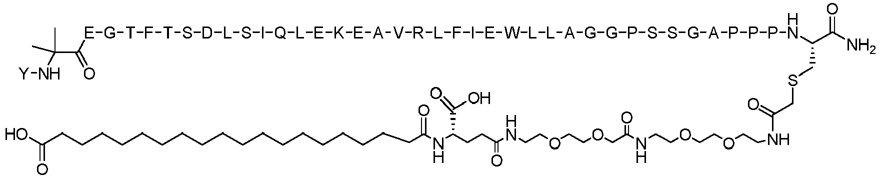 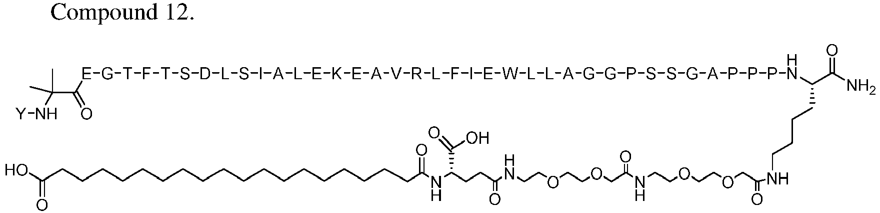 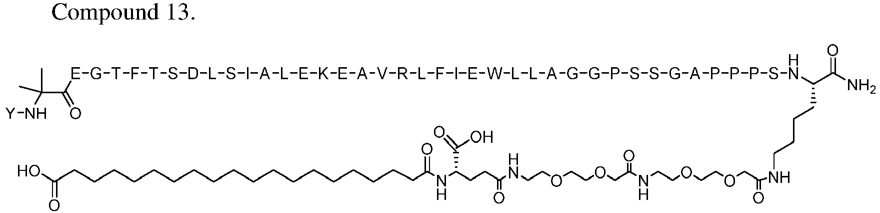 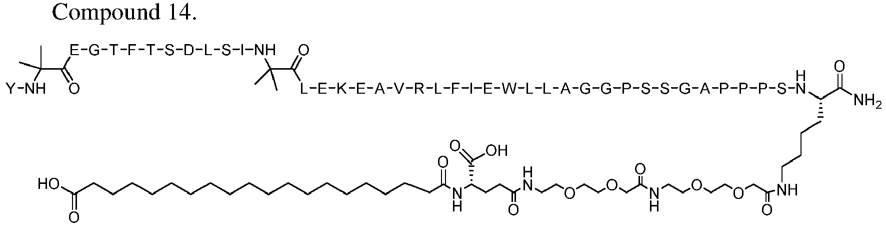 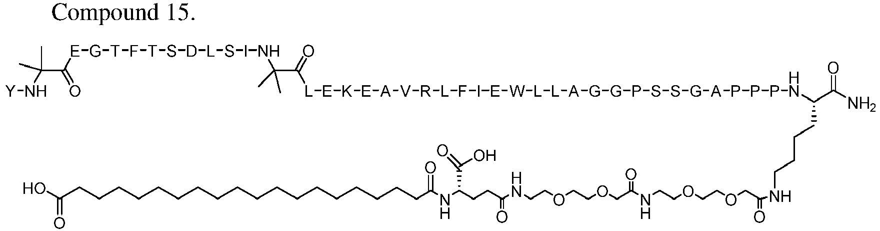 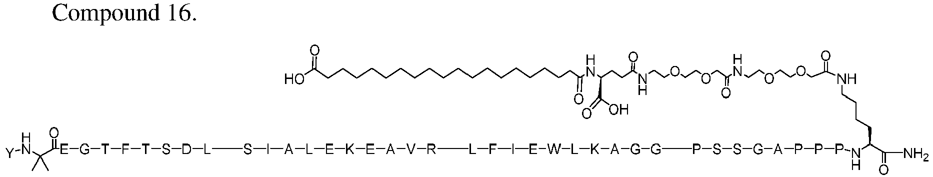 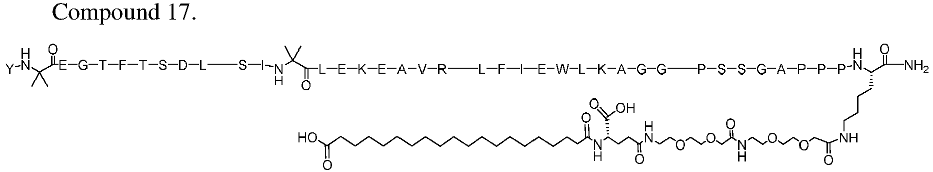 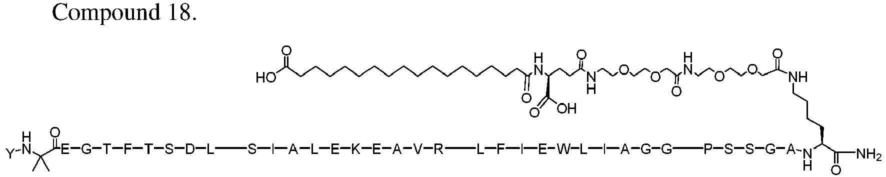 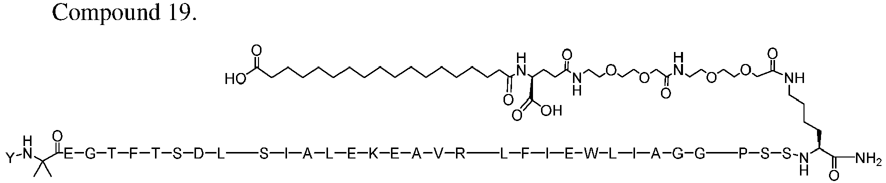 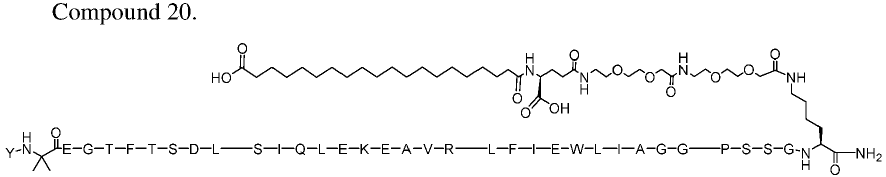 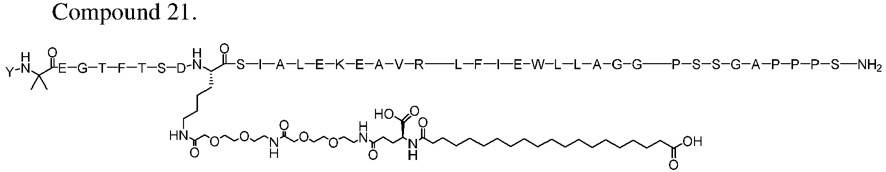 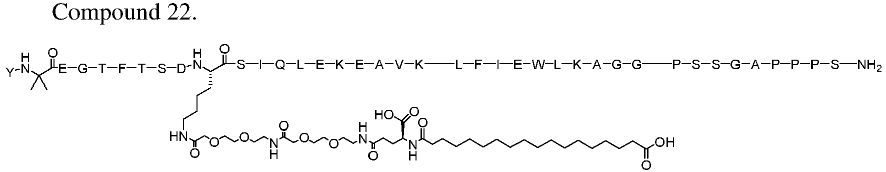 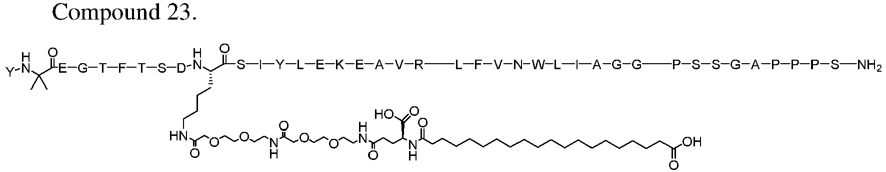 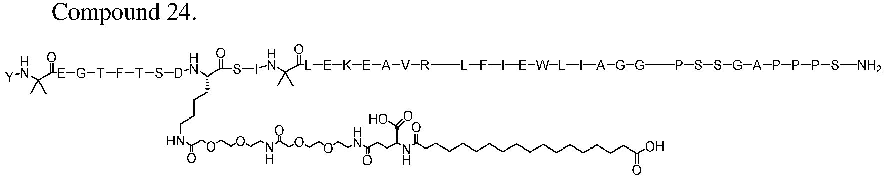 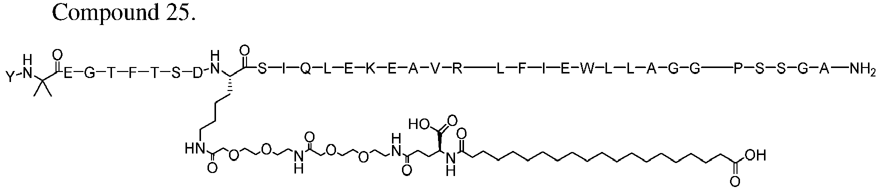 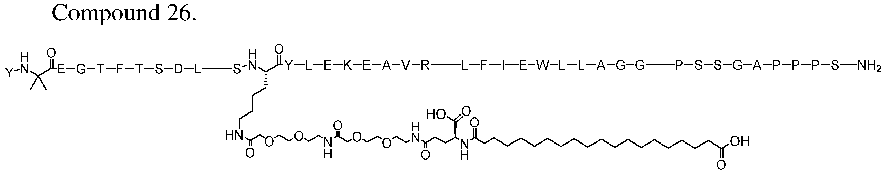 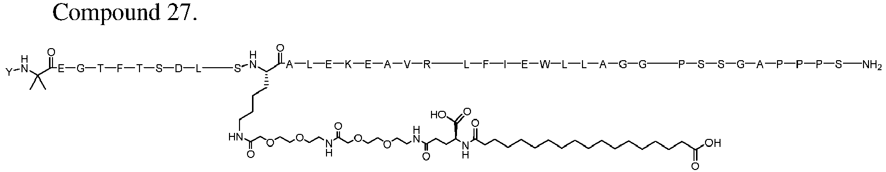 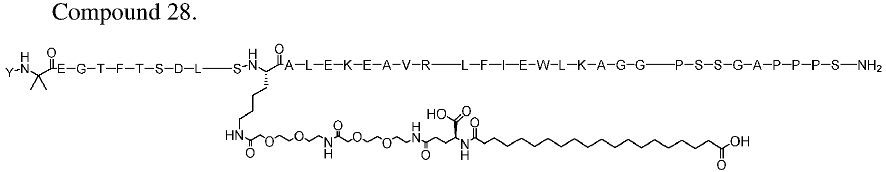 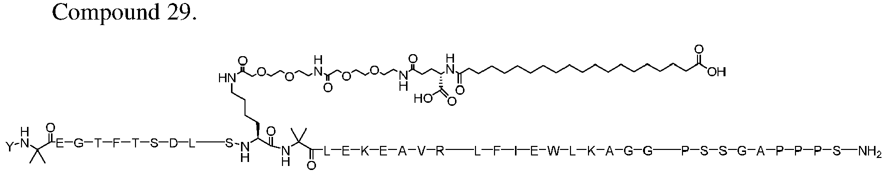 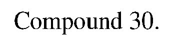 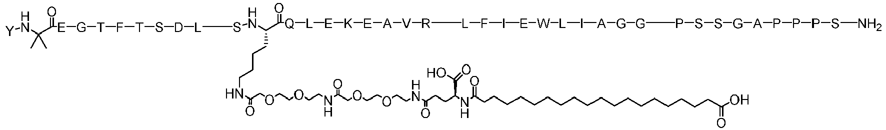 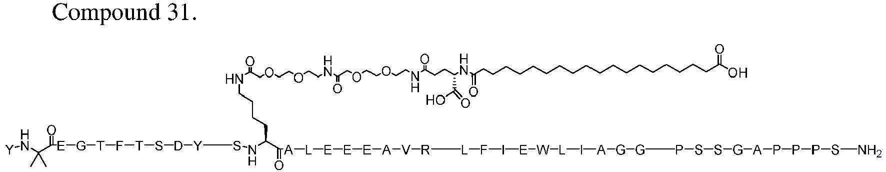 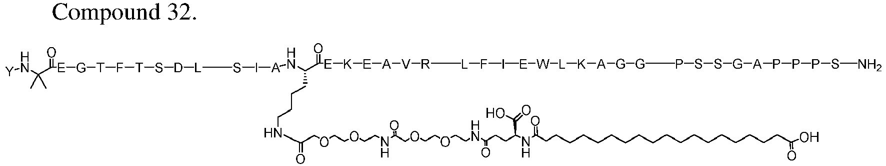 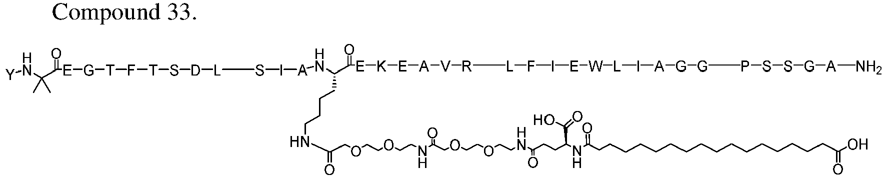 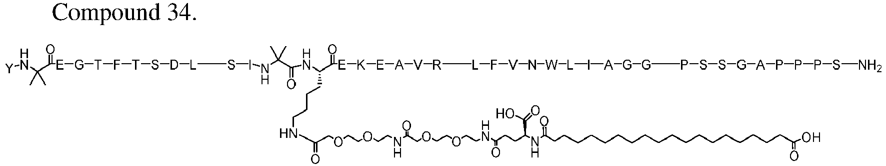 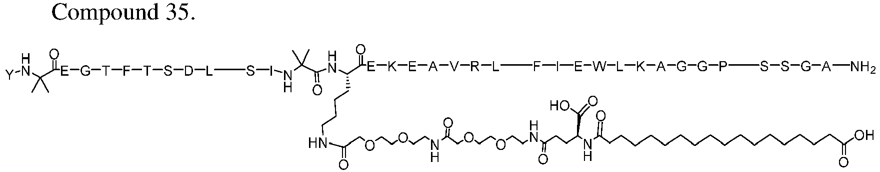 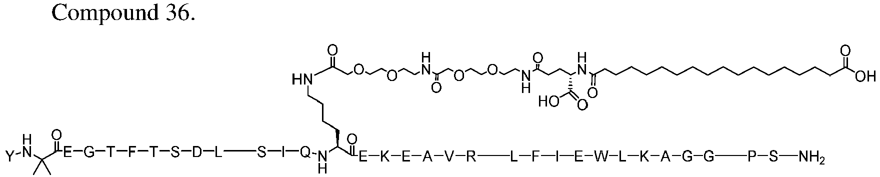 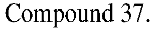 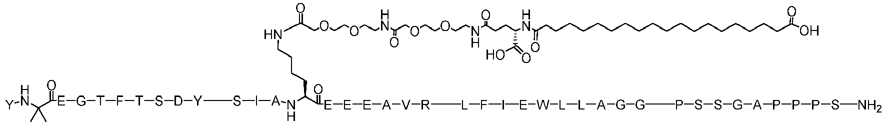 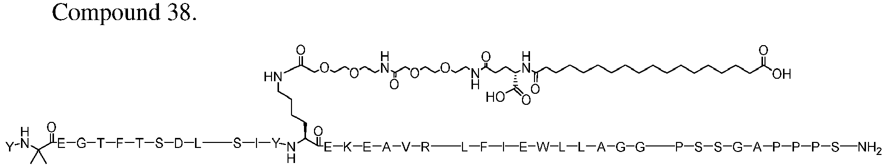 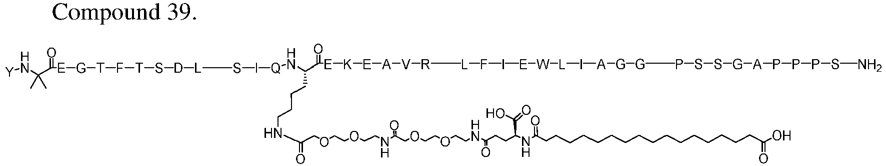 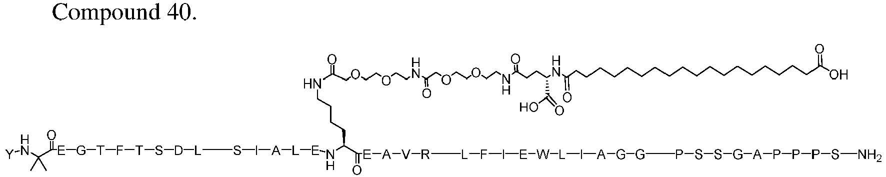 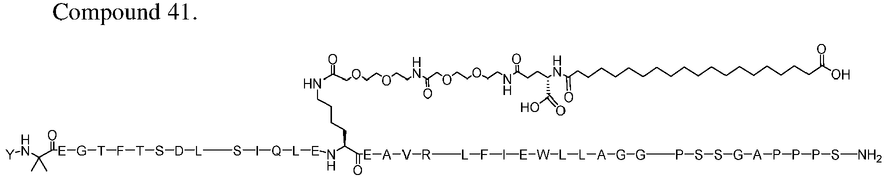 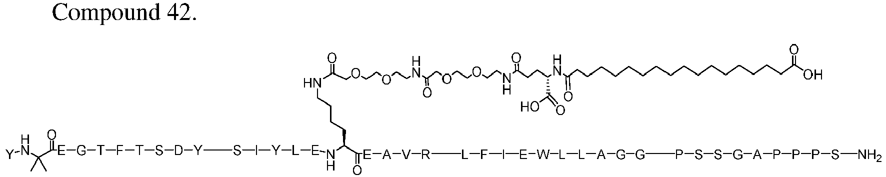 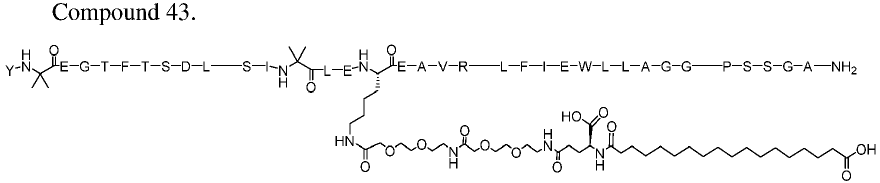 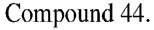 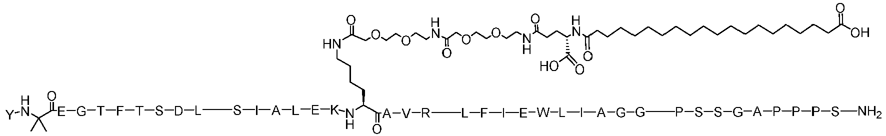 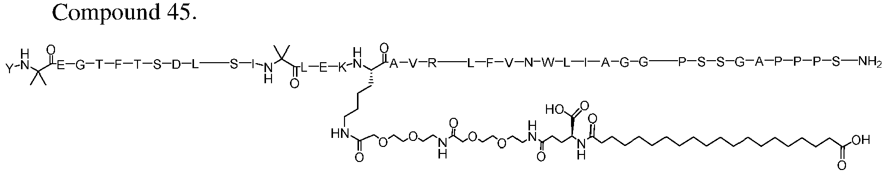 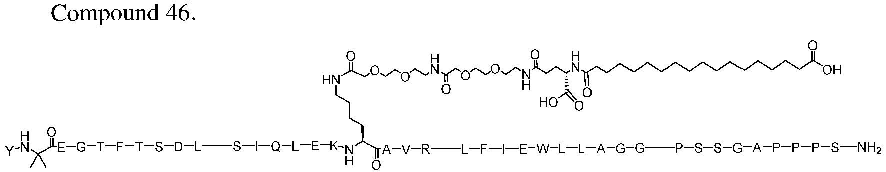 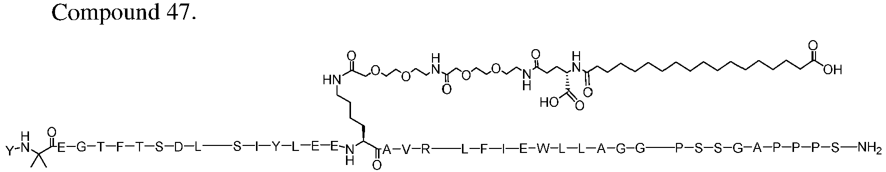 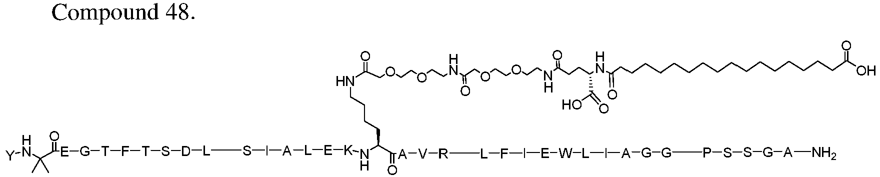 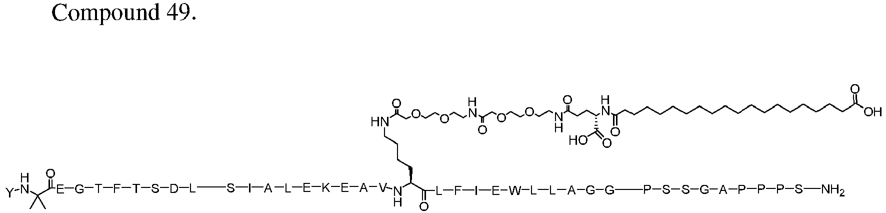 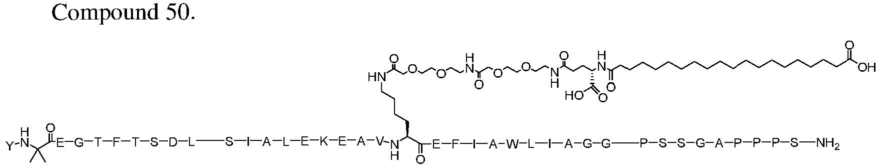 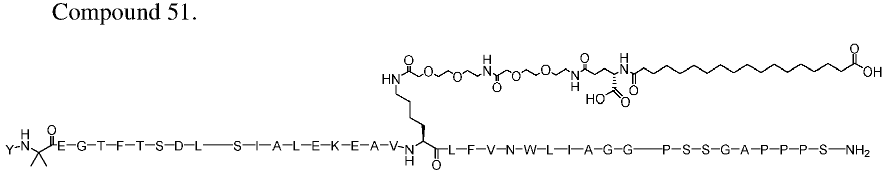 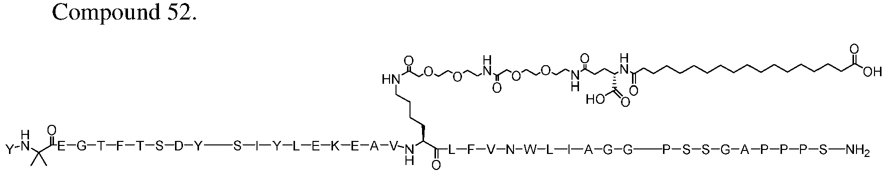 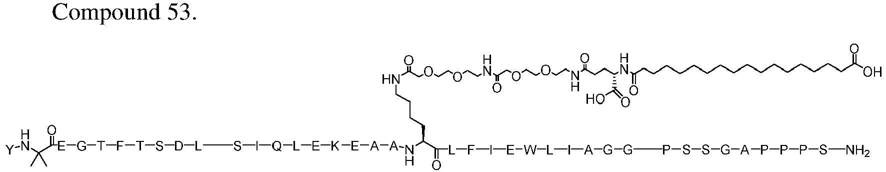 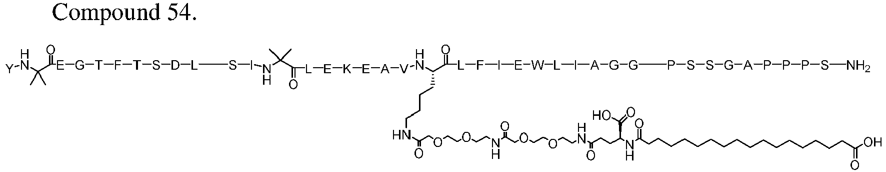 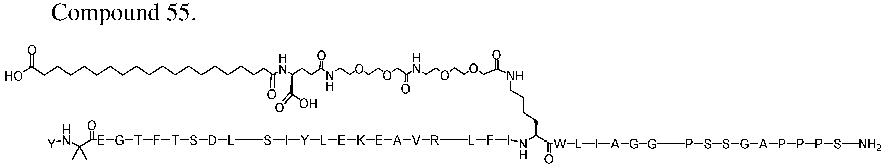 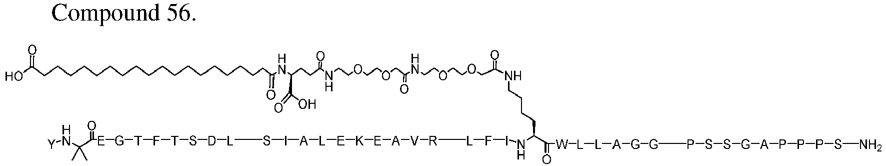 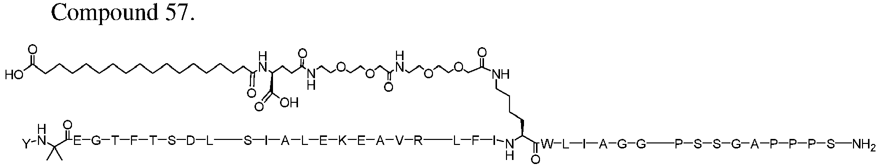 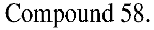 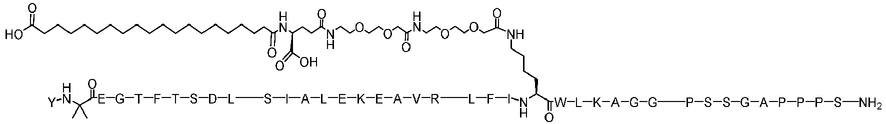 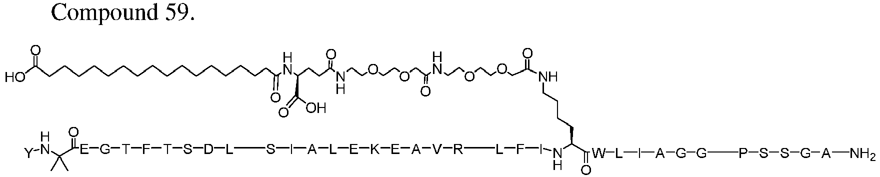 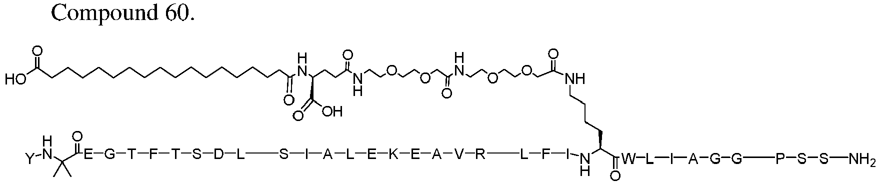 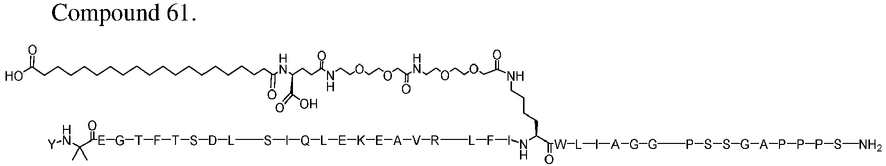 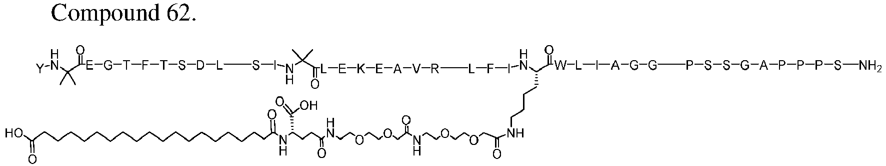 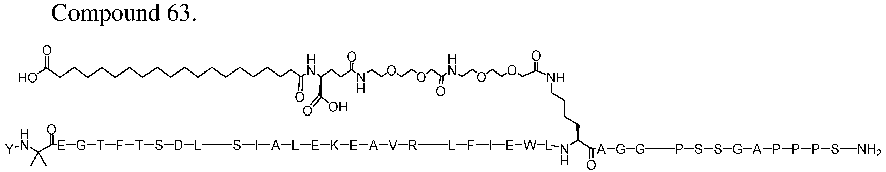 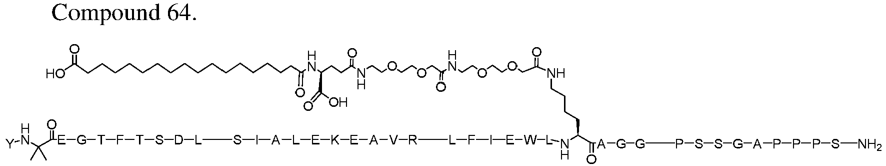 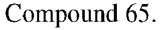 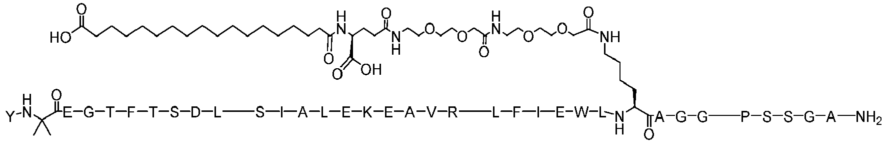 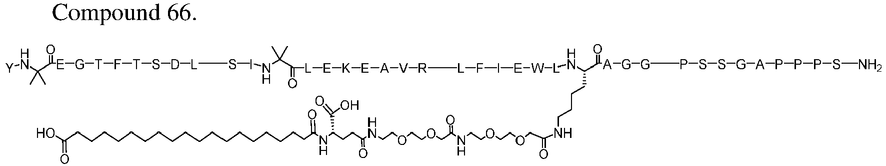 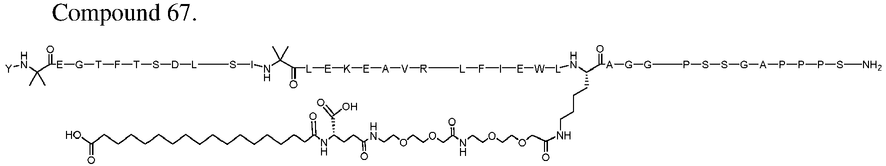 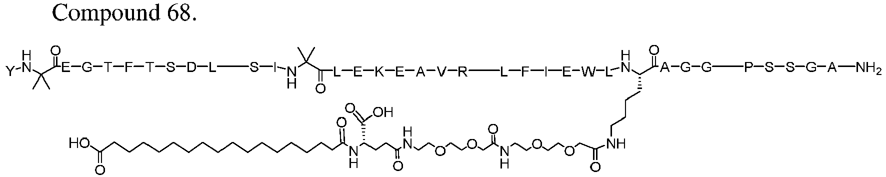 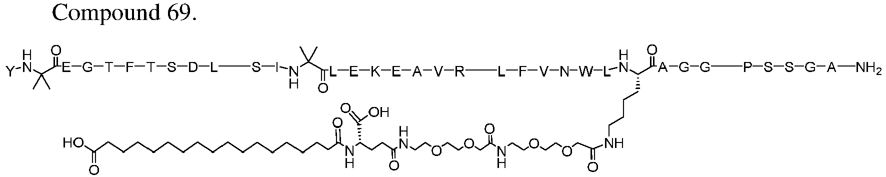 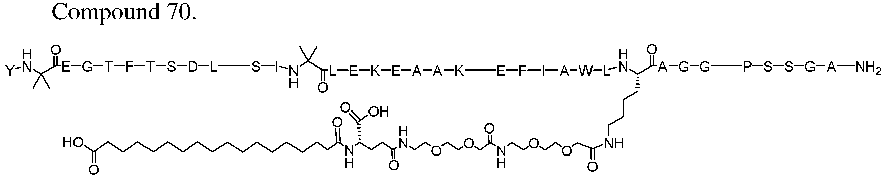 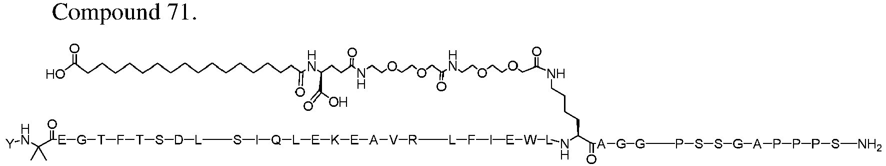 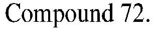 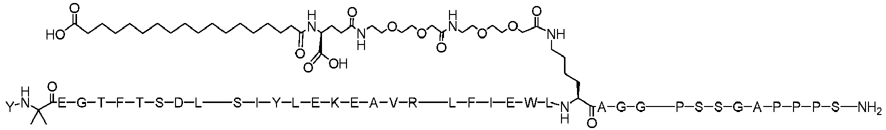 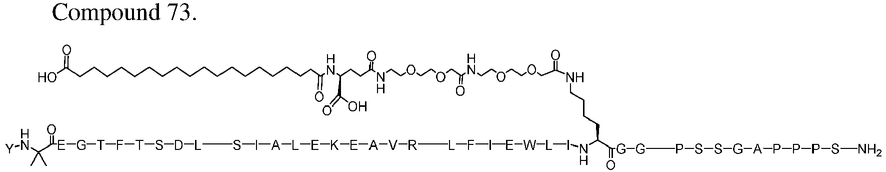 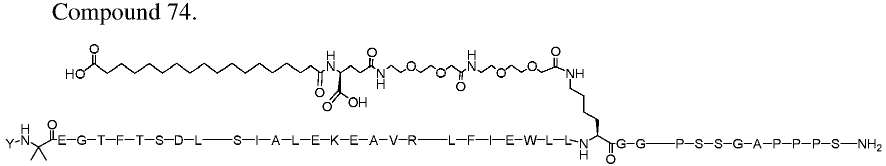 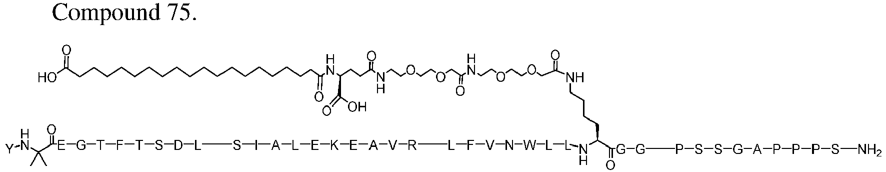 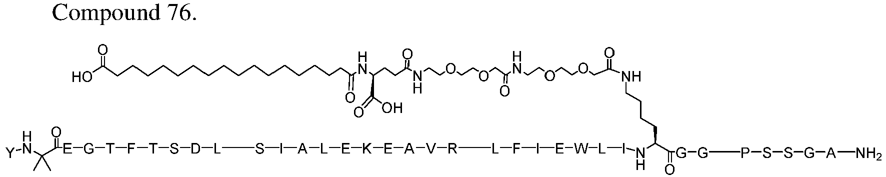 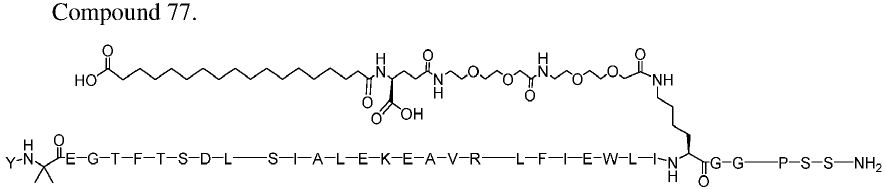 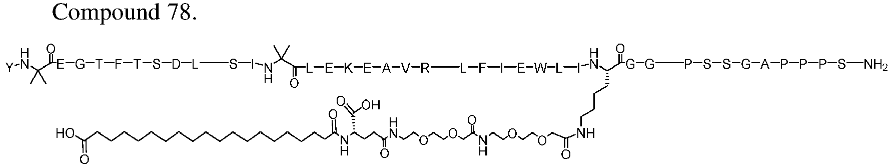 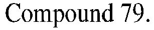 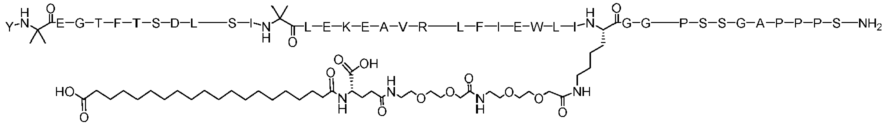 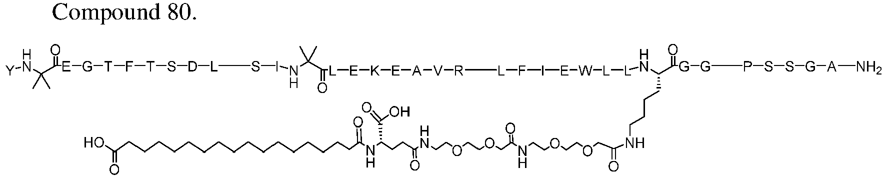 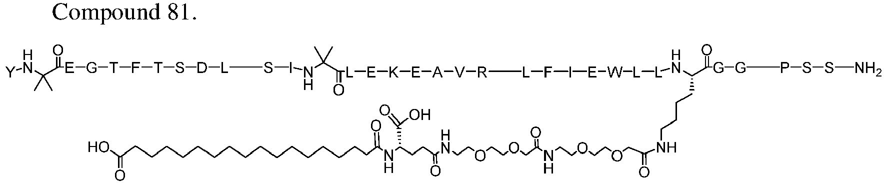 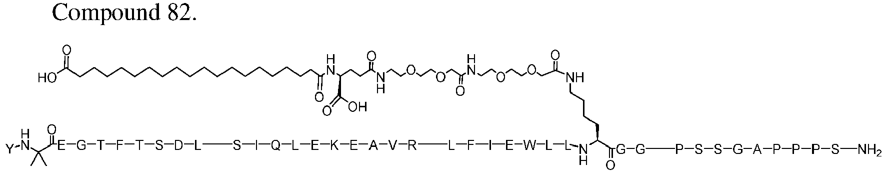 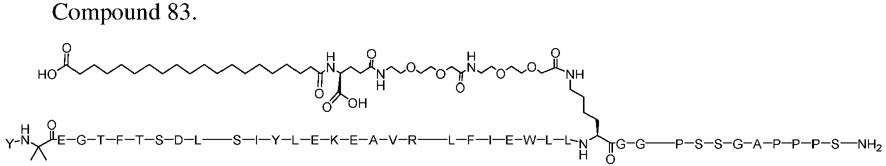 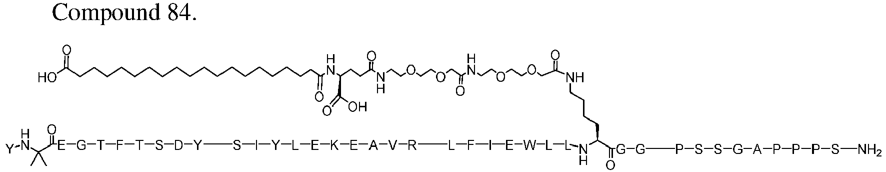 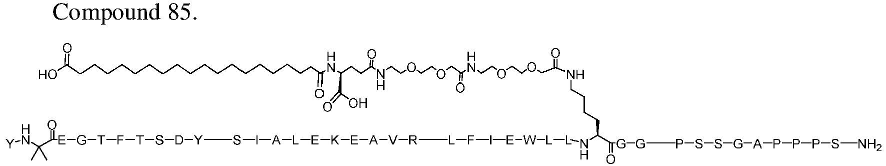
Compound 86. YaibEGTFTSDLSK(x20)ALEKEAVRLFIEWLIAGGPSSGA-NH₂
Compound 87. YaibEGTFTSDLSIQLEEEAVRLFIEWLIK(x20)GGPSSGAPPPS-NH₂
Compound 88. YaibEGTFTSDLSK(x18)ALEKEAVRLFIEWLKAGGPSS-NH2
Compound 89. YaibEGTFTSDLSIaibLEKEAVRLFIEWLIAGGPSSK(x18)-NH₂
Compound 90. YaibEGTFTSDLSIQLEKEAVRLFIEWLIK(x18)GGPSSGA-NH₂
Compound 91. YaibEGTFTSDLSK(x18)QLEKEAVRLFIEWLIAGGPSS-NH2
Compound 92. YaibEGTFTSDLSIaibLEKEAVRLFIEWLKAGGPSSGK(x18)-NH₂
Compound 93.YaibEGTFTSDLSK(x20)QLEKEAVQDFVNWLLAGGPSSGAPPPS-NH₂
Compound 94.YaibEGTFTSDLSIYLEKEAVRLFIEWLLK(x18)GGPSSGAPPPS-NH2
Compound 95.YaibEGTFTSDLSIALEKEAVRLFIEWLLAGGPSSGAPPPC(x20)-NH₂
Compound 96.YaibEGTFTSDLSIQLEKEAVRLFIEWLIK(x18)GGPSSGAPPPS-NH₂
Compound 97.YaibEGTFTSDLSK(x20)ALEKEAVKEFIAWLIAGGPSSGAPPPS-NH₂
Compound 98. YaibEGTFTSDLSIALEKEAVRLFIEWLKAGGPSSK(x20)-NH₂
Compound 99. YaibEGTFTSDLSIaibK(x20)EKEAVRLFIEWLIAGGPSSGAPPPS-NH₂
Compound 100. YaibEGTFTSDLSIQLEKEAVRLFIEWLIK(x18)GGPSS-NH₂
Compound 101.YaibEGTFTSDLSIaibK(x18)EKEAVRLFIEWLIAGGPSSGAPPPS-NH₂
Compound 102.YaibEGTFTSDLSIaibLEKEAVRLFIEWLIAGGPSK(x20)-NH₂
Compound 103.YaibEGTFTSDYSIYLEKEAVRLFIEWLLK(x20)GGPSSGAPPPS-NH₂
Compound 104.YaibEGTFTSDLSIAC(x18)EKEAVRLFIEWLLAGGPSSGAPPPS-NH₂
Compound 105.YaibEGTFTSDLSIALEKEAVRLFIEWLLAGGPSSGAPPPC(x18)-NH₂
Compound 106.YaibEGTFTSDLSIQLEKEAVKLFVNWLIK(x20)GGPSSGAPPPS-NH₂
Compound 107.YaibEGTFTSDLSIAC(x20)EKEAVRLFIEWLIAGGPSSGAPPPS-NH₂
Compound 108.YaibEGTFTSDLSIaibLEKEAVRLFIEWLLK(x20)GGPSS-NH2
Compound 109.YaibEGTFTSDLSIaibLEKEAVRLFIEWLIAGGPSSGAPPPC(x20)-NH₂
Compound 110.YaibEGTFTSDLSIaibLEKEAVRLFIEWLIAGGPSSK(x20)-NH₂
Compound 111.YaibEGTFTSDLSIALEKEAVRLFIEWLKAGGPSSGA-NH2
Compound 112.YaibEGTFTSDLSIALEKEAVRLFIEWLKAGGPSSGAPPPS-NH2
Compound 113.YaibEGTFTSDLSIQLEKEAVRLFIEWLLAGGPSSGAPPPC(x18)-NH₂
Compound 114.YaibEGTFTSDK(x18)SIYLEKEAVRLFIEWLIAGGPSSGA-NH₂
Compound 115.YaibEGTFTSDLSIALEKEAVRLFIEWLIAGGPSSGAK(x20)-NH₂
Compound 116.YaibEGTFTSDLSIaibLEKEAVRLFIEWLIAGGPSSGAPPPC(x18)-NH₂
Compound 117.YaibEGTFTSDK(x20)SIALEEEAVQDFVQWLIAGGPSSGAPPPS-NH₂
Compound 118.YaibEGTFTSDLSC(x20)ALEKEAVKEFIAWLIAGGPSSGAPPPS-NH₂
Compound 119.YaibEGTFTSDLSIYLEKEAVKLFIEWLIAGGPSSGK(x20)-NH₂
Compound 120.YaibEGTFTSDLSIaibLEKEAVRLFIEWLLK(x20)GGPSSGA-NH₂
Compound 121.YaibEGTFTSDLSIALEKEAVRLFIEWLKAGGPSS-NH2
Compound 122.YaibEGTFTSDK(x18)SIALEKEAVRLFIEWLLAGGPS-NH₂
Compound 123.YaibEGTFTSDLSIaibLEKEAVRLFIEWLIAGGPK(x20)-NH₂
Compound 124.YaibEGTFTSDLSIaibLEKK(x18)AVRLFIEWLKAGGPS-NH₂
Compound 125.YaibEGTFTSDLSIALEKEAVRLFIEWLIK(x20)GGPSS-NH2
Compound 126.YaibEGTFTSDLSIaibLEKEAVRLFIEWLIAGGPK(x18)-NH₂
Compound 127.YaibEGTFTSDLSIAK(x18)EKEAVRLFIEWLLAGGPSSGAPPPS-NH₂
Compound 128.YaibEGTFTSDLSIaibLEKEAVRLFIEWLKAGGPSSG-NH₂
Compound 129.YaibEGTFTSDLSIALEKEAVRLFIEWLKK(x20)GGPSSGAPPPS-NH₂
Compound l30.YaibEGTFTSDLSIaibLEKEAVRLFIEWLKAGGPSSGAK(x18)-NH₂
Compound 131.YaibEGTFTSDLSIaibLEKEAVRLFIEWLKAGGPSSGAPPPS-NH₂
Compound 132.YaibEGTFTSDLSIQK(x18)EKEAVRLFIEWLKAGGPSS-NH₂
Compound 133.YaibEGTFTSDLSIYLEKEAVRLFIEWLLK(x18)GGPS-NH2
Compound 134.YaibEGTFTSDLSIYLEKEAVKLFIEWLIAGGPSSGAPPPC(x18)-NH₂
Compound 135.YaibEGTFTSDLSIaibLEKEAVRLFIEWLKAGGPSSGA-NH₂
Compound 136.YaibEGTFTSDLSIaibLEKK(x20)AVRLFIEWLKAGGPSSGAPPPS-NH₂
Compound 137.YaibEGTFTSDLSIALEC(x18)EAVRLFIEWLLAGGPSSGAPPPS-NH₂
Compound 138.YaibEGTFTSDLSIYLEKEAVKLFIEWLIAGGPSSGAK(x20)-NH₂
Compound 139.YaibEGTFTSDLSIYK(x20)EKEAVRLFIEWLKAGGPSSGA-NH₂
Compound 140.YaibEGTFTSDK(x18)SIALEKEAVRLFIEWLKAGGPSSGAPPPS-NH₂
Compound 141.YaibEGTFTSDLSIaibLEKK(x18)AVRLFIEWLKAGGPSSG-NH₂
Compound 142.YaibEGTFTSDLSIYLEKEAVKLFIEWLIAGGPSSGAPPPK(x18)-NH₂
Compound 143.YaibEGTFTSDLSIaibLEKEAVRLFIEWLC(x20)AGGPSSGAPPPS-NH₂
Compound 144.YaibEGTFTSDLSIYLEKEAVRLFIEWLKAGGPSS-NH₂
Compound 145.YaibEGTFTSDK(x20)SIaibLEKEAVRLFIEWLKAGGPSSGAPPPS-NH₂
Compound 146.Y(aib)EGTFTSDLSIALEKEAVRLFIEWLIAGGPK(x20)-NH₂
Compound 147.YaibEGTFTSDC(x18)SIaibLEKEAVRLFIEWLLAGGPSSGAPPPS-NH₂
Compound 148.YaibEGTFTSDLSIaibLEKK(x18)AVRLFIEWLLAGGPSSGAPPPS-NH₂
Compound 149.YaibEGTFTSDLSIALEKEAVRLFIEWLIAGGPSSGK(x18)-NH₂
Compound l50.YaibEGTFTSDLSIYK(x18)EKEAVRLFIEWLLAGGPS-NH2
Compound 151.YaibEGTFTSDLSIALEKEAVRLFIEWLC(x20)AGGPSSGAPPPS-NH₂
Compound 152.YaibEGTFTSDC(x20)SIALEKEAVRLFIEWLLAGGPSSGAPPPS-NH₂
Compound 153.YaibEGTFTSDLSIQK(x18)EKEAVRLFIEWLKAGGPSSGA-NH₂
Compound 154.YaibEGTFTSDLSIaibLEKEAVRLFIEWLC(x18)AGGPSSGAPPPS-NH₂
Compound 155.YaibEGTFTSDLSIaibLEKEAVRLFIEWLIAGGPSK(x18)-NH₂
Compound l56.YaibEGTFTSDLSIAK(x18)EKEAVRLFIEWLKAGGPSSGA-NH₂
Compound 157.YaibEGTFTSDLSIALEKEAVRLFIEWLIAGGPSK(x18)-NH₂
Compound 158.YaibEGTFTSDLSIYLEKEAVRLFIK(x18)WLIAGGPSS-NH2
Compound 159.YaibEGTFTSDLSIAK(x20)EKEAVRLFIEWLIAGGPSSGAPPPS-NH₂
Compound 160.YaibEGTFTSDLSIQLEKEAVRLFIEWLIAGGPSSK(x18)-NH₂
Compound 161.YaibEGTFTSDLSIaibK(x18)EKEAVRLFIEWLIAGGPSSGA-NH₂
Compound 162.YaibEGTFTSDLSIALEKEAVRLFIEWLC(x20)AGGPSSGA-NH₂
Compound 163.YaibEGTFTSDLSIALEKEAVRLFIEWLIAGGPSK(x20)-NH₂
Compound 164.YaibEGTFTSDLSIALEKK(x18)AVRLFIEWLIAGGPSSGAPPPS-NH₂
Compound 165.YaibEGTFTSDLSIALEKEAVRLFIEWLKAGGPSSG-NH₂
Compound 166.YaibEGTFTSDLSIALEKK(x18)AVRLFIEWLLAGGPSSGA-NH₂
Compound 167.YaibEGTFTSDLSIYLEKEAVRLFIK(x18)WLIAGGPSSGA-NH₂
Compound 168.YaibEGTFTSDLSIaibLEKEAVRLFIEWLK(x20)AGGPSS-NH₂
Compound 169.YaibEGTFTSDLSIQLEKEAVRLFIEWLKAGGPSSGAPP-NH₂
Compound 170.YaibEGTFTSDLSIAK(x18)EKEAVRLFIEWLKAGGPSS-NH₂
Compound 171.YaibEGTFTSDLSIaibLEKEAVRLFIEWLKAGGPS-NH₂
Compound 172.YaibEGTFTSDLSIALEKK(x18)AVRLFIEWLLAGGPSS-NH₂
Compound 173. YaibEGTFTSDLSC(x20)aibLEKEA VKEFIAWLIAGGPS SGAPPPS - NH₂
Compound 174.YaibEGTFTSDLSIALEKEAVRLFIEWLKAGGPS-NH₂
Compound 175.YaibEGTFTSDLSIALEKK(x20)AVRLFIEWLKAGGPSSGAPPPS-NH₂
Compound 176.YaibEGTFTSDLSIALEKEAVRLFIEWLIAGGPSSGK(x20)-NH₂
Compound 177.YaibEGTFTSDLSIALEKEAVRLFIEWLC(x18)AGGPSSGA-NH₂
Compound 178.YaibEGTFTSDLSIQLEEEAVRLFIEWLK(x20)AGGPSSGAPPPS-NH₂
Compound 179.YaibEGTFTSDLSIYLEKEAVRLFIEWLLC(x20)GGPSSGAPPPS-NH₂
Compound 180.YaibEGTFTSDLSIALEKEAVRLFIEWLIAGGPK(x18)-NH₂
Compound 181.YaibEGTFTSDLSIaibLEK(x18)EAVRLFIEWLIAGGPSSGAPPPS-NH₂
Compound 182.YaibEGTFTSDLSIALEKEAVRLFIEWLLC(x20)GGPSS-NH₂
Compound 183.YaibEGTFTSDLSIALEC(x20)EAVRLFIEWLIAGGPSSGAPPPS-NH₂
Compound 184.YaibEGTFTSDLSIYLEKEAVRLFIEWLIAGGPSSK(x20)-NH₂
Compound 185.YaibEGTFTSDYSIYLEKK(x20)AVRLFIEWLIAGGPSSGAPPPS-NH₂
Compound 186.YaibEGTFTSDLSIQLEKEAVRLFIEWLIAGGPSK(x18)-NH₂
Compound 187.YaibEGTFTSDLSIYLEKK(x20)AVRLFIEWLLAGGPSSGAPPPS-NH₂
Compound 188.YaibEGTFTSDLSIaibLEKEAVRLFIC(x18)WLIAGGPSSGAPPPS-NH₂
Compound 189.YaibEGTFTSDLSIQLEKEAVRLFIEWLIC(x18)GGPSSG-NH₂
Compound 190.YaibEGTFTSDLSIALEKEAVK(x18)LFIEWLKAGGPSSGA-NH₂
Compound 191.YaibEGTFTSDLSIALEKEAVRLFIEWLIAGGPSSK(x20)-NH₂
Compound 192.YaibEGTFTSDLSIQLEKEAVRLFIEWLKK(x18)GGPSSGAPPPS-NH₂
Compound 193.YaibEGTFTSDLSIALEKEAVK(x18)LFIEWLKAGGPSS-NH₂
Compound 194.YaibEGTFTSDLSIYLEKK(x20)AVRLFIEWLKAGGPSSGAPPPS-NH₂
Compound 195.YaibEGTFTSDLSIQLEKEAVRLFIK(x18)WLKAGGPSS-NH₂
Compound 196.YaibEGTFTSDLSIYLEKK(x18)AVRLFIEWLKAGGPSSGA-NH₂
Compound 197.YaibEGTFTSDLSIYLEKEAVRLFIK(x18)WLLAGGPSSGAPPPS-NH₂
Compound 198.YaibEGTFTSDLSIQLEKEAVRLFIEWLKAGGPSSGAP-NH₂
Compound 199.YaibEGTFTSDLSIALEKEAVK(x20)LFIEWLKAGGPSSGAPPPS-NH₂
Compound 200.YaibEGTFTSDLSIYLEKEAVRLFIEWLIAGGPSSGAPPPC(x20)-NH₂
Compound 201.YaibEGTFTSDLSIaibLEKEAVRLFIK(x18)WLLAGGPSSGAPPPS-NH₂
Compound 202.YaibEGTFTSDLSIYLEKK(x18)AVRLFIEWLKAGGPSS-NH2
Compound 203.YaibEGTFTSDLSIALEKEAVK(x18)LFIEWLIAGGPSSGAPPPS-NH₂
Compound 204.YaibEGTFTSDLSIQLEKEAVRLFIEWLKAGGPSSGA-NH₂
Compound 205.YaibEGTFTSDLSIALEKEAVRLFIEWLLC(x18)GGPSS-NH₂
Compound 206.YaibEGTFTSDLSIALEKEAVK(x20)DFVNWLLAGGPSSGAPPPS-NH₂
Compound 207.YaibEGTFTSDLSIQLEKEAVRLFIEWLIAGGPSK(x20)-NH₂
Compound 208.YaibEGTFTSDLSIYLEKEAVRLFIEWLLK(x18)GGPSSGA-NH₂
Compound 209.YaibEGTFTSDLSIALEKEAVC(x18)LFIEWLIAGGPSSGAPPPS-NH₂
Compound 210.YaibEGTFTSDLSIALEKEAVRLFIC(x18)WLLAGGPSSGAPPPS-NH₂
Compound 211.YaibEGTFTSDLSIQLEKEAVRLFIEWLKAGGPSSG-NH₂
Compound 212.YaibEGTFTSDLSIALEKEAVC(x20)LFIEWLIAGGPSSGAPPPS-NH₂
Compound 213.YaibEGTFTSDLSIaibLEKK(x18)AVRLFIEWLLAGGPSS-NH₂
Compound 214.YaibEGTFTSDLSIALEKEAVRLFIEWLLC(x20)GGPSSGAPPPS-NH₂
Compound 215.YaibEGTFTSDLSIYLEKEAVRLFIEWLLC(x18)GGPSS-NH₂
Compound 216.YaibEGTFTSDLSIALEKC(x20)AVRLFIEWLIAGGPSSGAPPPS-NH₂
Compound 217.YaibEGTFTSDLSIYLEKEAVK(x20)LFIEWLLAGGPSSGAPPPS-NH₂
Compound 218.YaibEGTFTSDLSIQLEKEAVRLFIEWLKAGGPSS-NH₂
Compound 219.YaibEGTFTSDYSIYLEKEAVRLFIC(x20)WLLAGGPSSGAPPPS-NH₂
Compound 220.YaibEGTFTSDLSIYLEKEAVK(x20)LFIEWLKAGGPS-NH₂
Compound 221.YaibEGTFTSDLSIaibLEKEAVRLFIEWLK(x20)AGGPSSGA-NH₂
Compound 222.YaibEGTFTSDLSIaibLEKEAVRLFIK(x18)WLKAGGPSS-NH₂
Compound 223.YaibEGTFTSDLSIALEKC(x18)AVRLFIEWLIAGGPSSGAPPPS-NH₂
Compound 224.YaibEGTFTSDLSIQLEKEAVRLFIEWLKAGGPS-NH₂
Compound 225.YaibEGTFTSDLSIYLEKEAVK(x18)LFIEWLIAGGPSSGAPPPS-NH₂
Compound 226.YaibEGTFTSDLSIaibLEK(x20)EAVRLFIEWLIAGGPSSGAPPPS-NH₂
Compound 227.YaibEGTFTSDLSIYLEKEAVK(x18)LFIEWLLAGGPSSG-NH₂
Compound 228.YaibEGTFTSDLSIYLEKEAVRLFIEWLLC(x20)GGPSS-NH₂
Compound 229.YaibEGTFTSDLSIALEKEAVRLFIK(x20)WLKAGGPSSGA-NH₂
Compound 230.YaibEGTFTSDLSIaibLEKEAVRLFIEWLLC(x20)GGPSS-NH₂
Compound 231.YaibEGTFTSDLSIaibLEKEAVK(x18)LFIEWLKAGGPSSGA-NH₂
Compound 232.YaibEGTFTSDLSIALEKEAVRLFIK(x20)WLKAGGPSS-NH₂
Compound 233.YaibEGTFTSDLSIALEKEAVKEFIAWLK(x20)AGGPSSGAPPPS-NH₂
Compound 234.YaibEGTFTSDLSIYLEKEAVRLFIEWLKAGGPS-NH₂
Compound 235.YaibEGTFTSDLSIQLEKEAVK(x18)LFIEWLLAGGPSSGAPPPS-NH₂
Compound 236.YaibEGTFTSDLSIYLEK(x20)EAVRLFIEWLIAGGPSSGAPPPS-NH₂
Compound 237.YaibEGTFTSDLSIaibLEKEAVRLFIEWLIAGGPSSGAK(x20)-NH₂
Compound 238.YaibEGTFTSDLSIQLEKEAVRLFIEWLK(x20)AGGPSSGAPPPS-NH₂
Compound 239.YaibEGTFTSDLSIALEKEAVRLFIK(x18)WLKAGGPSS-NH₂
Compound 240.YaibEGTFTSDLSIaibLEKEAVRLFIEWLLC(x18)GGPSSGAPPPS-NH₂
Compound 24EYaibEGTFTSDYSIYLEKEAVRLFIC(x18)WLLAGGPSSG-NH₂
Compound 242.YaibEGTFTSDLSIaibLEKEAVRLFIEWLLC(x20)GGPSSGA-NH₂
Compound 243.YaibEGTFTSDLSIQLEKEAVRLFIC(x20)WLLAGGPSSGAPPPS-NH₂
Compound 244.YaibEGTFTSDLSIaibLEKEAVRLFIEWLIAGGPSSGK(x18)-NH₂
Compound 245.YaibEGTFTSDYSIYLEKEAVRLFIEWLK(x20)AGGPSSGAPPPS-NH₂
Compound 246.YaibEGTFTSDLSIQLEKEAVRLFIK(x18)WLIAGGPS-NH₂
Compound 247.YaibEGTFTSDLSIALEK(x18)EAVRLFIEWLLAGGPSSGAPPPS-NH₂
Compound 248.YaibEGTFTSDLSIaibLEKEAVRLFIEWLLC(x18)GGPSSGA-NH₂
Compound 249.YaibEGTFTSDLSIQLEK(x18)EAVRLFIEWLLAGGPSSG-NH₂
Compound 250.YaibEGTFTSDLSIALEKEAVRLFIK(x18)WLKAGGPSSGA-NH₂
Compound 251.YaibEGTFTSDLSIQLEK(x18)EAVRLFIEWLLAGGPS-NH₂
Compound 252. YaibEGTFTSDYSIYLEKEA VRLFIC(x 18)WLLAGGPSSGAPPPS-NH₂
Compound 253.Y aibEGTFTS DLS IaibLEKEA VRLFIEWLKK(x18)GGPSSGAPPPS-NH₂
Compound 254.YaibEGTFTSDLSIYLEKEAVRLFIEWLKAGGPSSGAPPPS-NH₂
Compound 255.YaibEGTFTSDLSIaibLEKEAVRLFIK(x20)WLIAGGPSSGA-NH₂
Compound 256.YaibEGTFTSDLSIQLEKEAVK(x18)LFIEWLLAGGPSSG-NH₂
Compound 257.YaibEGTFTSDLSIaibLEKEAVRLFIK(x18)WLIAGGPSS-NH₂
Compound 258.YaibEGTFTSDLSIYLEKEAVRLFIEWLLC(x20)GGPSSGA-NH₂
Compound 259.YaibEGTFTSDLSIQLEKK(x20)AVRLFIEWLIAGGPSSGAPPPS-NH₂
Compound 260.YaibEGTFTSDLSIaibLEKEAVRLFIK(x20)WLKAGGPSSGAPPPS-NH₂
Compound 261.YaibEGTFTSDLSIQLEKEAVK(x18)LFIEWLIAGGPS-NH₂
Compound 262.YaibEGTFTSDLSIaibLEKEAVRLFIC(x20)WLLAGGPSSGAPPPS-NH₂
Compound 263.YaibEGTFTSDLSIQLEKEAVRLFIEWLIC(x20)GGPSSG-NH₂
Compound 264.YaibEGTFTSDLSIYLEKEAVRLFIEWLKAGGPSSGAPP-NH₂
Compound 265.YaibEGTFTSDLSIQLEKEAVRLFIEWLK(x20)AGGPSSGA-NH₂
Compound 266.YaibEGTFTSDLSIaibLEKEAVRLFIC(x20)WLKAGGPSSGAPPPS-NH₂
Compound 267.YaibEGTFTSDLSIALEKEAVRLFIEWLIK(x20)GGPSSGA-NH₂
Compound 268.YaibEGTFTSDLSIQLEKEAVRLFIC(x18)WLLAGGPSSGAPPPS-NH₂
Compound 269.YaibEGTFTSDLSIALEKEAVRLFIEWLLC(x18)GGPSSGAPPPS-NH₂
Compound 270.YaibEGTFTSDLSIYLEKEAVRLFIEWLKAGGPSSGAP-NH₂
Compound 271.YaibEGTFTSDLSIaibLEKEAVRLFIC(x18)WLKAGGPSSGAPPPS-NH₂
Compound 272..YaibEGTFTSDLSIQLEKEAVRLFIEWLK(x18)AGGPSS-NH₂
Compound 273.YaibEGTFTSDLSIALEKEAVRLFIEWLKAGGPSSGAK(x20)-NH₂
Compound 274..YaibEGTFTSDLSIQLEKEAVRLFIEWLC(x18)AGGPSSGAPPPS-NH₂
Compound 275.YaibEGTFTSDLSIYLEKEAVRLFIEWLKAGGPSSGA-NH₂
Compound 276.YaibEGTFTSDLSIQLEKEAVRLFIK(x18)WLLAGGPSSGAPPPS-NH₂
Compound 277.YaibEGTFTSDLSIaibLEKEAVK(x18)LFIEWLKAGGPSS-NH₂
Compound 278.YaibEGTFTSDLSIYLEKEAVRLFIEWLLC(x18)GGPSSGAPPPS-NH2
Compound 279.YaibEGTFTSDLSIQLEKEAVRLFIK(x18)WLIAGGPSSG-NH₂
Compound 280.YaibEGTFTSDLSIQLEKEAVRLFIEWLIC(x18)GGPSSGAPPPS-NH₂
Compound 281.YaibEGTFTSDLSIaibLEKEAVRLFIEWLKAGGPSS-NH₂
Compound 282.YaibEGTFTSDLSIQLEKEAVRLFIK(x18)WLKAGGPSSGAPPPS-NH₂
Compound 283.YaibEGTFTSDLSIaibLEKEAVK(x20)LFIEWLLAGGPSSGAPPPS-NH₂
Compound 284.YaibEGTFTSDLSIQLEKEAVRLFIK(x18)WLKAGGPSSGA-NH₂
Compound 285.YaibEGTFTSDLSIALEKEAVRLFIK(xl 8)WLKAGGPSSGAPPPS-NH₂
Compound 286.YaibEGTFTSDLSIQLEKEAVRLFIEWLC(x20)AGGPSSGA-NH₂
Compound 287.YaibEGTFTSDLSIaibLEKEAVRLFIK(x18)WLKAGGPSSGA-NH₂
Compound 288.YaibEGTFTSDLSIALEKEAVRLFIEWLC(x18)AGGPSSGAPPPS-NH₂
Compound 289.YaibEGTFTSDLSIQLEKEAVRLFIEWLIAGGPSSK(x20)-NH₂
Compound 290.YaibEGTFTSDLSIALEKEAVRLFIK(x20)WLLAGGPSSGA-NH₂
Compound 291.YaibEGTFTSDLSIaibLEKEAVRLFIEWLK(x18)AGGPSSGA-NH₂
Compound 292.YaibEGTFTSDLSIQLEKEAVRLFIK(x20)WLKAGGPSSGAPPPS-NH₂
Compound 293.YaibEGTFTSDLSIYLEKEAVRLFIEWLLK(x20)GGPSSGA-NH₂
Compound 294.YaibEGTFTSDLSIALEKEAVRLFIK(x20)WLLAGGPSS-NH₂
Compound 295.YaibEGTFTSDLSIaibLEKEAVRLFIEWLK(x18)AGGPSS-NH₂
Compound 296.YaibEGTFTSDLSIYLEKEAVRLFIEWLLC(x18)GGPSSGA-NH₂
Compound 297.YaibEGTFTSDLSIaibLEKEAVRLFIEWLKAGGPSSGAPP-NH2
Compound 298.YaibEGTFTSDLSIALEKEAVRLFIEWLLC(x20)GGPSSGA-NH₂
Compound 299.YaibEGTFTSDLSIALEKEAVRLFIK(x18)WLKAGGPS-NH₂
Compound 300.YaibEGTFTSDLSIALEKEAVRLFIC(x18)WLKAGGPSSGAPPPS-NH₂
Compound 30EYaibEGTFTSDLSIALEKEAVRLFIEWLKK(x18)GGPSSGAPPPS-NH₂
Compound 302.YaibEGTFTSDLSIaibLEKEAVRLFIK(x20)WLKAGGPSSGA-NH₂
Compound 303.YaibEGTFTSDLSIQLEKK(x18)AVRLFIEWLIAGGPSSGA-NH₂
Compound 304.YaibEGTFTSDLSIaibLEKEAVRLFIEWLKK(x20)GGPSSGAPPPS-NH₂
Compound 305.YaibEGTFTSDLSIALEKEAVRLFIC(x20)WLKAGGPSSGAPPPS-NH₂
Compound 306.YaibEGTFTSDLSIYLEKEAVRLFIEWLKAGGPSSG-NH₂
Compound 307.YaibEGTFTSDLSIQLEKEAVRLFIEWLC(x20)AGGPSSGAPPPS-NH₂
Compound 308.YaibEGTFTSDK(x20)SIaibLEKEAVKEFIAWLLAGGPSSGAPPPS-NH₂
Compound 309.YaibEGTFTSDLSIALEKEAVRLFIEWLKAGGPSSGK(x20)-NH₂
Compound 310.YaibEGTFTSDLSK(x18)aibLEKEAVKEFIAWLIAGGPSSGAPPPS-NH₂
Compound 311.YaibEGTFTSDLSIALEKEAVRLFIC(x20)WLLAGGPSSGAPPPS-NH₂
Compound 312.YaibEGTFTSDLSIYLEKEAVRLFIEWLK(x20)AGGPSSGAPPPS-NH₂
Compound 313.YaibEGTFTSDLSIaibLEKEAVK(x18)LFIEWLKAGGPSSGAPPPS-NH₂
Compound 314.YaibEGTFTSDLSIQLEKEAVRLFIEWLIC(x20)GGPSSGAPPPS-NH₂
Compound 315.YaibEGTFTSDLSIaibLEKEAVRLFIK(x20)WLKAGGPSS-NH₂
Compound 316.YaibEGTFTSDLSIQLEKEAVQDFVNWLIK(x18)GGPSSGAPPPS-NH₂
Compound 317.YaibEGTFTSDLSIaibLEKEAVRLFIEWLKAGGPSK(x18)-NH₂
Compound 318.YaibEGTFTSDLSIYLEKEAVRLFIEWLC(x18)AGGPSSGAPPPS-NH₂
Compound 319. YaibEGTFTSDLSIQLEEEAVK(x18)EFIAWLLKGG-NH₂
Compound 320.YaibEGTFTSDLSIaibLEKEAVRLFIEWLIAGGPSSGAPPPK(x18)-NH₂
Compound 321.YaibEGTFTSDLSIQLEKEAVKLFVNWLK(x20)AGGPSSGAPPPS-NH₂
Compound 322.YaibEGTFTSDLSIALEKEAVRLFIEWLKAGGPSSGAP-NH₂
Compound 323.YaibEGTFTSDLSIaibLEKEAVRLFIEWLIAGGPSSGAK(x18)-NH₂
Compound 324.YaibEGTFTSDLSIQLEKEAVQDFVNWLK(x18)AGGPSSGAPPPS-NH₂
Compound 325.YaibEGTFTSDLSIYLEKEAVKLFIEWLIAGGPSK(x18)-NH₂
Compound 326.YaibEGTFTSDLSIaibLEKEAVRLFIK(x18)WLKAGGPSSGAPPPS-NH₂
Compound 327.YaibEGTFTSDLSIQLEKEAVRLFIEWLC(x18)AGGPSSGA-NH₂
Compound 328.YaibEGTFTSDK(x18)SIQLEKEAVRLFIEWLLAGGPSS-NH₂
Compound 329.YaibEGTFTSDLSIALEKEAVRLFIEWLK(x18)AGGPSS-NH₂
Compound 330.Y(aib)EGTFTSDLSIQLEKEAVRLFIEWLIAGGPSSGAK(x18)-NH₂
Compound 331.YaibEGTFTSDLSIALEKEAVRLFIEWLLC(x18)GGPSSGA-NH₂
Compound 332.YaibEGTFTSDLSIQLEKK(x18)AVRLFIEWLIAGGPSS-NH₂
Compound 333.YaibEGTFTSDLSIALEKEAVRLFIEWLC(x20)AGGPSS-NH₂
Compound 334.Y(aib)EGTFTSDLSIaibLEKEAVRLFIEWLKAGGPSSK(x18)-NH₂
Compound 335.YaibEGTFTSDLSIALEKEAVRLFIEWLC(x18)AGGPSS-NH₂
Compound 336.YaibEGTFTSDLSIaibLEKEAVRLFIEWLKAGGPSSGAP-NH₂
Compound 337.YaibEGTFTSDYSIYLEKEAVRLFIEWLLK(x18)GGPSSGAPPPS-NH₂
Compound 338.Y(aib)EGTFTSDLSIQLEKEAVRLFIEWLIAGGPSSGK(x18)-NH₂
Compound 339.YaibEGTFTSDLSIYLEKEAVRLFIEWLLK(x20)GGPSS-NH₂
Compound 340.YaibEGTFTSDK(x18)SIALEKEAVRLFIEWLLAGGPSSGA-NH₂
Compound 341.YaibEGTFTSDLSIQLEKEAVRLFIEWLKK(x20)GGPSSGAPPPS-NH₂
Compound 342.YaibEGTFTSDLSIQLEKEAVRLFIEWLIAGGPSSGAK(x20)-NH₂
Compound 343.YaibEGTFTSDLSIYLEKEAVRLFIC(x20)WLLAGGPSSGAPPPS-NH₂
Compound 344.YaibEGTFTSDLSK(x18)QLEKEAVRLFIEWLLAGGPSSGA-NH₂
Compound 345.YaibEGTFTSDLSIQLEKEAVRLFIEWLIC(x18)GGPS-NH₂
Compound 346. YaibEGTFTSDLSIALEKEAVRLFIEWLKAGGPSSGAPP-NH₂
Compound 347.YaibEGTFTSDLSIYLEKEAVRLFIEWLC(x20)AGGPSSGAPPPS-NH₂
Compound 348.YaibEGTFTSDLSIALEKEAVRLFIEWLIAGGPSSGAPPK(x20)-NH₂
Compound 349.YaibEGTFTSDLSIQLEKEAVRLFIK(x20)WLKAGGPSSGAPP-NH₂
Compound 350.Y(aib)EGTFTSDLSIALEKEAVRLFIEWLKAGGPSK(x18)-NH₂
Compound 351.Y(aib)EGTFTSDLSIaibLEKEAVRLFIEWLIAGGPSSGK(x20)-NH₂
Compound 352.YaibEGTFTSDLSIaibLEKEAVRLFIEWLLC(x18)GGPSS-NH₂
Compound 353.YaibEGTFTSDLSIYLEKEAVRLFIC(x18)WLLAGGPSSGAPPPS-NH₂
Compound 354.YaibEGTFTSDLSSYLEKEAVRLFVQWLKRGGPSSGAPC(x20)-NH₂
Compound 35 5. YaibEGTFTSDLSIaibLEKEA VRLFIEWLLC(x20)GGPS SGAPPPS - NH₂
Compound 356. YaibEGTFTSDLSIQLEKEAVRLFIK(x20)WLIAGGPSSGAP-NH₂
Compound 357. YaibEGTFTSDLSIYLEEEAAK(x18)EFIAWLLKGGP-NH₂

The peptide compounds according to any of the preceding aspects, wherein the peptide compounds contain only natural amino acids.

In one aspect, the invention relates to a pharmaceutical composition comprising a peptide compound or a pharmaceutically acceptable salt or solvate thereof of any of the preceding aspects, and a pharmaceutically acceptable carrier or excipient thereof.

In one aspect, the invention relates to methods of treating or preventing the following diseases or conditions: impaired glucose tolerance (IGT), hyperglycemia, type 1 diabetes, type 2 diabetes, obesity, metabolic syndrome and neurodegenerative diseases, especially for delaying or preventing disease progression in type 2 diabetes, delaying the progression from impaired glucose tolerance to type 2 diabetes; delaying the progression from type 2 diabetes to diabetes requiring insulin; treating metabolic syndrome, regulating appetite, inducing satiety, reducing food intake, increasing energy expenditure, treating obesity or preventing overweight; preventing weight rebound after successful weight loss; treating diseases or conditions associated with overweight or obesity; treating bulimia; treating binge eating; treating blood lipid abnormality, atherosclerosis, hypertension, coronary heart disease, β-blocker poisoning; treating non-alcoholic fatty liver disease (NAFLD) (which can be divided into simple fatty liver (SFL), non-alcoholic steatohepatitis (NASH) and its associated cirrhosis); inhibiting gastrointestinal motility, for use in conjunction with gastrointestinal investigation techniques such as X-ray, CT, and NMR scanning; the method comprises administering to the patient an effective amount of the peptide compound or the pharmaceutically acceptable salt or solvate thereof of any of the preceding aspects or a pharmaceutical composition thereof.

In one aspect, the present invention relates to the use of the peptide compound or the pharmaceutically acceptable salt or solvate thereof or the pharmaceutical composition thereof in any one of the preceding aspects in the preparation of a medicament for reducing blood glucose or treating diabetes.

In one aspect, the present invention relates to the use of the peptide compound or the pharmaceutically acceptable salt or solvate thereof or the pharmaceutical composition thereof in any one of the preceding aspects in the preparation of a medicament for weight loss.

In one aspect, the present invention relates to the use of the peptide compound or the pharmaceutically acceptable salt or solvate thereof or the pharmaceutical composition of any one of the preceding aspects in the preparation of a medicament for reducing blood lipids, preferably reducing the blood lipid components selected from the following: cholesterol, triglycerides, free fatty acids, low density lipoprotein; more preferably, reducing low density lipoprotein cholesterol.

In one aspect, the present invention relates to a method for preparing a peptide compound according to any one of the foregoing aspects, wherein the preparation method is a chemical synthesis method.

The inventors made a series of structural modifications to the peptide derivatives of GLP-1 receptor agonists, including selecting specific amino acids, or introducing new amino acids at the C-terminus of peptides, or replacing the C-terminal amino acid residues of peptides, and the unique long-acting groups are linked to peptides through either the side chain thiol group of cysteine residue or the side chain amino group of lysine residue at the C-terminus of peptides to obtain a series of new peptide compounds. Unexpected technical effects have been achieved.

### DESCRIPTION OF THE FIGURES

Figure 1: Compounds 12 and 15 of the present invention lowered blood glucose of db/db mice.
Figure 2: Compounds 55, 58, and 243 of the present invention reduced body weight of DIO mice.

### DETAILED DESCRIPTION

Unless otherwise stated, the following definitions apply throughout the present invention. Undefined terms can be understood in accordance with conventional definitions in the industry.

"Amino acid" refers to a molecule containing both amino and carboxyl functional groups, and the amino and carboxyl groups of an α-amino acid are attached to the same carbon atom (α carbon). The alpha carbon may have 1-2 additonal organic substituents. Amino acids include L and D isomers and racemic mixtures. Unless otherwise specified, the amino acid residues in the peptide sequence of the present invention are all L isomers, that is, L-amino acids, and D-amino acids are indicated by a lowercase letter "d" before the amino acid name or abbreviation, such as dK.

The amino acid sequences of the present invention contain the conventional one-letter or three-letter codes for naturally occurring amino acids, as well as the generally recognized three-letter codes for other amino acids, such as Tic (1,2,3,4-tetrahydroisoquinoline -3-carboxylic acid), Aib (α-aminoisobutyric acid) or GABA (γ-aminobutyric acid). The abbreviated codes of commonly used molecular structures include:
hGlu is homoglutamic acid;
α-hGlu is the L isomer of -HNCH(CO-)CH₂CH₂CH₂COOH;
δ-hGlu is the L isomer of-HNCH(COOH)CH₂CH₂CH₂CO-;
α-Glu is the L isomer of -HNCH(CO-)CH₂CH₂COOH;
γ-Glu or gGlu is the L isomer of -HNCH(COOH)CH₂CH₂CO-;
α-Asp is the L isomer of -HNCH(CO-)CH₂COOH;
β-Asp is the L isomer of -HNCH(COOH)CH₂CO-;
β-Ala is -HN-CH₂-CH₂-COOH ;

PEG2 is 2-(2-(2-aminoethoxy)ethoxy)acetic acid (CAS No. 134978-97-5).

The amino acid composition of a peptide in the present invention can be changed without substantially affecting its biological activity. For example, a peptide sequence may contain one or more conservative amino acid substitutions. Conservative amino acid substitution is the substitution of one amino acid residue with another amino acid residue with a similar side chain. In the literature, amino acid residues are classified according to the nature of their side chains. Amino acid residues containing basic side chains include lysine, arginine, and histidine; amino acid residues containing acidic side chains and amide side chains include aspartic acid, glutamic acid, asparagine, and glutamine; amino acid residues containing small aliphatic, non-polar or weakly polar side chains include glycine, alanine, threonine, serine, and proline; amino acid residues containing large aliphatic, non-polar side chains include leucine, isoleucine, and valine; amino acid residues with aromatic side chains include phenylalanine, tryptophan, and tyrosine; amino acid residues with sulfur-containing side chains include cysteine and methionine.

In some embodiments, the derivative comprises a substituent containing a lipophilic moiety and an optional 1-3 negatively charged moiety, wherein one of the negatively charged moieties is distal to the lipophilic moiety. In some embodiments, the substituent is attached to the side chain of the C-terminal amino acid of the sequence. If the C-terminus of the sequence is lysine it is attached to the ε amino group of the lysine residue.

As used herein, "expression vector" includes a vector capable of expressing DNA that is operably linked to, for example, regulatory sequences in a promoter region that can affect the expression of such DNA fragments. Such additional fragments may include promoter and terminator sequences, and may optionally include one or more origins of replication, one or more selection markers, enhancers, polyadenylation signals, etc. Expression vectors are generally derived from plasmid or viral DNA, or may contain elements of both. Thus, expression vector refers to a recombinant DNA or RNA construct, such as a plasmid, a bacteriophage, a recombinant virus, or other vectors, which leads to the expression of cloned DNA when introduced into an appropriate host cell. Suitable expression vectors are well known to those skilled in the art, and include expression vectors that can be replicated in eukaryotic cells and/or prokaryotic cells as well as expression vectors that remain free or are integrated into the genome of the host cell. In one embodiment, when the compound contains genetically encoded amino acid residues, the invention further provides a nucleic acid (which may be DNA or RNA) encoding the compound, a vector containing such nucleic acid, as well as a host cell containing such nucleic acid or expression vector.

As used herein, the term "treatment" includes inhibiting, slowing, stopping, or reversing the progress or severity of existing symptoms of patients. Therefore, treatment includes prevention, treatment, and/or cure. Prevention refers to preventing the underlying disease and/or preventing the deterioration of symptoms or the development of the disease.

As used herein, "efficacy" means an effect caused by an individual's treatment that changes, usually improves or ameliorates the symptoms of the disease or disease condition, or cures the disease or disease condition.

As used herein, "therapeutically effective amount" or "therapeutically effective dose" refers to an amount of a substance, compound, material, or composition containing a compound that is at least sufficient to produce a therapeutic effect after administration to a subject. Therefore, it is the amount necessary to prevent, cure, ameliorate, block or partially block the symptoms of a disease or condition.

As used herein, "prophylactically effective amount" or "prophylactically effective dose" refers to the amount of a substance, compound, material, or composition containing a compound that will have the desired prophylactic effect when administered to a subject, for example, to prevent or delay occurrence or recurrence of disease or symptom, reduce the possibility of the occurrence or recurrence of disease or symptoms. Completely prophylactically effective doses do not have to occur through the administration of one dose, and may only occur after the administration of a series of doses. Therefore, the prophylactically effective amount can be administered in one or more applications.

As used herein, the term "patient" refers to a mammal, such as a human.

Holst (Holst, JJ Physiol. Rev. 2007, 87, 1409) and Meier (Meier, JJ Nat. Rev. Endocrinol. 2012, 8, 728) describe GLP-1 receptor agonists, such as GLP-1, liraglutide and exendin-4.

Certain compounds of the present invention are generally effective over a wide dosage range. For example, the dose administered once a week may be in the range of about 0.05 to about 30 mg per person per week. Certain compounds of the invention can be administered daily. In addition, certain compounds of the present invention can be administered once a week.

It should be understood that the therapeutic agent according to the embodiments will be administered together with pharmaceutically acceptable carriers, excipients, and other agents which are incorporated into the formulation to provide improved transfer, delivery, tolerance, etc. Many suitable formulations can be found in the pharmacopoeia known to all medicinal chemists: Remington's Pharmaceuticals (15th edition, Mack Publishing, Easton, Pa. (1975)), in particular Chapter 87 by Blaug and Seymour. These formulations include, for example, powders, pastes, ointments, gels, waxes, oils, lipids, lipid-containing (cationic or anionic) carriers (such as Lipofectin^{™}), DNA conjugates, anhydrous slurries, oil-in-water and water-in-oil emulsions, polyethylene glycols (polyethylene glycols of various molecular weights) emulsion, semi-solid gels, and semi-solid mixtures containing polyethylene glycol. Any of the foregoing mixtures may be suitable for treatment or therapy according to the present invention, provided that the active ingredients in the formulation are not inactivated by the formulation and the formulation is physiologically compatible and tolerates the route of administration.

As used herein, the term "pharmaceutically acceptable carrier" is intended to include any and all solvents, dispersion media, coatings, antibacterial and antifungal agents, isotonic and absorption delaying agents, etc. that are compatible with drug administration. Suitable carriers are described in the latest edition of Remington's Pharmaceutical Sciences, which is a standard bibliography in the art, which is incorporated herein by reference. Preferred examples of such carriers or diluents include, but are not limited to water, saline, Ringer's solution, glucose solution, and 5% human serum albumin. Liposomes and non-aqueous carriers, such as immobilized oil, can also be used. The use of such media and agents for pharmaceutically active substances is well known in the art.

The preparations to be used for clinical in vivo administration must be sterile. This can be easily achieved by filtration through a sterile filter.

The compounds of the present invention can react with a variety of inorganic or organic acids to form pharmaceutically acceptable acid addition salts. Pharmaceutically acceptable salts and common methods for preparing them are well known in the art. Refer to, for example, Handbook of Pharmaceutical Salts: Properties, Selection, and Use. Second revision (Wiley-VCH, 2011); S.M. Berge et al. "Pharmaceutical Salts", Journal of Pharmaceutical Sciences, Vol. 66, No. 1, January 1977. Commonly used pharmaceutically acceptable salts include trifluoroacetate, phosphate, acetate, citrate, hydrochloride, etc.

The compounds of the present invention can react with a variety of inorganic or organic bases to form pharmaceutically acceptable salts. Commonly used pharmaceutically acceptable salts include sodium salt, potassium salt, ammonium salt, etc.

The pharmaceutical compositions of the embodiments are formulated to be compatible with their intended routes of administration. Examples of administration routes include parenteral, such as intravenous, intradermal, subcutaneous, oral (e.g. inhalation), transdermal (e.g. topical), transmucosal, and rectal administration. Solutions or suspensions for parenteral, intradermal or subcutaneous administration may include the following components: sterile diluents for injection, such as water, saline solutions, fixed oils, polyethylene glycols, glycerin, propylene glycol or other synthetic solvents; antibacterial agents, such as benzyl alcohol, methyl 4-hydroxybenzoate, phenol, or m-cresol; antioxidants, such as ascorbic acid or sodium bisulfite; chelating agents, such as ethylenediaminetetraacetic acid (EDTA); buffers, such as acetates, citrates or phosphates, and agents that regulate osmotic pressure, such as sodium chloride or dextrose. pH can be adjusted with acids or bases, such as hydrochloric acid or sodium hydroxide. The parenteral preparation can be packaged in ampoules, penicillin vials, disposable syringes, multi-dose glass or plastic vials or injection pens. There are two major types of injection pens. One is a disposable pre-filled pen, which contains medicine and can be thrown away after use without changing the cartridge; the other is a more commonly used durable injection pen. It includes an injector and a medicine cartridge. This type of injection pens can be used again after replacing the cartridge.

Pharmaceutical compositions suitable for injection include sterile aqueous solutions (herein water-soluble) or dispersions and sterile powders for immediate preparation of sterile injectable solutions or dispersions. For intravenous administration, suitable carriers include saline, bacteriostatic water, Cremophor EL^{™} (BASF, Parsippany, N.J.) or phosphate buffered saline (PBS). In all cases, the compositions must be sterile, and their fluidity should make injection easy. The compositions must be stable under manufacturing and storage conditions and must be able to prevent contamination by microorganisms such as bacteria and fungi. The carrier can be a solvent or dispersion medium containing, for example, water, ethanol, polyol (e.g. glycerin, propylene glycol, and liquid polyethylene glycol, etc.), and suitable mixtures thereof. A desired particle size can be maintained in the case of dispersion, for example, by using a coating such as lecithin, and, proper fluidity can be maintained by using a surfactant. Prevention of microorganisms can be achieved by various antibacterial and antifungal agents such as parabens, chlorobutanol, phenol, m-cresol, ascorbic acid, thimerosal, etc. In many cases, it is preferable to include isotonic agents in the compositions, such as sugars, polyols (e.g. mannitol, sorbitol), and sodium chloride. Prolonged absorption of the composition for injection can be achieved by including in the composition an agent that delays absorption, such as aluminum monostearate and gelatin.

If desired, a sterile injectable solution can be prepared by incorporating a required amount of the compound of the present invention in a suitable solvent with one or a combination of ingredients listed above (as required), followed by filtration and sterilization. In general, dispersions are prepared by incorporating the compounds of this invention into sterile vehicles that contain a dispersion medium and those required other ingredients listed above. In the case of sterile powders for the preparation of sterile injectable solutions, the methods of preparation are vacuum drying and freeze-drying of the powders, which contains the active ingredients and any additional desired ingredients from the aforementioned sterile filtered solutions of these ingredients.

For administration by inhalation, the compound is delivered in the form of an aerosol spray from a pressurized container or dispenser or nebulizer containing a suitable propellant gas such as carbon dioxide.

It can also be administered systemically by transmucosal or transdermal methods. For transmucosal or transdermal administration, penetrants suitable for penetrating barriers are used in the formulations. Such penetrants are generally known in the art and include, for example, detergents, bile salts and fusidic acid derivatives for transmucosal administration. Transmucosal administration can be achieved through the use of nasal sprays or suppositories. For transdermal administration, one or more of the compounds of the present invention may be formulated as plasters, ointments, gels, or creams as generally known in the art.

The compounds can also be prepared in the form of suppositories (e.g. with conventional suppository bases such as cocoa butter or other glycerides) or retentive enemas for transrectal delivery.

In one embodiment, the compounds of the present invention can be prepared with carriers that prevent their rapid elimination by the body, such as sustained/controlled release formulations, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparing such formulations are obvious to those skilled in the art.

For instance, these active ingredients can be encapsulated in microcapsules prepared, for example, by coacervation technique or by interfacial polymerization, for example, hydroxymethyl cellulose or gelatin microcapsules and poly(methyl methacrylate) microcapsules, respectively, in colloidal drug delivery systems (e.g. liposomes, albumin microspheres, microemulsions, nanoparticles, and nanocapsules) or in macroemulsions.

Sustained release formulations can be prepared. Examples of suitable sustained-release preparations include semipermeable matrices of solid hydrophobic polymers containing the compounds of the present invention, and the matrices are in the form of shaped articles such as films or microcapsules. Examples of sustained-release matrices include polyester, hydrogel (e.g. poly(2-hydroxyethyl-methyl propionate), or poly(vinyl alcohol)), polylactide (US Patent No. 3,773,919), copolymers of L-glutamic acid and γ-ethyl-L-glutamate, non-degradable ethylene-vinyl acetate, degradable lactic acid-glycolic acid copolymers such as LUPRON DEPOT^{™} (injection microspheres composed of lactic acid-glycolic acid copolymer and leuprorelin acetate), and poly-D-(-)-3-hydroxybutyric acid. Although polymers made from, for example, ethylene-vinyl acetate and lactic acid-glycolic acid can release molecules for more than 100 days some hydrogels release proteins for a shorter time. Polylactic acid (PLA) and polylactic acid-glycolic acid copolymer (PLGA) have been the focus of research in recent years. In addition, there are albumin microspheres, chitosan microspheres, gelatin microspheres, etc.

Liposomal suspensions can also be used as pharmaceutically acceptable carriers. These can be prepared according to methods known to those skilled in the art, such as description in US Patent No. 4,522,811.

It is especially advantageous to formulate parenteral compositions in the form of dosage unit for ease of administration and uniformity of dosage. As used herein, a dosage unit refers to a physically separable unit which is suitable as a unit dose for the subject to be treated; Each unit contains a predetermined amount of one or more of the compounds of the present invention calculated in combination with the required pharmaceutical carrier to produce the desired therapeutic effects. The specifications of the dosage unit of the embodiment are indicated by and directly dependent on: the unique characteristics of the compounds of the present invention and the specific therapeutic effects to be achieved, and the limitations inherent in the formulations of such compounds of the present invention for personalized treatment.

The pharmaceutical composition can be placed in a container, package, or dispenser together with instructions for administration.

The present invention provides a method for treating type 2 diabetic patients, comprising administering an effective amount of a compound or a pharmaceutically acceptable salt of the present invention to patients in need of such treatment. The present invention also provides a method for treating type 2 diabetic patients, comprising administering to patients in need of such treatment an effective amount of a compound or a pharmaceutically acceptable salt of the present invention, wherein the administration is subcutaneous. The present invention also provides a method for treating type 2 diabetic patients, comprising administering an effective amount of a compound or a pharmaceutically acceptable salt of the present invention to patient in need of such treatment, and administering the effective amount simultaneously, separately, or sequentially with one or more other active ingredients. In one embodiment, the other one or more active ingredients are currently available oral glucose-lowering drugs that are considered the standard of care prior to administration (as determined by industry guidelines from, for example, the American Diabetes Association).

The present invention also provides methods for treating or preventing the following diseases or conditions: impaired glucose tolerance (IGT), hyperglycemia, type 1 diabetes, type 2 diabetes, obesity, metabolic syndrome, and neurodegenerative diseases, especially for delaying or preventing disease progression in type 2 diabetes, delaying progression from impaired glucose tolerance to type 2 diabetes; delaying progression from type 2 diabetes to insulin-requiring diabetes; treating metabolic syndrome, regulating appetite, inducing satiety, reducing food intake, increasing energy expenditure, treating obesity or preventing overweight; preventing weight rebound after successful weight loss; treating diseases or conditions related to overweight or obesity; treating bulimia; treating binge eating; treating dyslipidemia, atherosclerosis, hypertension, coronary heart disease, β-blocker poisoning; treating non-alcoholic fatty liver disease (NAFLD) (can be divided into simple fatty liver (SFL), non-alcoholic Steatohepatitis (NASH) and its associated cirrhosis); inhibiting gastrointestinal motility, for use in conjunction with gastrointestinal investigation techniques such as X-ray, CT, and NMR scanning. The method includes administering an effective amount of a compound or a pharmaceutically acceptable salt or solvate of the present invention to patients in need of such treatment, and administering the effective amount simultaneously, separately, or sequentially with one or more other active ingredients.

Further preferred medical uses include treatment or prevention of degenerative disorders, particularly neurodegenerative disorders such as Alzheimer's disease, Parkinson's disease, Huntington's disease, ataxia (e.g spinocerebellar ataxia), Kennedy disease, myotonic dystrophy, Lewy body dementia, multi-systemic atrophy, amyotrophic lateral sclerosis, primary lateral sclerosis, spinal muscular atrophy, prion-associated diseases (e.g. Creutzfeldt-Jacob disease), multiple sclerosis, telangiectasia, Batten disease, corticobasal degeneration, subacute combined degeneration of spinal cord, Tabes dorsalis, Tay-Sachs disease, toxic encephalopathy, infantile Refsum disease, Refsum disease, neuroacanthocytosis, Niemann-Pick disease, Lyme disease, Machado-Joseph disease, Sandhoff disease, Shy-Drager syndrome, wobbly hedgehog syndrome, proteopathy, cerebral β-amyloid angiopathy, retinal ganglion cell degeneration in glaucoma, synucleinopathies, tauopathies, frontotemporal lobar degeneration (FTLD), dementia, cadasil syndrome, hereditary cerebral hemorrhage with amyloidosis, Alexander disease, seipinopathies, familial amyloidotic neuropathy, senile systemic amyloidosis, serpinopathies, AL (light chain) amyloidosis (primary systemic amyloidosis), AH (heavy chain) amyloidosis, AA (secondary) amyloidosis, aortic medial amyloidosis, ApoAI amyloidosis, ApoAII amyloidosis, ApoAIV amyloidosis, familial amyloidosis of the Finnish type (FAF), lysozyme amyloidosis, fibrinogen amyloidosis, dialysis amyloidosis, inclusion body myositis/myopathy, cataracts, retinitis pigmentosa with rhodopsin mutations, medullary thyroid carcinoma, cardiac atrial amyloidosis, pituitary prolactinoma, hereditary lattice corneal dystrophy, cutaneous lichen amyloidosis, Mallory bodies, corneal lactoferrin amyloidosis, pulmonary alveolar proteinosis, odontogenic (Pindborg) tumor amyloid, cystic fibrosis, sickle cell disease or critical illness myopathy (CIM). Further medical uses include the treatment of bone-related disorders, such as osteoporosis or osteoarthritis, for which increased bone formation and decreased bone resorption may be beneficial.

### ACRONYMS

### Protective Groups:

Aloc or AOC, allyloxycarbonyl; Bom, benzyloxymethyl; 2-Br-Z, 2-bromobenzyloxycarbonyl; tBu, tert-butyl; Bz, benzoyl; Bzl, benzyl; Boc, tert-butoxycarbonyl; CHO, formyl; cHx, cyclohexyl; Cbz or Z, benzyloxycarbonyl; 2-Cl-Z, 2-chlorobenzyloxycarbonyl; Fm, 9-fluorenylmethyl; Fmoc, 9-fluorenylmethoxycarbonyl; Mtt, 4-methyltrityl; Pmc, (2,2,5,7,8-pentamethylchroman-6-sulphonyl; Tos, 4-toluenesulphonyl; Trt, triphenylmethyl; Xan, xanthyl.

### Reagents and Solvents:

ACN : acetonitrile; BOP: benzotriazol-1-yloxytris (dimethylamino) phosphoronium hexafluoro phosphate; DCC: N, N'-Dicyclohexylcarbodiimide; DCM: dichloromethane; DEPBT: 3-(Diethoxyphosphoryloxy)-1,2,3-benzotriazin-4(3H)-one; DIC: N, N'-Diisopropylcarbodiimide; DIPEA (or DIEA): diisopropylethylamine; DMF: N,N-dimethylformamide; DMSO: dimethylsulfoxide; EDC or EDCI: 1-ethyl-3-(3-dimethylaminopropyl)carbodiimide; EtOAc: ethyl acetate; HATU: 1-[Bis (dimethylamino)methylene]-1H-1,2,3-triazolo[4,5-b]pyridinium 3 -oxide hexafluorophosphate; HBTU: O-(1H-benzotriazole-1-yl)-N,N,N',N'-tetramethyluronium hexafluorophosphate; HOAT: 1-Hydroxy-7-azabenzotriazole; HOBT: 1-hydroxybenzotriazole; Cl-HOBT: 6-chloro-1-hydroxybenzotriazole; NMM: N-methylmorpholine; NMP: N-methylpyrrolidinone; Su: succinimide; TBTU: 2-(1H-Benzotriazole-1-yl)-1,1,3,3-tetramethylaminium tetrafluoroborate; TEA: triethylamine; TFA: trifluoroacetic acid; TIS: triisopropylsilane; TMP: 2,2,6,6-Tetramethylpiperidine.

### PEPTIDE CHEMICAL SYNTHESIS METHODS

Solid phase peptide synthesis is a well-developed methodology, which can be referenced in the literature such as R.C. Sheppard, Solid Phase Peptide Synthesis. A Practical Approach, Oxford-IRL Press, New York, 1989.

Linear peptides are made with Boc solid phase peptide synthesis or Fmoc solid phase peptide synthesis. If Fmoc chemistry is used to synthesize a peptide with a C-terminal carboxyl group, Wang resin is usually used; a peptide with a C-terminal amide is usually made with Rink amide resin (including Rink amide-AM resin, Rink amide-MBHA resin, and other similar resins). If Boc chemistry is used to synthesize a peptide with a C-terminal carboxyl group, Pam resin is usually used; a peptide with a C-terminal amide is usually made with MBHA resin. Commonly used peptide coupling reagent and activator are DIC and HOBT, and other optional peptide bond coupling reagents include EDC, BOP, HBTU, DEPBT, TBTU etc. Based on reaction difficulty, 1.1∼10 equivalents of amino acids can be used in the systhesis. Coupling reaction is usually 15mins~24 hour. Peptides can be synthesized manually, or they can be synthesized using a peptide solid phase synthesizer.

Fmoc protecting group is removed with 20% piperidine/DMF. Boc protecting group is removed with TFA. Peptide bond condensation reaction is monitored with Ninhydrin (2,2-Dihydroxyindane-1,3-dione) reagent.

For solid phase synthesis, either resins preloaded with the C-terminal amino acids or resins not loaded with amino acids can be used.

The method of loading the first amino acid on Rink amide resin can refer to the usual practice in the industry. A common method is briefly described as follows: weigh an appropriate amount of resin, remove the Fmoc protecting group with 20% piperidine/DMF in a solid-phase synthesis tube (15 mL/g resin, 30 minutes X 2), and wash the resin with DMF. Weigh Fmoc amino acid, HATU, and HOAT equivalent to 5 times the moles of the amino group on the resin, and NMM equivalent to 10 times the moles of the amino group on the resin, add DMF to dissolve and mix the reagents, and transfer to solid phase synthesis tube. After overnight reaction, the resin is washed with DMF. Add 1:1 acetic anhydride/pyridine (v/v) to the solid phase synthesis tube, evacuate after 30 minutes, and wash the resin with DMF. The first amino acid is loaded.

When Fmoc solid phase peptide synthesis method is employed, the commonly used amino acids and protecting groups are as follows:
Fmoc-Cys(Trt)-OH, Fmoc-Asp(OtBu)-OH, Fmoc-Glu(OtBu)-OH, Fmoc-His(Trt)-OH, Fmoc-Lys(Boc)-OH, Fmoc-Asn(Trt)-OH, Fmoc-Gln(Trt)-OH, Fmoc-Arg(Pmc)-OH, Fmoc-Ser(tBu)-OH, Fmoc-Thr(tBu)-OH, Fmoc-Trp(Boc)-OH, Fmoc-Tyr(tBu)-OH
Appropriately protected structural units are used in the synthesis process, such as the above-mentioned standard amino acids, Fmoc-8-amino-3,6-dioxaoctanoic acid (CAS No. 166108-71-0), and Fmoc-Glu-OtBu (CAS No. 84793-07-7). The introduction of fatty acid moieties can be achieved using structural units such as, but not limited to, eicosanedioic acid mono-t-butyl ester. After each coupling step, the unreacted peptide intermediate can be capped with acetic anhydride (10 equivalents) and excess collidine (20 equivalents).

After solid phase Fmoc chemical synthesis of peptides, the commonly used cleavage reagent is TFA. Place the dry resin in a flask, add an appropriate amount of cleavage solution (10-25mL/g resin) containing 90:4:2:2:2 (v/v) trifluoroacetic acid: triisopropylsilane: 1,2-ethanedithiol: water: thioanisole, cover with lid, and perform intermittent rotary shaking at room temperature. After 2 hours, the resin is filtered with suction, and washed with new TFA 2-3 times. The filtrate is combined, and ice-cooled ether is added dropwise. Finally, the precipitated crude peptide is collected by centrifugation.

When Boc solid-phase peptide synthesis method is employed, commonly used amino acids and protecting groups are as follows:
Boc-Cys(4-MeBzl)-OH, Boc-Asp(OcHx)-OH, Boc-Glu(OcHx)-OH, Boc-His(Bom)-OH, Boc-Lys(2-Cl-Z)-OH, Boc-Asn(Xan)-OH, Boc-Arg(Tos)-OH, Boc-Ser(Bzl)-OH, Boc-Thr(Bzl)-OH, Boc-Trp(CHO)-OH and Boc-Tyr(2-Br-Z)-OH
If the side chain amino group of lysine is used for lactam synthesis or acylation, the side chain amino group of lysine can be protected with allyloxycarbonyl (aloc) or Fmoc. If the side chain carboxyl group of aspartic acid or glutamic acid is used for lactam synthesis or acylation reaction, the carboxyl group should be converted to allyl ester or 9-fluorenylmethyl protection, such as Boc-Glu(OAll)-OH and Boc -Glu(Ofm)-OH.

After solid-phase Boc chemical synthesis of peptides, PAM and MBHA resins are usually cleaved with HF, 5 ml of HF per 0.1 mmol of resin, and the reagents such as p-cresol, p-mercaptophenol or anisole are added. The mixture is stirred for 1 hour under ice bath conditions. After HF is vacuum-dried, the peptide is precipitated with ether on ice, the precipitate is collected by centrifugation, separated and purified by HPLC, and then lyophilized to obtain the final product.

### Purification

A crude peptide is dissolved in a suitable mixture of water and acetonitrile (e.g. water/acetonitrile 3:1) and purified by reversed-phase preparative HPLC (e.g. AKTA purifier, Shimadzu LC-20AR). Columns with different packings and sizes are selected according to the quantity and polarity of peptides, such as C8 or C18 semi-preparative or preparative columns. Buffer A is a 0.1% TFA aqueous solution, and buffer B is 0.1% TFA acetonitrile solution. The gradient of buffer B is increased to elute peptides, and the relevant fractions are checked by analytical HPLC. A ZORBAX 300 SB-C18 (4.6 X 250mm, 5µM) column was used, buffer A was 0.1% TFA aqueous solution, and buffer B was 0.1% TFA, 100% acetonitrile. The flow rate was 1ml/min with UV detection at 210nm. Fractions containing the peptides of interest of acceptable purity were combined and lyophilized to obtain the peptides as white solids. The products were stored separately in glass vials.

### PREPARATION (1)

The compounds of the present invention are linear peptides. Each amino acid can be coupled step by step from the C-terminus to the N-terminus of the peptide sequence to obtain the peptide backbone. The process is as follows: first, an amino acid whose amino group is protected by a blocking group is covalently attached to the solid phase carrier, and the amino protecting group of the first amino acid is removed, so that the first amino acid is attached to the solid phase carrier. Then the carboxyl group of the second amino acid whose amino group is protected is activated and reacts with the amino group of the first amino acid that has been attached to the solid phase carrier to form a peptide bond, in this way, a dipeptide with a protective group is generated on the solid phase carrier. Repeat the above peptide bond formation reaction to extend the peptide chain from the C-terminus to the N-terminus until the desired peptide chain is generated. Finally, the peptide is cleaved and separated from the resin, purified and lyophilized to obtain the peptide product.

Some compounds of the present invention are conjugated with long-acting groups or modifying groups through the side chain amino groups or thiol groups of amino acids whose side chains contain amino groups or thiol groups. Take compound 12 as an example to illustrate the synthetic route and method.

The synthesis of compound 12 includes the following steps:
Step A: Coupling Fmoc-Lys(PG)-OH and resin to obtain Fmoc-Lys(PG)-resin, PG is a protecting group for the amino group of lysine side chain;
Step B: Fmoc-Lys(PG)-resin is coupled with amino acids or amino acid derivatives, through the first progressive coupling, to obtain the first peptide resin with the sequence shown in the main peptide chain of compound 12;
Step C: Remove the side chain protecting group PG of C-terminal Lys in the first peptide resin whose sequence is shown in the main peptide chain of compound 12, and couple, through the second progressive coupling, 2-(2-(2-aminoethoxy) ethoxy) acetic acid, 2-(2-(2-aminoethoxy) ethoxy) acetic acid, γ-Glu, and eicosanedioic acid to obtain the second peptide resin;
Step D: The second peptide resin is cleaved, and the peptide is purified and lyophilized to obtain compound 12.

Preferably, Rink Amide resin (including Rink Amide-AM resin, Rink Amide-MBHA resin, and other similar resins ) can be used in step A. Preferably, the side chain protecting group of Lys is aloc, Mmt, Mtt, Dde, or ivDde.

Preferably, the coupling reagents are selected from DIC+A, B+A+C or B+C, wherein A is selected from HOAt or HOBt, B is selected from EDC, HATU, BOP, PyBOP, PyAOP, HBTU, TBTU or DEPBT, C is selected from DIPEA, TEA, NMM or TMP.

For example, the coupling agent used in the first progressive coupling in step B and the second progressive coupling in step C includes a condensing agent and a reaction solvent. The condensing agent may be a mixed solution of DIC and HOBt, or a mixed solution of PyBOP, HOBt and DIEA, or a mixed solution of HATU, HOAt and DIEA, or a mixed solution of DEPBT and DIEA, or a mixed solution of HBTU and DIEA, and the reaction solvent is one or more mixed solutions of DMF, DCM, NMP, or DMSO.

In the first progressive coupling in Step B, according to the sequence of compound 12, Fmoc-Pro-OH, Fmoc-Pro-OH, Fmoc-Pro-OH, Fmoc-Ala-OH, Fmoc-Gly-OH, Fmoc-Ser(tBu)-OH, Fmoc-Ser(tBu)-OH, Fmoc-Pro-OH, Fmoc-Gly-OH, Fmoc-Gly-OH, Fmoc-Ala-OH, Fmoc-Leu-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Lys(Boc)-OH, Fmoc-Glu(OtBu)-OH, Fmoc-Leu-OH, Fmoc-Ala-OH, Fmoc-Ile-OH, Fmoc-Ser(tBu)-OH, Fmoc-Leu-OH, Fmoc-Asp(OtBu)-OH, Fmoc-Ser(tBu)-OH, Fmoc-Thr(tBu)-OH, Fmoc-Phe-OH, Fmoc-Thr(tBu)-OH, Fmoc-Gly-OH, Fmoc-Glu(OtBu)-OH, Fmoc-Aib-OH, Boc-Tyr(tBu)-OH were coupled stepwise.

When PG is aloc, the deprotection reagents used in step C are 1-20 equivalents of morpholine (or 1-20 equivalents of phenylsilane instead of morphine) and 0.05-3 equivalents of Pd(PPh₃)₄. Preferably, aloc protective group is removed with 5-10 equivalents of morpholine (or 5-10 equivalents of phenylsilane instead of morphine) and 0.1-0.3 equivalents of Pd(PPh₃)₄. The protective group removal reaction can be performed twice, 10-30 minutes each time, and CH₂Cl₂ is preferably used as the solvent.

Another method for removing Aloc protective group is to use a catalytic amount of tetrakis(triphenylphosphine)palladium (0) and 37:2:1 ratio of DCM, glacial acetic acid, and NMM (15mL/g resin) in an argon atmosphere. The reaction is stirred at room temperature for 2 hours. After the reaction, each gram of resin is washed with 0.5% DIPEA/DMF (10 mL), 0.5% sodium diethyldithiocarbamate/DMF (3×10 mL), 1:1 DCM: DMF (5×10 mL).

When PG is Dde or ivDde, the reagent used to remove the protective group in step C is a hydrazine hydrate/DMF mixed solution. Prepare 2% (w/v) hydrazine hydrate in DMF solution (25mL/g resin), add the resin, drain it after 5 minutes, and wash the resin with DMF. The process of deprotection with 2% hydrazine hydrate/DMF and DMF washing is repeated 3 times.

When PG is Mmt or Mtt, the reagent used to remove the protective group in step C is 1-2% TFA/DCM mixed solution.

In the second progressive coupling in step C, according to the sequence of compound 12, Fmoc-8-amino-3,6-dioxaoctanoic acid (CAS No. 166108-71-0), Fmoc-8-amino-3,6-dioxaoctanoic acid, Fmoc-Glu-OtBu (CAS No.84793-07-7) and eicosanedioic acid mono-t-butyl ester HOOC-(CH₂)₁₈-COOtBu are coupled stepwise.

The reagents used for the cleavage in step D include TFA and one, two or more compound mixtures of PhSMe, PhOMe, EDT, H₂O, TIS and PhOH. For example, the reagents used for cleavage are a mixture of TFA, anisole, dimethyl sulfide and EDT, and the volume ratio of TFA, anisole, dimethyl sulfide and EDT is 90:5:3:2. Preferably, the reagent used for cleavage is a mixture of TFA, H₂O and TIS, and the volume ratio is 90:2.5:2.5.

### PREPARATION ( 2 )

Some compounds of the present invention are conjugated with long-acting groups or modifying groups through the side chain amino groups or thiol groups of amino acids whose side chains contain amino groups or thiol groups. Each amino acid is coupled step by step to the compound in the order of the peptide sequence from the C-terminal to the N-terminus, until the amino acid residue X that needs to be conjugated to a long-acting group or a modifying group is coupled. Under the premise of not affecting the protecting group of α-amino group of amino acid residue X, the side chain protecting group of amino acid residue X is removed to complete the synthesis of the long-acting group or modifying group. Then the protecting group of α-amino group of amino acid residue X is removed and each remaining amino acid of the peptide backbone is sequentially and stepwise coupled to obtain a complete peptide. Finally, the peptide was cleaved and separated from the resin, purified, and lyophilized to obtain the peptide product.

Take compound 12 as an example to illustrate the synthetic route and method. The synthesis of compound 12 includes the following steps:
Step A: Coupling PG1-Lys(PG2)-OH and resin to obtain PG1-Lys(PG2)-resin, PG1 is a protecting group for α-amino group of lysine; PG2 is a protecting group for the amino group of lysine side chain;
Step B: Remove the side chain protecting group PG2 of Lys in PG1-Lys (PG2)-resin, and couple, through the second progressive coupling, 2-(2-(2-aminoethoxy) ethoxy) acetic acid, 2-(2-(2-amino ethoxy) ethoxy) acetic acid, γ-Glu and eicosanedioic acid to obtain the third peptide resin;
Step C: Remove the protecting group PG1 of α-amino group of Lys, through the first progressive coupling, to obtain the fourth peptide resin with a sequence as shown in compound 12;
Step D: The fourth peptide resin is cleaved, and the peptide is purified and lyophilized to obtain compound 12.

Preferably, Rink Amide resin (including Rink Amide-AM resin, Rink Amide-MBHA resin and other similar resins) is used in step A.

Preferably, the side chain protecting group PG2 of Lys is Fmoc, aloc, Mmt, Mtt, Dde or ivDde. The protecting group PG1 of the α-amino group of Lys is aloc, Mmt, Mtt, Dde or ivDde. PG1 and PG2 must be orthogonal. Examples of PG1-Lys(PG2)-OH used in compounds 12 synthesis include Dde-Lys(Fmoc)-OH, Alloc-Lys(Fmoc)-OH, etc. The methods for protecting group removal can refer to Preparation (1), Examples, or literature on solid phase synthesis of polypeptides.

Preferably, the coupling reagents are selected from DIC+A, B+A+C or B+C, wherein A is selected from HOAt or HOBt, B is selected from EDC, HATU, BOP, PyBOP, PyAOP, HBTU, TBTU or DEPBT, C is selected from DIPEA, TEA, NMM or TMP.

Through the second progressive coupling in step B, according to the sequence of compound 12, Fmoc-8-amino-3,6-dioxaoctanoic acid (CAS No. 166108-71-0), Fmoc-8-amino-3,6-dioxaoctanoic acid, Fmoc-Glu-OtBu (CAS No.84793-07-7), and eicosanedioic acid mono-t-butyl ester HOOC-(CH₂)₁₈-COOtBu were coupled stepwise.

Through the first progressive coupling in Step C, according to the sequence of compound 12, Fmoc-Pro-OH, Fmoc-Pro-OH, Fmoc-Pro-OH, Fmoc-Ala-OH, Fmoc-Gly-OH, Fmoc-Ser(tBu)-OH, Fmoc-Ser(tBu)-OH,Fmoc-Pro-OH, Fmoc-Gly-OH, Fmoc-Gly-OH, Fmoc-Ala-OH, Fmoc-Leu-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Lys(Boc)-OH, Fmoc-Glu(OtBu)-OH, Fmoc-Leu-OH, Fmoc-Ala-OH, Fmoc-Ile - OH, Fmoc-Ser(tBu)-OH, Fmoc-Leu-OH, Fmoc-Asp(OtBu)-OH, Fmoc-Ser(tBu)-OH, Fmoc-Thr(tBu)-OH, Fmoc-Phe-OH, Fmoc-Thr(tBu)-OH, Fmoc-Gly-OH, Fmoc-Glu(OtBu)-OH, Fmoc-Aib-OH, and Boc-Tyr(tBu)-OH were couple stepwise.

Preferably, the reagents and methods used for cleavage in step D are the same as those in Preparation (1).

The present invention also includes new intermediates and methods that can be used to synthesize compounds or pharmaceutically acceptable salts of the present invention thereof. The intermediates and compounds of the present invention can be prepared by various methods known in the art. In particular, the following examples illustrate methods using chemical synthesis. The specific synthetic steps of each route described can be combined in different ways to prepare compounds or their salts of the invention thereof. Reagents and raw materials are readily available to those of ordinary skill in the art.

### EXAMPLES

The invention is further explained with reference to the examples. These examples are by no means intended to limit the scope of the claims in this invention. Those skilled in the art can learn from the content of the present invention, appropriately improve the process parameters, make changes, or appropriately modify and combine the methods and applications of the present invention for implementation and application of the technology of the present invention. All similar substitutions and modifications are obvious to those skilled in the art, and they are all considered to be included in the present invention.

Amino acids and condensation reagents were purchased from GL Biochem (Shanghai) Co., Ltd. Rink Amide resin was purchased from Shangyu Puer Company and Tianjin Nankai Hecheng Science & Technology Co., Ltd.

### Example 1 Synthesis of Compound 58

A pre-loaded Fmoc-Ser(tBu)-Rink Amide resin was used. Fmoc protecting group was removed with 20% piperidine/DMF (2X10 minutes), and the resin was washed 3 times with DMF. Fmoc solid phase peptide synthesis method was used. An amino acid (10 times the molar amount of the pre-loaded amino acid on the resin) was dissolved in DMF, then HOBT and DIC (10 times the molar amount of the amino acid on the resin respectively) were added to the solution. The solution was mixed well before added to the resin, and the coupling was performed at room temperature for 120 minutes. The solution in the solid phase reaction tube was drained, and the resin was washed 3 times with DMF. The coupling of a synthetic unit was thus completed. The same coupling method was used for each synthetic unit from the C-terminus to the N-terminus of the peptide. Amino acid residues were protected by protective groups commonly used in Fmoc solid phase synthesis and listed in the "Peptide Chemical Synthesis Methods" section, "Preparation" section, or other protective groups suitable for this sequence. Tyrosine at the N-terminus of the main peptide chain uses Boc-Tyr(tBu)-OH. Lys24, the 24th amino acid counted from the N-terminus, uses Fmoc-Lys(Dde)-OH.

After synthesis of the main peptide chain, Dde protecting group of Lys24 side chain was removed with 2% hydrazine hydrate/DMF. A 2% (w/v) hydrazine hydrate solution in DMF (25 mL/g resin) was prepared, added to the resin, and drained after 10 minutes. The resin was washed with DMF. The process of deprotection with 2% hydrazine hydrate/DMF and DMF washing was repeated 3 times.

Synthesis of Lys24 side chain substituent uses the structural units Fmoc-8-amino-3,6-dioxoctanoic acid (CAS No. 166108-71-0), Fmoc-Glu -OtBu (CAS No. 84793-07-7), and eicosanedioic acid mono-t-butyl ester HOOC-(CH₂)₁₈-COOtBu. The amount of each structural unit of the side chain substituent is 10 equivalents of the resin amino acid load, and the coupling time per step is 4 hours.

After synthesis of the peptide, the resin was washed with DCM and the cleavage solution containing trifluoroacetic acid: triisopropylsilane: 1,2-ethanedithiol: water: anisole 90:4:2:2:2 (v/v) was added (10-25 mL/g resin). The reaction was agitated at room temperature for 2 hours and the peptide was precipitated with ice-cooled ether. The crude peptide was dissolved in 0.1% TFA, 30% acetonitrile aqueous solution and purified with a semi-preparative or a preparative RP-HPLC, using e.g. Luna C8 30X250mm, 5 µM reverse phase column. Buffer A is 0.1% TFA aqueous solution, and buffer B is 0.1% TFA in acetonitrile. The gradient of buffer B was gradually increased for elution of the peptide, and the correct fractions were combined and lyophilized at low temperature to obtain a white solid. The structure of the peptide was determined by mass spectrometry and amino acid sequencing. In the present invention, all compounds with a serine at the C-terminus can be synthesized according to the method of Example 1.

### Example 2 Synthesis of Compound 15

A pre-loaded Fmoc-Lys (Aloc)-Rink Amide resin (e.g., 0.29 mmole/g) was used. The coupling method described in Example 1 was used for each synthetic unit from the C-terminus to the N-terminus of the peptide. Amino acid residues other than C-terminal lysine and Boc-Tyr(tBu)-OH at the N-terminus were protected by protective groups commonly used in Fmoc solid phase synthesis and listed in the "Peptide Chemical Synthesis Methods" section,

"Preparation" section, or other protective groups suitable for this sequence. After the main peptide chain was synthesized allyloxycarbonyl group at the side chain of C-terminal lysine was removed by using tetrakis(triphenylphosphine)palladium(0) and 37:2:1 ratio of DCM, glacial acetic acid and NMM (15mL/g resin). The reaction was stirred for 2 hours under argon atmosphere at room temperature. Tetrakis(triphenylphosphine)palladium(0) could be used in a catalytic amount to 1 equivalent. After the reaction, each gram of resin was washed with 0.5% DIPEA/DMF (10 mL), 0.5% sodium diethyldithiocarbamate/DMF (3x10 mL), 1:1 DCM:DMF (5x10 mL). The Aloc protective group could also be removed with 5-10 equivalents of morpholine (or 5-10 equivalents of phenylsilane) and 0.1-0.3 equivalents of Pd(PPh₃)₄. The protective group removal reaction could be carried out twice, 30 minutes each time, using CH₂Cl₂ as solvent. After side chain Aloc protective group removal, methods and steps for synthesis of this lysine side chain substituent refer to Example 1.

Another method was to use a pre-loaded Fmoc-Lys (ivDde)-Rink Amide resin. The method for synthesizing the main peptide chain was the same as the method using Fmoc-Lys(Aloc) Rink Amide resin in the previous paragraph. After synthesis of the main peptide chain, ivDde protecting group of C-terminal lysine side chain was removed with 2% hydrazine hydrate/DMF. A 2% (w/v) hydrazine hydrate solution in DMF (25 mL/g resin) was prepared, added to the resin, and drained after 10 minutes. The resin was washed with DMF. The process of deprotection with 2% hydrazine hydrate/DMF and DMF washing was repeated 3 times. The methods and steps for synthesis of this lysine side chain substituent are similar to those in Example 1.

When PG is Mmt or Mtt, the reagent used to remove the protective group in Step C is a 1-2% TFA/DCM mixture.

In the present invention, the compounds with a C-terminal lysine whose side chain amino group is linked to a long-acting group can be synthesized according to the methods described in Example 2, or can be synthesized according to the synthesis routes and methods of compound 12 described in Preparation sections.

### Example 3 Synthesis of Compound 11

A pre-loaded Fmoc-Cys(Trt)-Rink Amide resin was used. The synthesis methods and steps were similar to those in Example 1. In the present invention, all compounds with a C-terminal cysteine can be synthesized according to the methods described in Example 3.

### Example 4 Synthesis of Compound 65

A pre-loaded Fmoc-Ala-Rink Amide resin was used. The synthesis methods and steps were similar to those in Example 1. In the present invention, all compounds with an alanine at the C-terminus can be synthesized according to the methods described in Example 4.

### Example 5 Synthesis of Compound 279

A pre-loaded Fmoc-Gly-Rink Amide resin was used. The synthesis methods and steps were similar to those in Example 1. In the present invention, all compounds with a glycine at the C-terminus can be synthesized according to the methods described in Example 5.

### Example 6 Synthesis of Compound 356

A pre-loaded Fmoc-Pro-Rink Amide resin was used. The synthesis methods and steps were similar to those in Example 1. In the present invention, all compounds with a proline at the C-terminus can be synthesized according to the methods described in Example 6.

Table 1 lists calculated molecular weight and measured molecular weight of some compounds in the present invention.

**Table 1**

| Compound | Calculated molecular weight | Observed molecular weight | Compound | Calculated molecular weight | Observed molecular weight |
|---|---|---|---|---|---|
| Compound 1 | 5077.8 | 5077.6 | Compound 2 | 4990.7 | 4989.6 |
| Compound 3 | 5076.8 | 5076.7 | Compound 4 | 4989.8 | 4988.6 |
| Compound 5 | 4963.6 | 4962.5 | Compound 6 | 4948.7 | 4947.8 |
| Compound 7 | 5038.8 | 5037.9 | Compound 8 | 5020.8 | 5019.6 |
| Compound 9 | 4933.7 | 4933.0 | Compound 10 | 4940.8 | 4939.7 |
| Compound 11 | 4951.8 | 4950.7 | Compound 12 | 4876.7 | 4875.9 |
| Compound 13 | 4963.8 | 4962.5 | Compound 14 | 4977.8 | 4977.1 |
| Compound 15 | 4890.7 | 4889.8 | Compound 16 | 4891.7 | 4890.8 |
| Compound 17 | 4905.7 | 4905.6 | Compound 18 | 4557.3 | 4556.4 |
| Compound 19 | 4429.1 | 4428.4 | Compound 20 | 4642.4 | 4642.2 |
| Compound 21 | 4850.6 | 4850.3 | Compound 22 | 4866.6 | 4865.9 |
| Compound 23 | 4913.7 | 4913.5 | Compound 24 | 4836.6 | 4835.7 |
| Compound 26 | 4942.7 | 4942.2 | Compound 27 | 4822.5 | 4822.3 |
| Compound 28 | 4865.6 | 4864.7 | Compound 29 | 4879.6 | 4879.0 |
| Compound 30 | 4907.6 | 4906.9 | Compound 32 | 4865.6 | 4864.6 |
| Compound 33 | 4444.1 | 4443.4 | Compound 35 | 4473.2 | 4472.5 |
| Compound 36 | 4301.0 | 4300.3 | Compound 38 | 4914.6 | 4914.4 |
| Compound 39 | 4879.6 | 4878.8 | Compound 40 | 4835.6 | 4835.2 |
| Compound 41 | 4892.6 | 4891.9 | Compound 43 | 4443.1 | 4442.4 |
| Compound 44 | 4834.6 | 4834.5 | Compound 45 | 4819.6 | 4818.9 |
| Compound 46 | 4863.6 | 4862.7 | Compound 47 | 4899.6 | 4899.1 |
| Compound 48 | 4428.2 | 4427.4 | Compound 49 | 4807.6 | 4807.7 |
| Compound 53 | 4808.5 | 4807.9 | Compound 54 | 4793.5 | 4792.6 |
| Compound 55 | 4926.7 | 4926.3 | Compound 56 | 4834.6 | 4834.4 |
| Compound 57 | 4806.6 | 4806.1 | Compound 58 | 4849.7 | 4849.9 |
| Compound 59 | 4428.2 | 4427.6 | Compound 60 | 4300.0 | 4299.3 |
| Compound 61 | 4891.7 | 4891.1 | Compound 62 | 4848.7 | 4848.5 |
| Compound 63 | 4850.6 | 4850.4 | Compound 64 | 4822.5 | 4821.7 |
| Compound 65 | 4444.1 | 4443.7 | Compound 66 | 4864.6 | 4864.2 |
| Compound 67 | 4836.6 | 4836.8 | Compound 68 | 4458.1 | 4457.4 |
| Compound 71 | 4879.6 | 4879.0 | Compound 72 | 4914.6 | 4913.8 |
| Compound 73 | 4892.7 | 4891.9 | Compound 74 | 4864.6 | 4864.1 |
| Compound 76 | 4486.2 | 4485.4 | Compound 77 | 4358.1 | 4357.4 |
| Compound 78 | 4906.7 | 4906.5 | Compound 79 | 4878.6 | 4878.3 |
| Compound 80 | 4500.2 | 4499.8 | Compound 81 | 4372.1 | 4371.5 |
| Compound 82 | 4949.7 | 4949.3 | Compound 83 | 4984.8 | 4985.0 |
| Compound 126 | 4269.0 | 4268.6 | Compound 160 | 4431.2 | 4431.1 |
| Compound 207 | 4427.2 | 4427.5 | Compound 243 | 5038.8 | 5038.6 |
| Compound 279 | 4414.1 | 4414.4 | Compound 356 | 4610.4 | 4610.7 |

Mass spectrometry and amino acid sequencing results prove that the peptide structures are correct.

A peptide was dissolved in saline (pH 7.4) to make a stock solution. The peptide concentration in the stock solution was quantified by conventional methods such as Bradford method and ultraviolet spectrophotometry. Before the drug efficacy experiments in animals, the required doses were taken from the peptide stock solution, diluted with phosphate buffered saline (PBS, pH 7.4) to prepare an injection solution. An appropriate injection volume for each animal was 5ml/kg body weight, which was used to calculate the injection volume that need to be configured.

All data of animal experiments was entered into Excel spreadsheets, and all values were expressed as mean±SEM. The significance of the differences among groups was evaluated by one way or two-way ANOVA followed by Dunnett's test using graphpad Prism 6 statistic software. The differences between the two groups were compared by the unpaired T-Test method, and a P value less than 0.05 was considered to be a significant difference.

### Example 7

In vivo efficacy of the peptides of the present invention can be determined in any suitable animal models known in the art and in clinical trials. For example, db/db mouse is a suitable animal model of diabetes.
db/db mice were housed in animal feeding rooms with strictly controlled environmental conditions. The temperature in the feeding room was maintained at 20-24°C and the humidity was maintained at 40-70%. The temperature and humidity of the feeding room were monitored in real time by a temperature and humidity meter, and the temperature and humidity were recorded twice a day (once in the morning and once in the afternoon). The lighting in the animal feeding room was controlled by an electronic timing light-on system. The lights were turned on for 12 hours and turned off for 12 hours every day (on at 7:00 in the morning and off at 19:00 in the afternoon). During the experiment, the mice were individually housed in each cage, and toys were provided to the mice in each cage. During the experiment mice had free access to water. The db/db male mice (7 weeks old) were given one week to acclimate to the environmental conditions of the test facility. Baseline blood glucose and body weight were recorded for three days before the test (-3 day to -1 day). The mice were randomly grouped based on three-day blood glucose and body weight, with 6 mice in each group. The mice were injected subcutaneously with saline (5ml/kg, control group) or peptide compounds 12 and 15 (10nmol/kg). Blood was collected at 0 hour before drug administration, and 1, 4, 8, 24, 36 and 48 hours after administration, and blood glucose was measured using a OneTouch glucose meter (Johnson & Johnson) and test strips. Blood glucose curves were plotted with time as the abscissa and the blood glucose value at different time point as the ordinate, the areas under the curve (AUC) were calculated, and hypoglycemic effects and duration of action of the peptide compounds were compared.

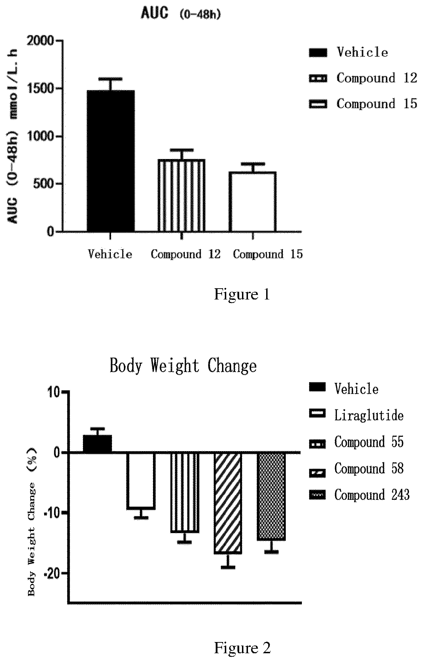

The results in Figure 1 indicate that compounds 12 and 15 could significantly reduce blood glucose of type 2 diabetic mice, and the differences between animals in the control group and in the treatment groups were statistically significant. These two compounds have the potential to be used as long-acting diabetes drugs.

### Example 8

In vivo efficacy of the peptides of the present invention can be determined in any suitable animal models known in the art and in clinical trials. Diet-induced obesity (DIO) mouse is an animal model of obesity, insulin resistance and hyperlipidemia.

Five-week-old male C57BL/6 mice were housed in the animal feeding room with strictly controlled environmental conditions. The temperature in the feeding room was maintained at 20-24°C, and the humidity was maintained at 30-70%. The temperature and humidity of the feeding room were monitored in real time by a temperature and humidity meter, and the temperature and humidity were recorded twice a day (once in the morning and once in the afternoon). The lighting in the animal feeding room is controlled by an electronic timing light system, which turns on the lights for 12 hours and turns off for 12 hours a day (on at 6:00 in the morning and off at 18:00 in the afternoon). During the experiment, the mice were individually housed in each cage, and toys were provided to the mice in each cage. Animals were fed on high-fat feed (the weight ratio of each nutrient was 26.2% protein, 26.3% carbohydrate, 36.9% fat, and the percentage of calories provided was 20%, 20%, and 60%, respectively) starting at 6 weeks of age. During the feeding process, the animals drank freely. 26-week-old male DIO mice with an average body weight of about 50 grams were selected for the experiment. The animals were adapted to grasping and subcutaneous injection for 1 week. The body weight and food intake were measured for 3 consecutive days before the experiment. The blood glucose of the animals was measured 1 day before the experiment, and the animals were grouped based on their blood glucose and body weight, 8 animals in each group.

During the experiment, the animals in the control group were injected subcutaneously with PBS every two days, and the animals in liraglutide group were injected subcutaneously with liraglutide (60 nmol/kg) every two days. The other 3 groups of animals were injected subcutaneously with peptides 55, 58, and 243 (20 nmol/kg) every two days, for a total of 11 doses. On the 23^{rd} day, the animal's body weight was measured. The percentage change in body weight is [(final body weight-starting body weight)/starting body weight] * 100.

As shown in Figures 2 peptide compounds 55, 58, and 243 all reduced the body weight of type 2 diabetes mice, which was statistically significant compared with the control group and significantly better than liraglutide. These compounds have the potential to be used as long-acting weight loss drugs.

### Example 9 Pharmacokinetics Test

8-weeks-old SD rats of body weight 200-300g were grouped with 8 rats in each group, a half are male and the other half are female, to study the pharmacokinetic characteristics of the compound of the present invention in the rats after a single subcutaneous injection. Compound 12, 15 and the control compound (peptide compound 27 described in PCT/CN2020/070697) were dissolved in PBS and 1 mg/ml solutions were prepared. Subcutaneous injections at a dose of 75 µg/kg were performed. 0.2 ml of whole blood was collected at 0, 0.5, 2, 4, 8, 12, 24, 48, 72, 96, 120, 144, and 168 hours, transferred to EDTA-K2 anticoagulation tubes, put into a 2-8 °C centrifugal machine to centrifuge at 1200-1500 ×g for 10-15 minutes. Transfer plasma into a clean tube, label the information and store it in a -60∼-90°C refrigerator. The polypeptide concentration in each sample was analyzed using LC-MS / MS. The mass spectrometer was QTRAP^{®}6500 (Applied Biosystems / SciEx), and pharmacokinetic parameters were calculated using non-compartmental models of WinNonLin v6.4 software (Pharsight Inc.). The half -life of compound 12 is 9.82 ± 0.49 hours, the half -life of compound 15 is 10.46 ± 0.71 hours, and the half -life of the control compound is 7.93 ± 0.72 hours.

## Claims

1. A peptide compound of formula (I) or a pharmaceutically acceptable salt or solvate thereof,
Y-aib-E-G-T-F-T-S-D-X1-S-X11-X2-X12-E-X3-X4-A-XS-X6-X13-F-X7-X8-W-L-X9-X14-G-X10 (I)
wherein X1 is an amino acid residue selected from L, K, C, or Y, or formula(II), or formula(III); X2 is an amino acid residue selected from Q, A, aib, or Y; X3 is an amino acid residue selected from E, C, or K, or formula(II), or formula(III); X4 is an amino acid residue selected from E, C, or K, or formula(II), or formula(III); X5 is an amino acid residue selected from V or A; X6 is an amino acid residue selected from K, R, C, or Q, or formula(II), or formula(III); X7 is an amino acid residue selected from I or V; X8 is an amino acid residue selected from E, Q, N, K, C, or A, or formula(II), or formula(III); X9 is an amino acid residue selected from I, L, C, or K, or formula(II), or formula(III); X10 is absent or is GPSSGAPPP, GPPSGAPPP,GPSSGKPPP, GPSSGEPPP, GPSSaibAPPP, GPSSGAPP, GPSSGAP, GPSSGA, GPSSG, GPSS, GPS, GP, or G; X11 is I, C, S, K, or formula(II), or formula(III); X12 is L, C, or K, or formula(II), or formula(III); X13 is L, E, or D; X14 is an amino acid residue selected from A, C, K, R, or formula(II), or formula(III); said structure of formula(II) or formula(III) is as follows: 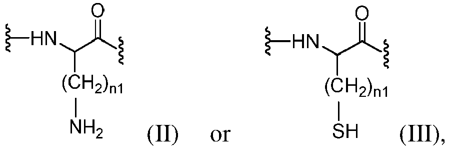
wherein the wavy line indicates the attachment point to an adjacent group, n1 is an integer of 1-7,
optionally, the amino acid residue of formula(II) containing a side chain amino group is modified at its side chain amino group with a long-acting group, the amino acid residue of formula(III) containing a side chain mercapto group is modified at its side chain mercapto group with a long-acting group; preferably, formula(II) is a lysine residue, formula(III) is a cysteine residue,
optionally, one or two amino acids selected from S or an amino acid whose side chain cotains an amino group or a mercapto group are added to the C-terminus of X10, and the carboxyl group of the C-terminal amino acid is optionally amidated to a C-terminal amide, said amino acid residue containing an amino group or a mercapto group has the structure of formula(II) or (III); when formula (II) or (III) is a C-terminal amino acid, its carboxyl part is COOH or CONH₂, preferably, formula(II) is lysine, and formula (III) is cysteine,
preferably, said long-acting group has the structure of formula (IV):
O1-O2-O3-O4-O5-O6-O7-O8- (IV),
wherein O1 has the structure of formula (V) or (VI): 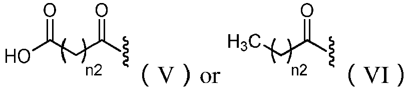
wherein n2 is an integer of 6-24, preferably 10-24, further preferably 16-22;
wherein the wavy line indicates the attachment point to the amino group of an adjacent structure, and O2-O3-O4-O5-O6-O7-O8- represents a linker, wherein each of O2 to O8 is independently selected from any one of the following amino acid residues or long chain structures: α-Glu, γ-Glu, α-Asp, β-Asp, α-hGlu, δ-hGlu, Gly, Ala, β-Ala, GABA, or PEG2, or one or more residues from O2 to O8 are absent, provided that at least two residues from O2 to O8 are present, preferably, O2 to O8 contain at least one negatively charged moiety.

2. The peptide compound or the pharmaceutically acceptable salt or solvate thereof according to claim 1, wherein O2-O3-O4-O5-O6-O7-O8- represents a linker selected from the group consisting of γGlu-PEG2-γGlu-, γGlu-PEG2-2×γGlu-, γGlu-PEG2-, γGlu-2×PEG2-, γGlu-3×PEG2-, γGlu-PEG2-γGlu-PEG2, γGlu-2 × PEG2-γGlu-, γGlu-2×PEG2-2×γGlu-, 2×γGlu-, 2×γGlu-PEG2-, 2×γGlu-PEG2-γGlu-, 2×γGlu-PEG2-γGlu-PEG2-, 2×γGlu-2×PEG2-, 2×γGlu-2×PEG2-γGlu, 2×γGlu-2×PEG2-2×γGlu-.

3. The peptide compound or the pharmaceutically acceptable salt or solvate thereof according to claim 1 or 2, wherein an amino acid at the C-terminus of X10 contains a side chain thiol group which is modified with a long-acting group of formula (IV), the side chain thiol of said amino acid is connected to one end of the linking group L through a Michael acceptor or a thiol reactive group, preferably, the other end of said linking group L forms a covalent bond with the long-acting group of formula (IV) through an amino group or a carboxyl group, preferably, the linking group L is selected from: -NH-(CH₂)ₙ₅-(CH₂CH₂O)ₙ₆-(CH₂)ₙ₇-, -NH-(CH₂)ₙ₅-(CH₂CH₂O)ₙ₆-(CH₂)ₙ₇-NH-, -NH-(CH₂)ₙ₅-(CH₂CH₂O)ₙ₆-(CH₂)ₙ₇-CO-, -NH-(CH₂)ₙ₅-(CH₂CH₂O)ₙ₆-(CH₂)ₙ₇-NHCO-(CH₂)ₙ₈-, -NH-(CH₂)ₙ₅-(CH₂CH₂O)ₙ₆-(CH₂)ₙ₇-NHCO-(CH₂)ₙ₈-NH-, or any combination thereof, wherein n3, n4, n5, n6, n7, n8 are each an integer from 0 to 10, more preferably, L is -NH-CH₂- (CH₂CH₂O)₃-(CH₂)₃-NH- or -NH-(CH₂)ₙ₅-(CH₂CH₂O)ₙ₆-(CH₂)ₙ₇-NHCO-(CH₂)ₙ₈-.

4. The peptide compound or the pharmaceutically acceptable salt or solvate thereof according to any one of the preceding claims, wherein a long-acting group is linked to the side chain amino group of lysine, said molecular structure is abbreviated as K(x18) and has the structure of formula(VII): 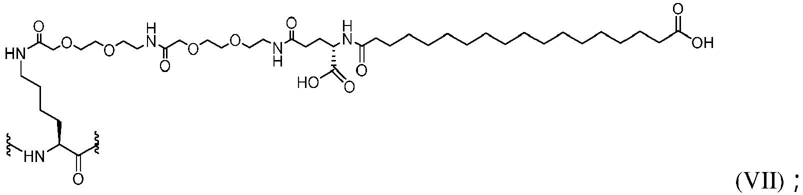 or wherein a long-acting group is linked to the side chain amino group of lysine, said molecular structure is abbreviated as K(x20) and has the structure of formula(VIII): 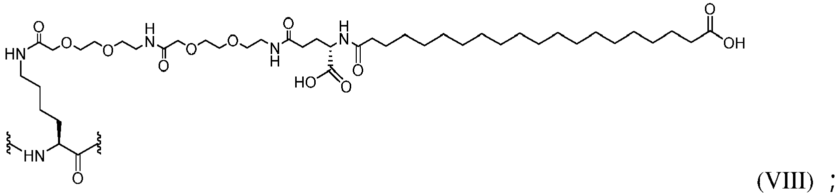 wherein the wavy line indicates the attachment point to the adjacent amino acid residue.

5. The peptide compound or the pharmaceutically acceptable salt or solvate thereof according to any one of the preceding claims, wherein at least one of X1, X3, X4, X6, X8, X9, X11, X12, and X14 is formula(II) or formula(III); or wherein at least one of X1, X4, X8, X11, X12, and X14 is formula(II) or formula(III); or wherein at least one of X1, X3, X4, X6, X8, X9, X11, X12, andX14 is formula(II) or formula(III), and at least one formula(II) or formula(III) has the side chain which is linked to a long-acting group or a modifying group.

6. The peptide compound or the pharmaceutically acceptable salt or solvate thereof according to any one of the preceding claims, wherein the peptide compound is selected from: 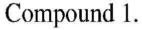 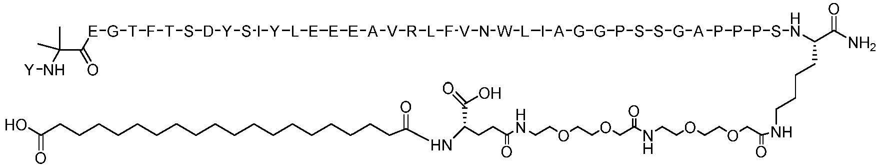 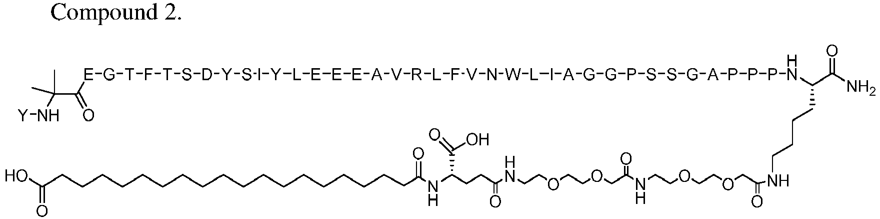 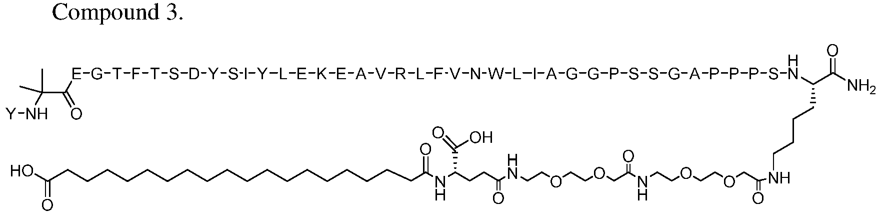 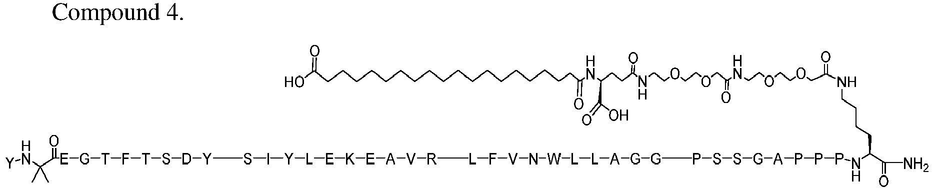 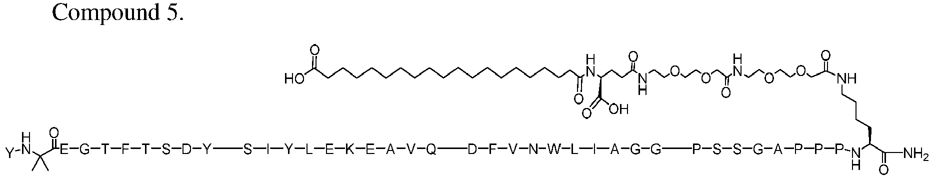 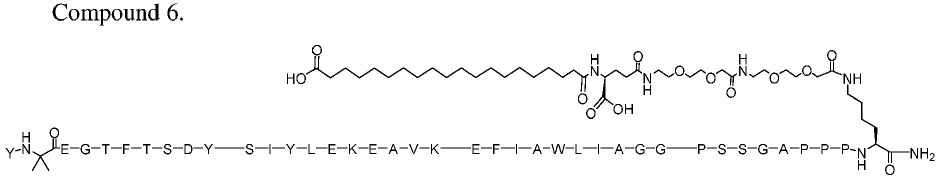 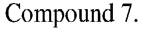 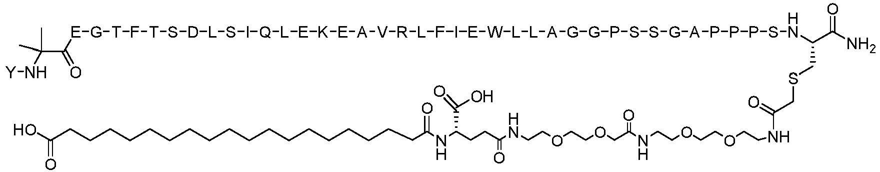 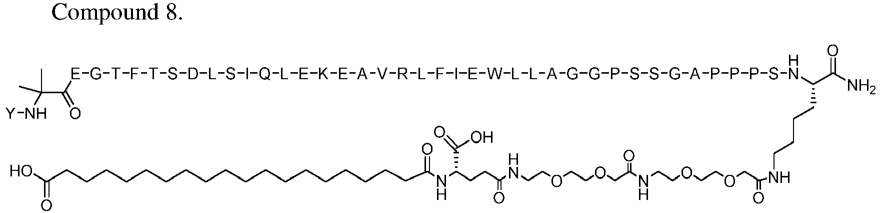 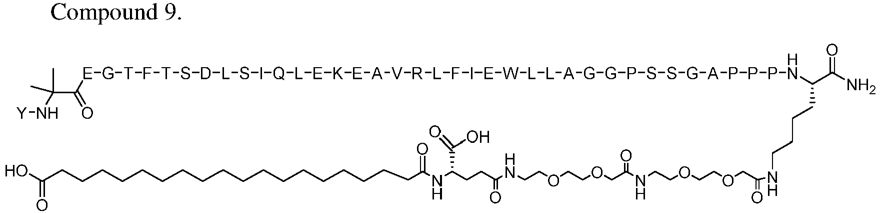 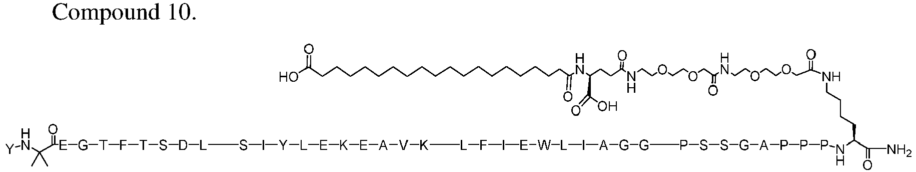 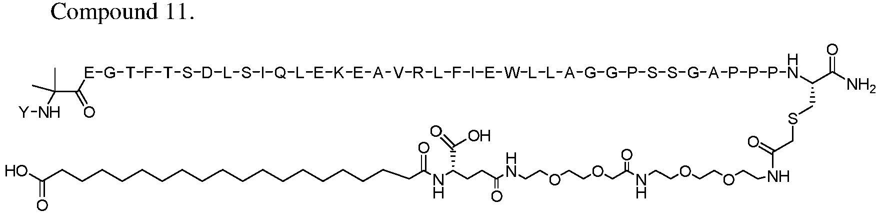 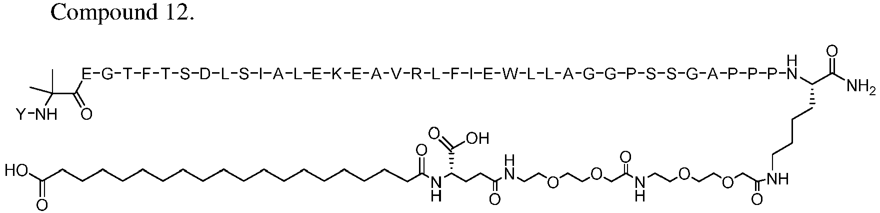 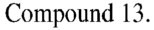 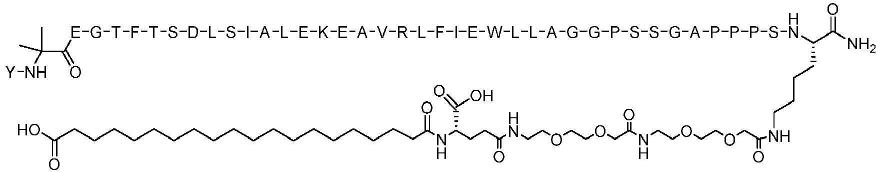 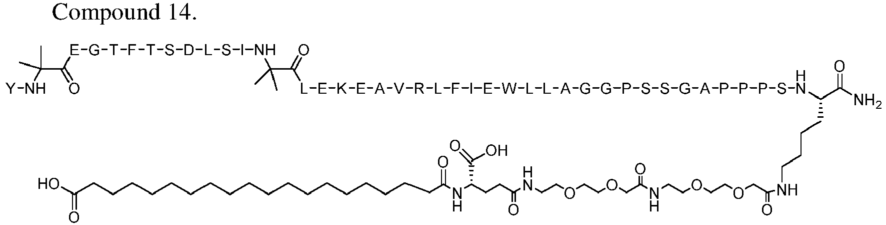 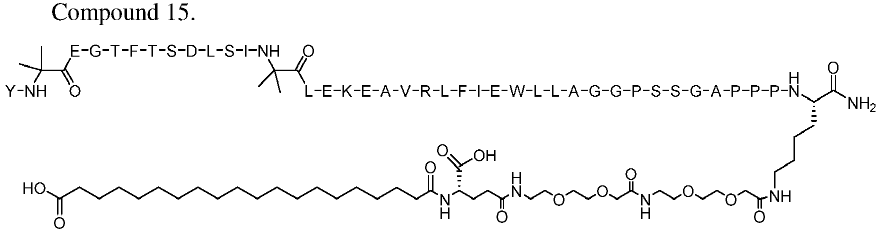 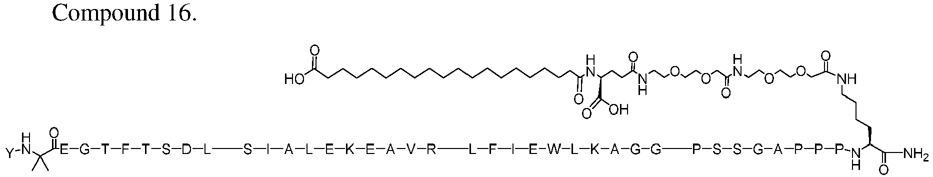 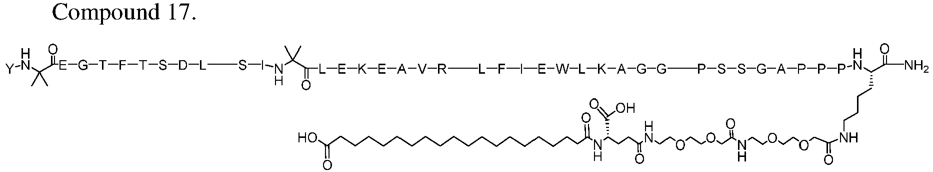 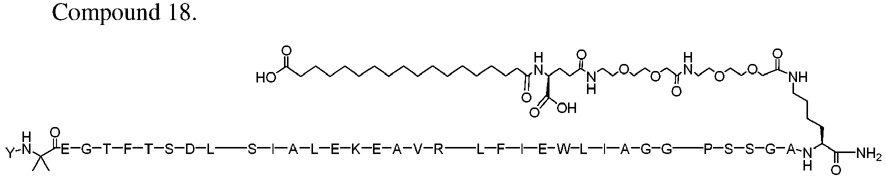 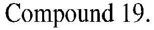 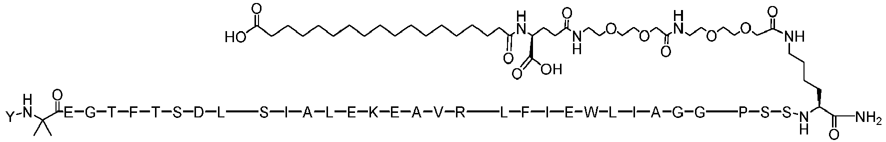 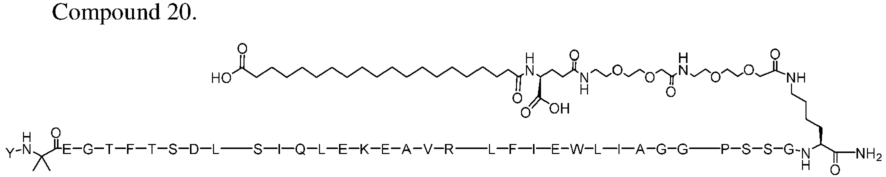 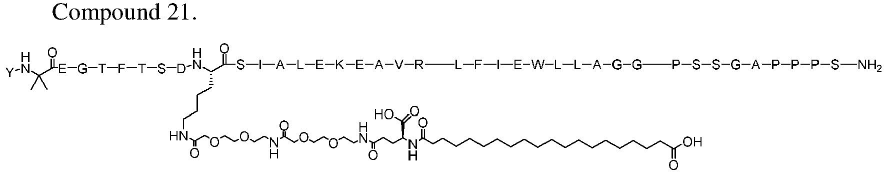 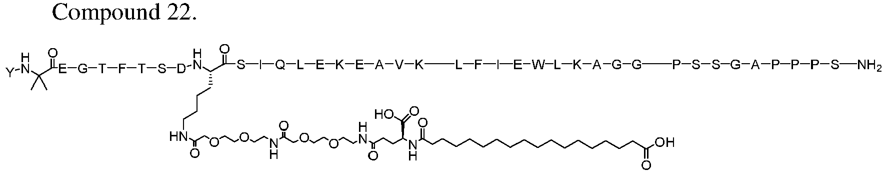 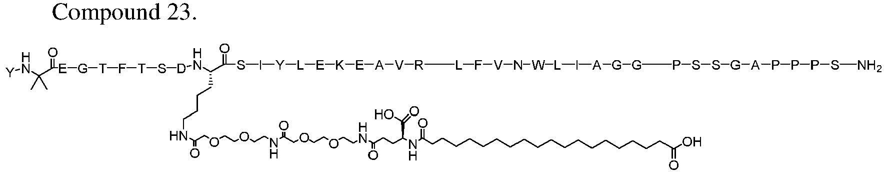 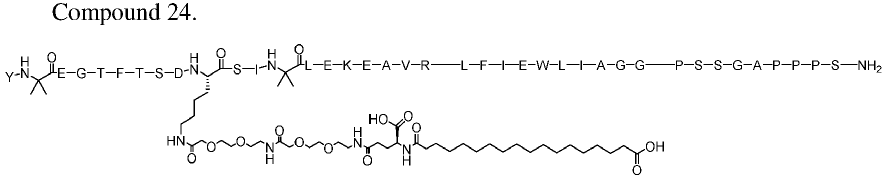 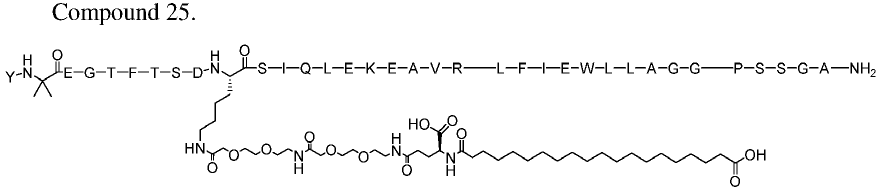 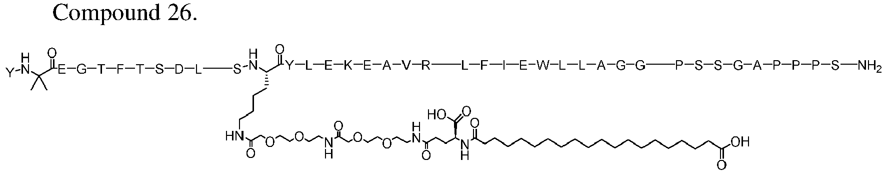 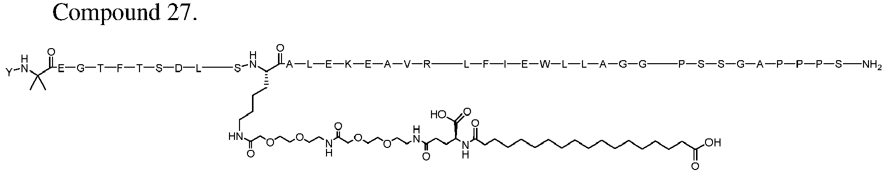 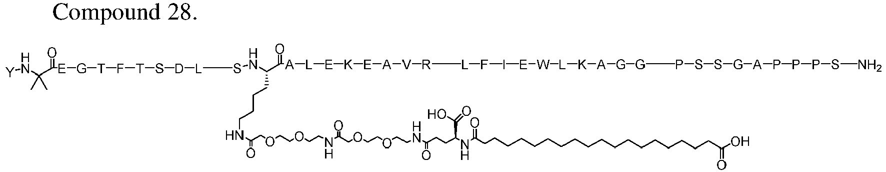 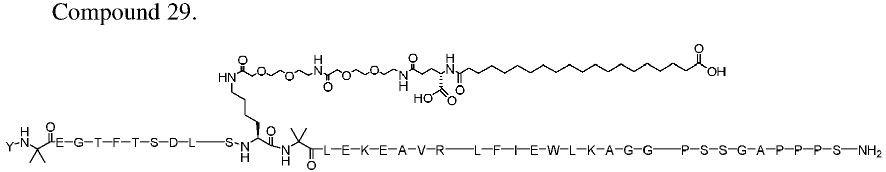 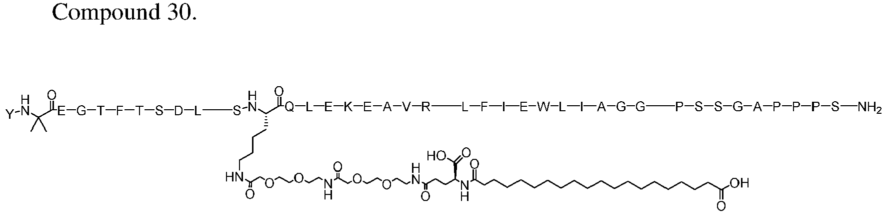 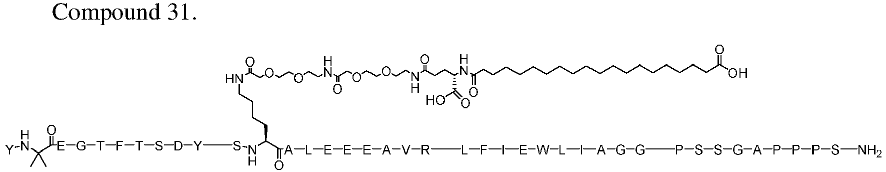 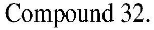 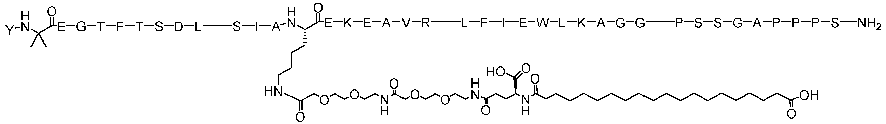 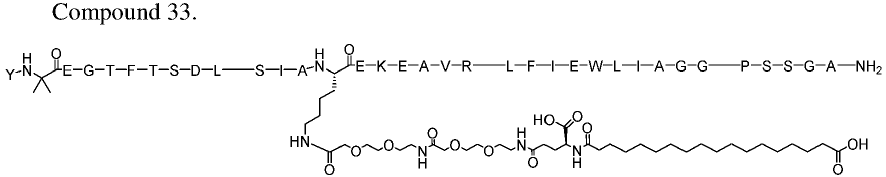 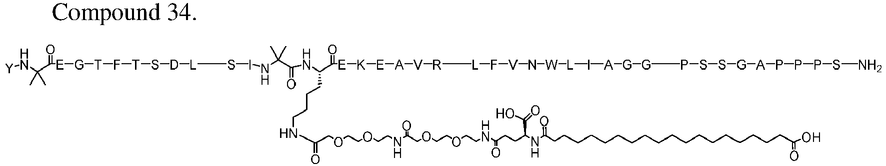 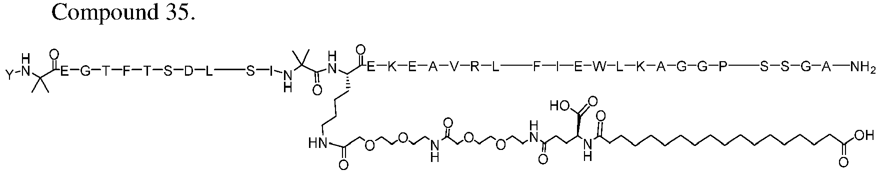 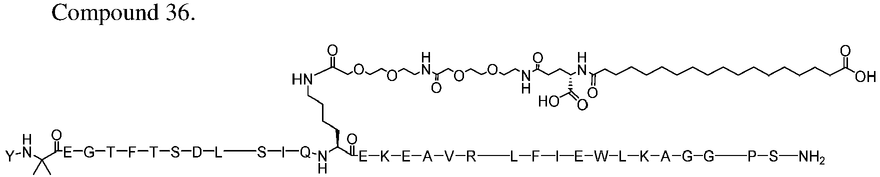 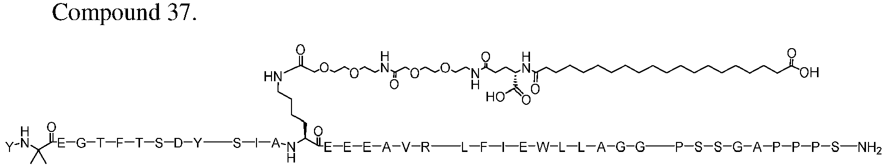 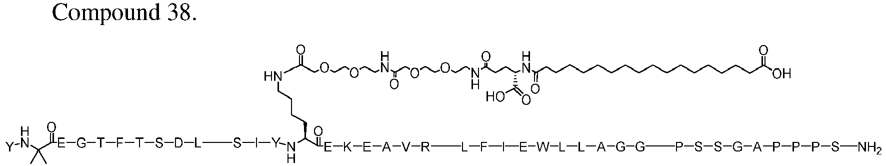 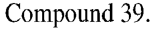 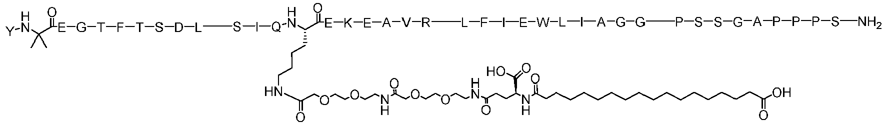 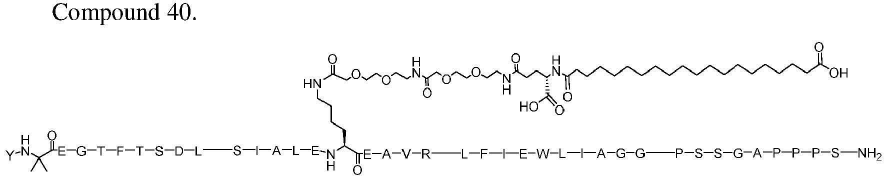 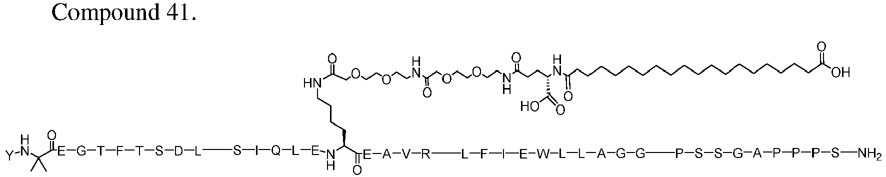 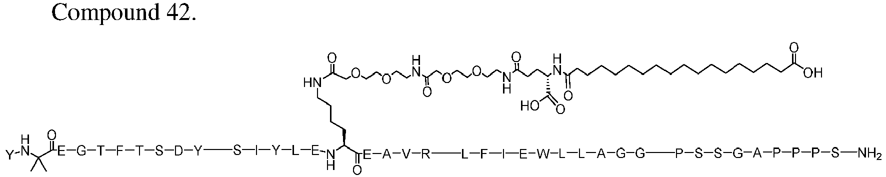 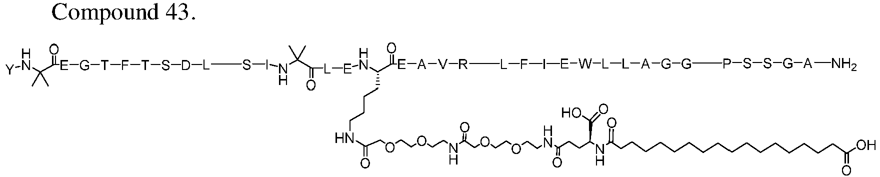 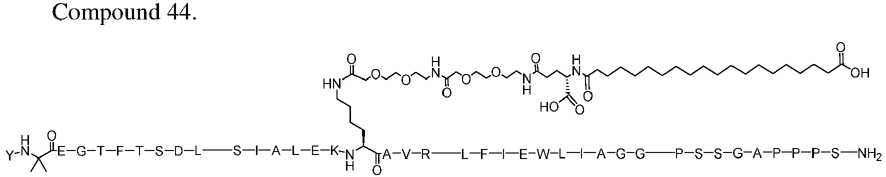 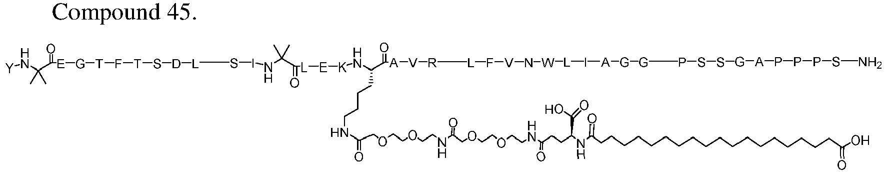 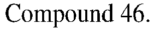 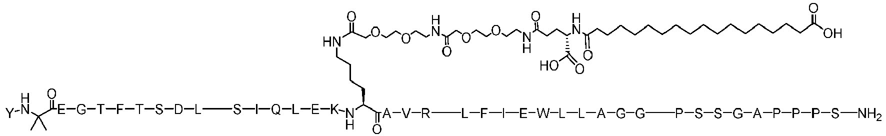 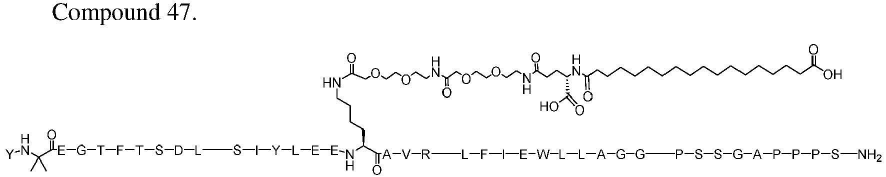 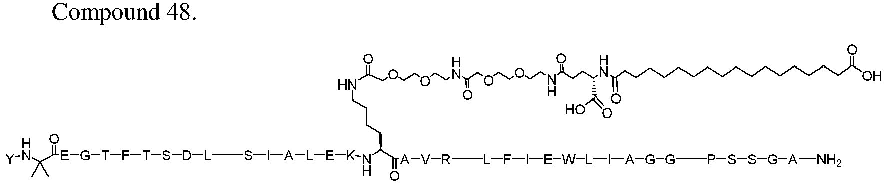 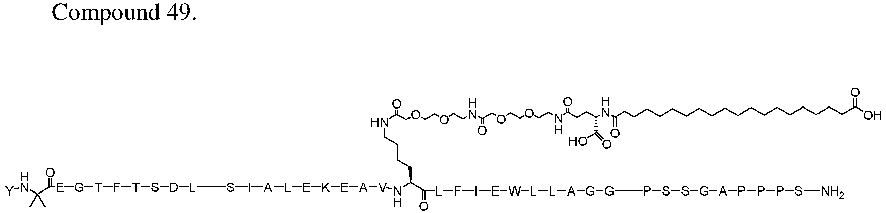 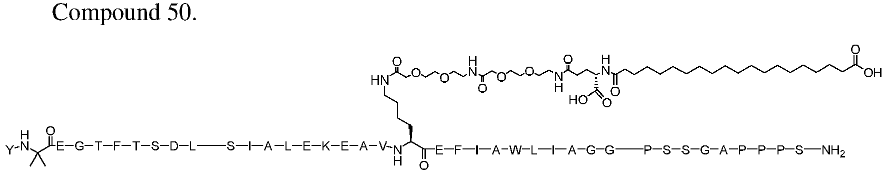 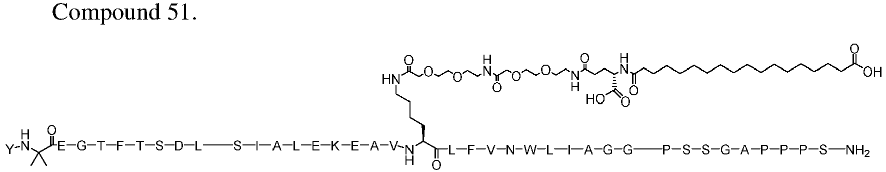 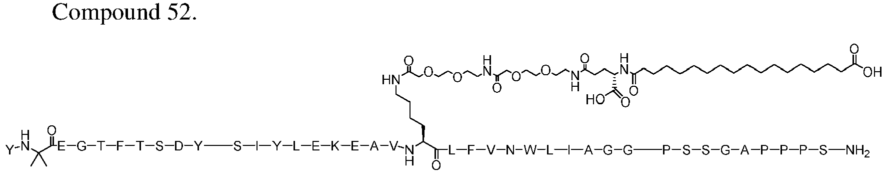 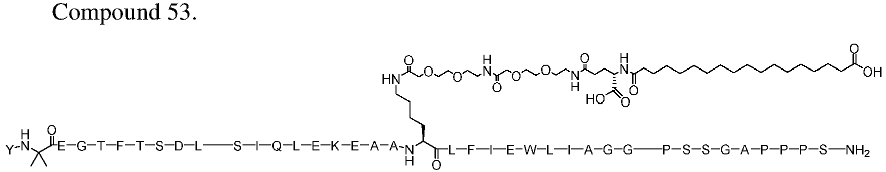 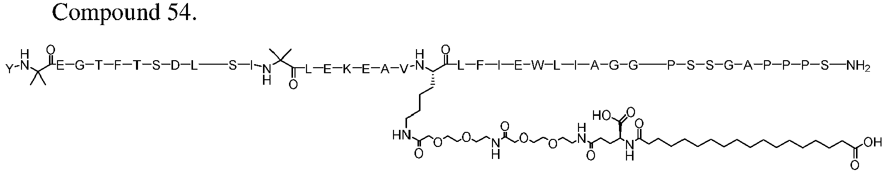 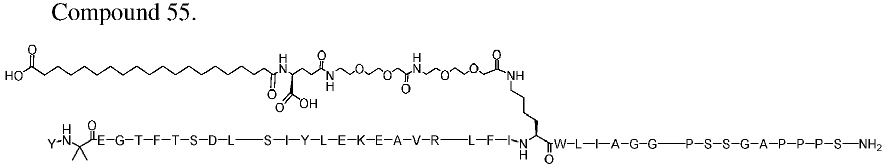 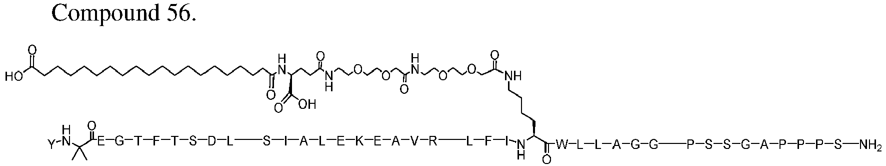 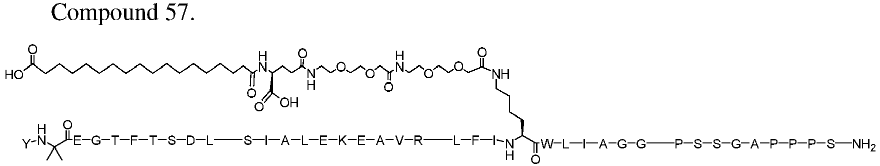 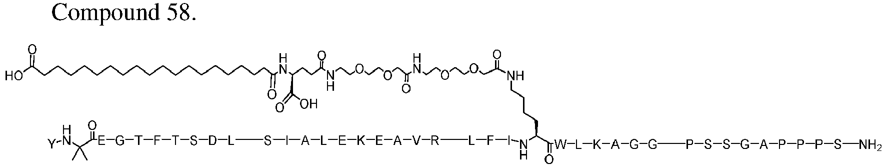 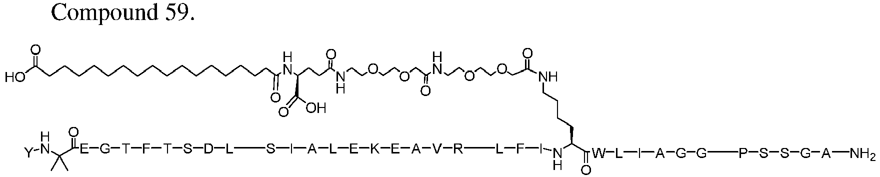 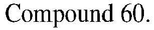 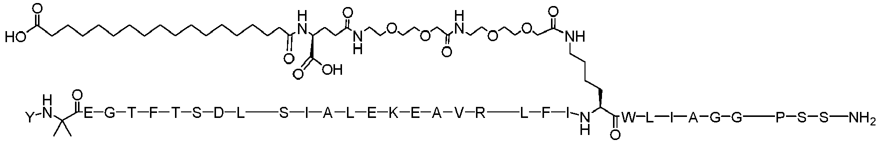 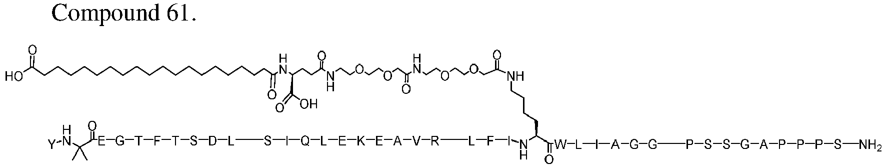 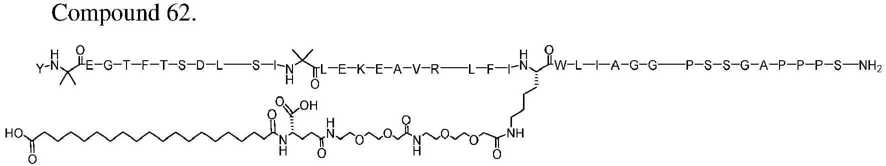 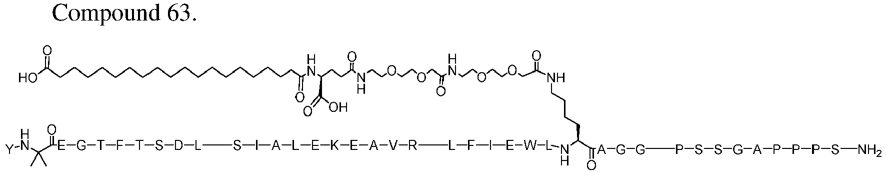 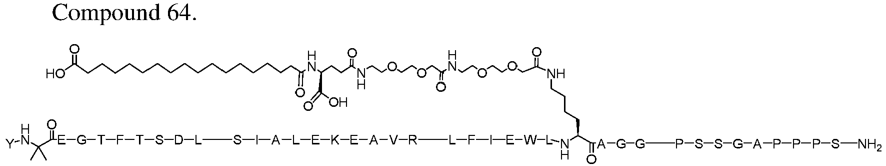 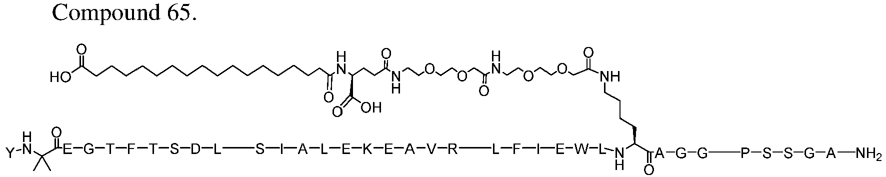 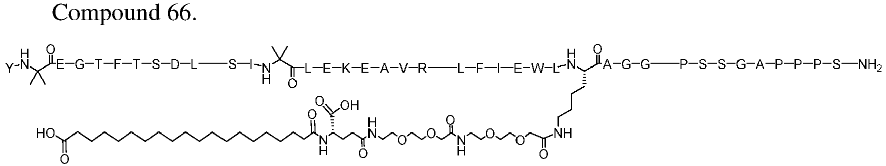 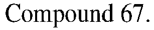 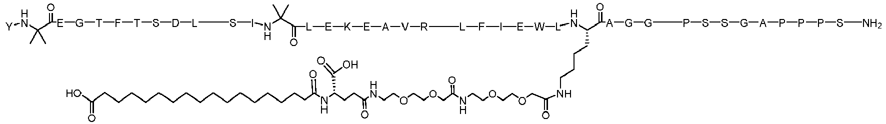 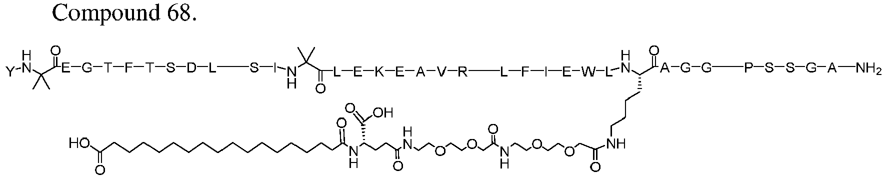 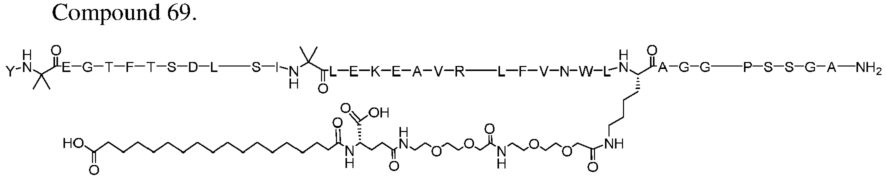 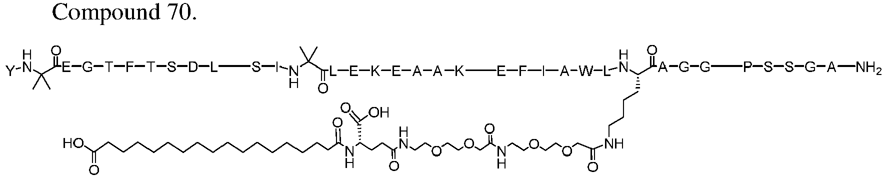 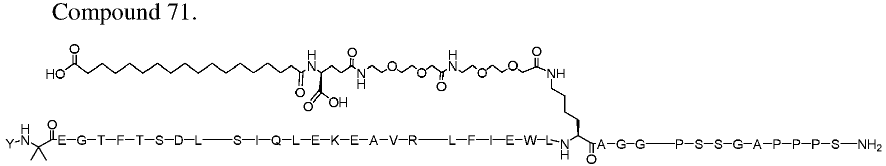 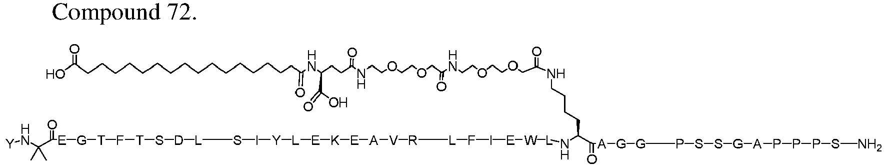 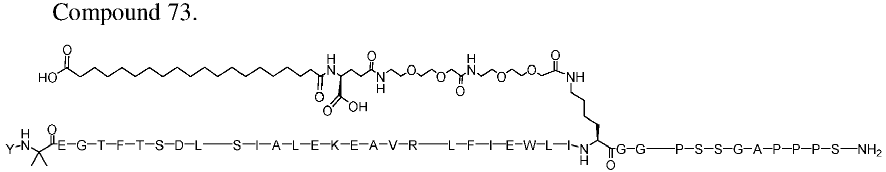 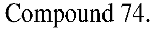 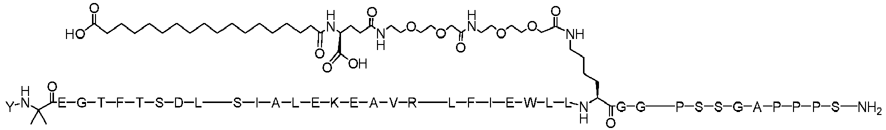 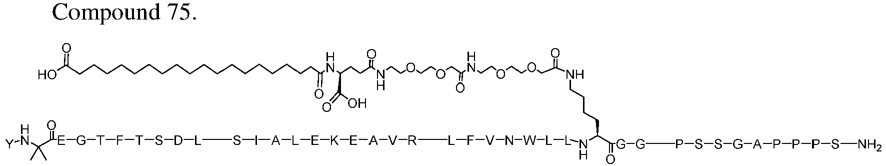 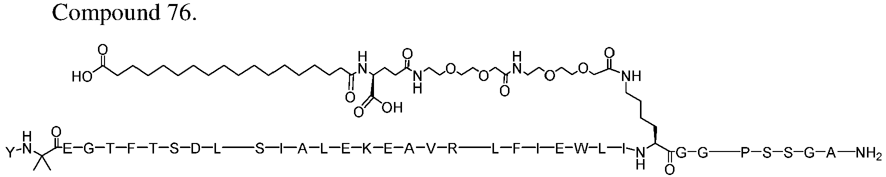 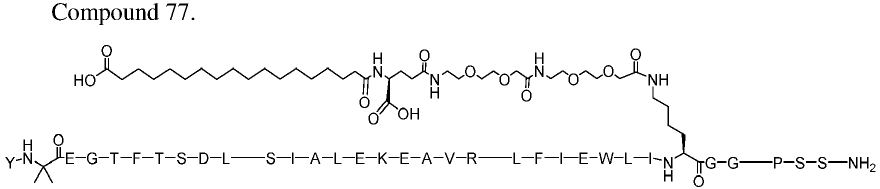 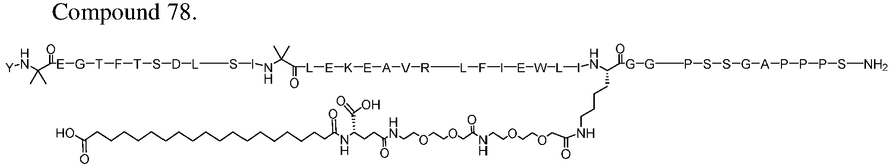 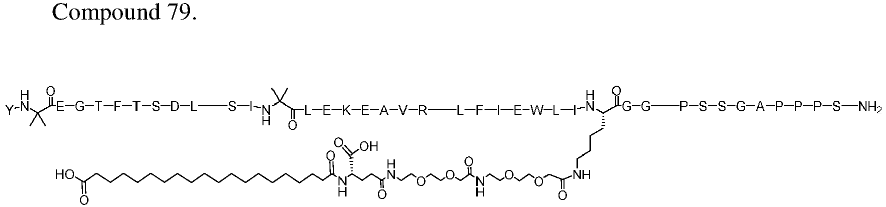 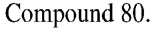 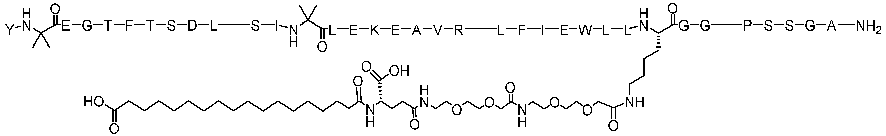 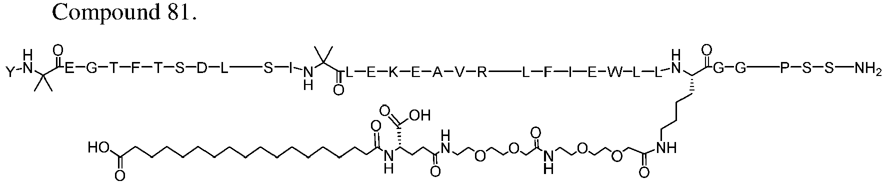 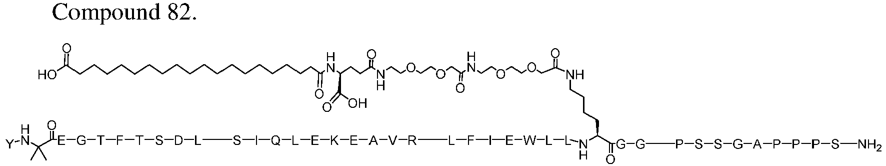 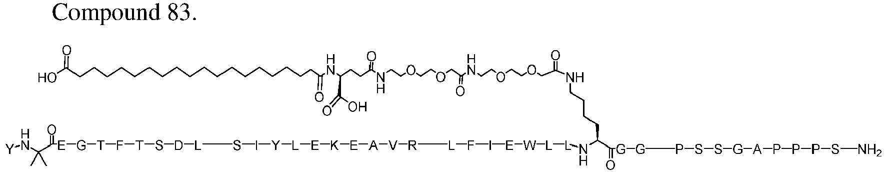 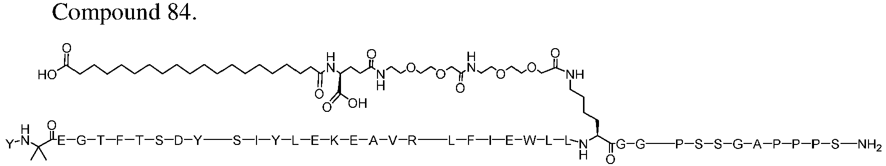 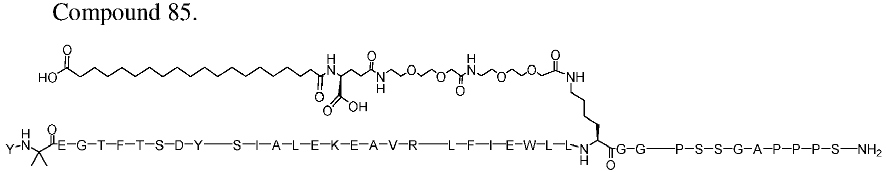
Compound 86. YaibEGTFTSDLSK(x20)ALEKEAVRLFIEWLIAGGPSSGA-NH₂
Compound 87. YaibEGTFTSDLSIQLEEEAVRLFIEWLIK(x20)GGPSSGAPPPS-NH₂
Compound 88. YaibEGTFTSDLSK(x18)ALEKEAVRLFIEWLKAGGPSS-NH2
Compound 89. YaibEGTFTSDLSIaibLEKEAVRLFIEWLIAGGPSSK(x18)-NH₂
Compound 90. YaibEGTFTSDLSIQLEKEAVRLFIEWLIK(x18)GGPSSGA-NH₂
Compound 91. YaibEGTFTSDLSK(x18)QLEKEAVRLFIEWLIAGGPSS-NH2
Compound 92. YaibEGTFTSDLSIaibLEKEAVRLFIEWLKAGGPSSGK(x18)-NH₂
Compound 93.YaibEGTFTSDLSK(x20)QLEKEAVQDFVNWLLAGGPSSGAPPPS-NH₂
Compound 94.YaibEGTFTSDLSIYLEKEAVRLFIEWLLK(x18)GGPSSGAPPPS-NH2
Compound 95.YaibEGTFTSDLSIALEKEAVRLFIEWLLAGGPSSGAPPPC(x20)-NH₂
Compound 96.YaibEGTFTSDLSIQLEKEAVRLFIEWLIK(x18)GGPSSGAPPPS-NH₂
Compound 97.YaibEGTFTSDLSK(x20)ALEKEAVKEFIAWLIAGGPSSGAPPPS-NH₂
Compound 98. YaibEGTFTSDLSIALEKEAVRLFIEWLKAGGPSSK(x20)-NH₂
Compound 99. YaibEGTFTSDLSIaibK(x20)EKEAVRLFIEWLIAGGPSSGAPPPS-NH₂
Compound 100. YaibEGTFTSDLSIQLEKEAVRLFIEWLIK(x18)GGPSS-NH₂
Compound 101.YaibEGTFTSDLSIaibK(x18)EKEAVRLFIEWLIAGGPSSGAPPPS-NH₂
Compound 102.YaibEGTFTSDLSIaibLEKEAVRLFIEWLIAGGPSK(x20)-NH₂
Compound 103.YaibEGTFTSDYSIYLEKEAVRLFIEWLLK(x20)GGPSSGAPPPS-NH₂
Compound 104.YaibEGTFTSDLSIAC(x18)EKEAVRLFIEWLLAGGPSSGAPPPS-NH₂
Compound 105.YaibEGTFTSDLSIALEKEAVRLFIEWLLAGGPSSGAPPPC(x18)-NH₂
Compound 106.YaibEGTFTSDLSIQLEKEAVKLFVNWLIK(x20)GGPSSGAPPPS-NH₂
Compound 107.YaibEGTFTSDLSIAC(x20)EKEAVRLFIEWLIAGGPSSGAPPPS-NH₂
Compound 108.YaibEGTFTSDLSIaibLEKEAVRLFIEWLLK(x20)GGPSS-NH2
Compound 109.YaibEGTFTSDLSIaibLEKEAVRLFIEWLIAGGPSSGAPPPC(x20)-NH₂
Compound 110.YaibEGTFTSDLSIaibLEKEAVRLFIEWLIAGGPSSK(x20)-NH₂
Compound 111.YaibEGTFTSDLSIALEKEAVRLFIEWLKAGGPSSGA-NH2
Compound 112.YaibEGTFTSDLSIALEKEAVRLFIEWLKAGGPSSGAPPPS-NH2
Compound 113.YaibEGTFTSDLSIQLEKEAVRLFIEWLLAGGPSSGAPPPC(x18)-NH₂
Compound 114.YaibEGTFTSDK(x18)SIYLEKEAVRLFIEWLIAGGPSSGA-NH₂
Compound 115.YaibEGTFTSDLSIALEKEAVRLFIEWLIAGGPSSGAK(x20)-NH₂
Compound 116.YaibEGTFTSDLSIaibLEKEAVRLFIEWLIAGGPSSGAPPPC(x18)-NH₂
Compound 117.YaibEGTFTSDK(x20)SIALEEEAVQDFVQWLIAGGPSSGAPPPS-NH₂
Compound 118.YaibEGTFTSDLSC(x20)ALEKEAVKEFIAWLIAGGPSSGAPPPS-NH₂
Compound 119.YaibEGTFTSDLSIYLEKEAVKLFIEWLIAGGPSSGK(x20)-NH₂
Compound 120.YaibEGTFTSDLSIaibLEKEAVRLFIEWLLK(x20)GGPSSGA-NH₂
Compound 121.YaibEGTFTSDLSIALEKEAVRLFIEWLKAGGPSS-NH2
Compound 122.YaibEGTFTSDK(x18)SIALEKEAVRLFIEWLLAGGPS-NH₂
Compound 123.YaibEGTFTSDLSIaibLEKEAVRLFIEWLIAGGPK(x20)-NH₂
Compound 124.YaibEGTFTSDLSIaibLEKK(x18)AVRLFIEWLKAGGPS-NH₂
Compound 125.YaibEGTFTSDLSIALEKEAVRLFIEWLIK(x20)GGPSS-NH2
Compound 126.YaibEGTFTSDLSIaibLEKEAVRLFIEWLIAGGPK(x18)-NH₂
Compound 127.YaibEGTFTSDLSIAK(x18)EKEAVRLFIEWLLAGGPSSGAPPPS-NH₂
Compound 128.YaibEGTFTSDLSIaibLEKEAVRLFIEWLKAGGPSSG-NH₂
Compound 129.YaibEGTFTSDLSIALEKEAVRLFIEWLKK(x20)GGPSSGAPPPS-NH₂
Compound 130.YaibEGTFTSDLSIaibLEKEAVRLFIEWLKAGGPSSGAK(x18)-NH₂
Compound 131.YaibEGTFTSDLSIaibLEKEAVRLFIEWLKAGGPSSGAPPPS-NH₂
Compound 132.YaibEGTFTSDLSIQK(x18)EKEAVRLFIEWLKAGGPSS-NH₂
Compound 133.YaibEGTFTSDLSIYLEKEAVRLFIEWLLK(x18)GGPS-NH2
Compound 134.YaibEGTFTSDLSIYLEKEAVKLFIEWLIAGGPSSGAPPPC(x18)-NH₂
Compound 135.YaibEGTFTSDLSIaibLEKEAVRLFIEWLKAGGPSSGA-NH₂
Compound 136.YaibEGTFTSDLSIaibLEKK(x20)AVRLFIEWLKAGGPSSGAPPPS-NH₂
Compound 137.YaibEGTFTSDLSIALEC(x18)EAVRLFIEWLLAGGPSSGAPPPS-NH₂
Compound 138.YaibEGTFTSDLSIYLEKEAVKLFIEWLIAGGPSSGAK(x20)-NH₂
Compound 139.YaibEGTFTSDLSIYK(x20)EKEAVRLFIEWLKAGGPSSGA-NH₂
Compound 140.YaibEGTFTSDK(x18)SIALEKEA VRLFIEWLKAGGPSSGAPPPS-NH₂
Compound 141.YaibEGTFTSDLSIaibLEKK(x18)AVRLFIEWLKAGGPSSG-NH₂
Compound 142.YaibEGTFTSDLSIYLEKEAVKLFIEWLIAGGPSSGAPPPK(x18)-NH₂
Compound 143.YaibEGTFTSDLSIaibLEKEAVRLFIEWLC(x20)AGGPSSGAPPPS-NH₂
Compound 144.YaibEGTFTSDLSIYLEKEAVRLFIEWLKAGGPSS-NH₂
Compound 145.YaibEGTFTSDK(x20)SIaibLEKEAVRLFIEWLKAGGPSSGAPPPS-NH₂
Compound 146.Y(aib)EGTFTSDLSIALEKEAVRLFIEWLIAGGPK(x20)-NH₂
Compound 147.YaibEGTFTSDC(x18)SIaibLEKEA VRLFIEWLLAGGPSSGAPPPS-NH₂
Compound 148.YaibEGTFTSDLSIaibLEKK(x18)AVRLFIEWLLAGGPSSGAPPPS-NH₂
Compound 149.YaibEGTFTSDLSIALEKEAVRLFIEWLIAGGPSSGK(x18)-NH₂
Compound 150.YaibEGTFTSDLSIYK(x18)EKEA VRLFIEWLLAGGPS-NH2
Compound 151.YaibEGTFTSDLSIALEKEAVRLFIEWLC(x20)AGGPSSGAPPPS-NH₂
Compound 152.YaibEGTFTSDC(x20)SIALEKEAVRLFIEWLLAGGPSSGAPPPS-NH₂
Compound 153.YaibEGTFTSDLSIQK(x18)EKEAVRLFIEWLKAGGPSSGA-NH₂
Compound 154.YaibEGTFTSDLSIaibLEKEAVRLFIEWLC(x18)AGGPSSGAPPPS-NH₂
Compound 155.YaibEGTFTSDLSIaibLEKEAVRLFIEWLIAGGPSK(x18)-NH₂
Compound 156.YaibEGTFTSDLSIAK(x18)EKEAVRLFIEWLKAGGPSSGA-NH₂
Compound 157.YaibEGTFTSDLSIALEKEAVRLFIEWLIAGGPSK(x18)-NH₂
Compound 158.YaibEGTFTSDLSIYLEKEAVRLFIK(x18)WLIAGGPSS-NH2
Compound 159.YaibEGTFTSDLSIAK(x20)EKEAVRLFIEWLIAGGPSSGAPPPS-NH₂
Compound 160.YaibEGTFTSDLSIQLEKEAVRLFIEWLIAGGPSSK(x18)-NH₂
Compound 161.YaibEGTFTSDLSIaibK(x18)EKEAVRLFIEWLIAGGPSSGA-NH₂
Compound 162.YaibEGTFTSDLSIALEKEAVRLFIEWLC(x20)AGGPSSGA-NH2
Compound 163.YaibEGTFTSDLSIALEKEAVRLFIEWLIAGGPSK(x20)-NH₂
Compound 164.YaibEGTFTSDLSIALEKK(x18)AVRLFIEWLIAGGPSSGAPPPS-NH₂
Compound 165.YaibEGTFTSDLSIALEKEAVRLFIEWLKAGGPSSG-NH₂
Compound 166.YaibEGTFTSDLSIALEKK(x18)AVRLFIEWLLAGGPSSGA-NH₂
Compound 167.YaibEGTFTSDLSIYLEKEAVRLFIK(x18)WLIAGGPSSGA-NH₂
Compound 168.YaibEGTFTSDLSIaibLEKEAVRLFIEWLK(x20)AGGPSS-NH₂
Compound 169.YaibEGTFTSDLSIQLEKEAVRLFIEWLKAGGPSSGAPP-NH₂
Compound 170.YaibEGTFTSDLSIAK(x18)EKEAVRLFIEWLKAGGPSS-NH₂
Compound 171.YaibEGTFTSDLSIaibLEKEAVRLFIEWLKAGGPS-NH₂
Compound 172.YaibEGTFTSDLSIALEKK(x18)AVRLFIEWLLAGGPSS-NH2
Compound 173.YaibEGTFTSDLSC(x20)aibLEKEAVKEFIAWLIAGGPSSGAPPPS-NH₂
Compound 174.YaibEGTFTSDLSIALEKEAVRLFIEWLKAGGPS-NH2
Compound 175.YaibEGTFTSDLSIALEKK(x20)AVRLFIEWLKAGGPSSGAPPPS-NH₂
Compound 176.YaibEGTFTSDLSIALEKEAVRLFIEWLIAGGPSSGK(x20)-NH₂
Compound 177.YaibEGTFTSDLSIALEKEAVRLFIEWLC(x18)AGGPSSGA-NH2
Compound 178.YaibEGTFTSDLSIQLEEEAVRLFIEWLK(x20)AGGPSSGAPPPS-NH₂
Compound 179.YaibEGTFTSDLSIYLEKEAVRLFIEWLLC(x20)GGPSSGAPPPS-NH₂
Compound 180.YaibEGTFTSDLSIALEKEAVRLFIEWLIAGGPK(x18)-NH₂
Compound 181.YaibEGTFTSDLSIaibLEK(x18)EAVRLFIEWLIAGGPSSGAPPPS-NH₂
Compound 182.YaibEGTFTSDLSIALEKEAVRLFIEWLLC(x20)GGPSS-NH₂
Compound 183.YaibEGTFTSDLSIALEC(x20)EAVRLFIEWLIAGGPSSGAPPPS-NH₂
Compound 184.YaibEGTFTSDLSIYLEKEAVRLFIEWLIAGGPSSK(x20)-NH₂
Compound 185.YaibEGTFTSDYSIYLEKK(x20)AVRLFIEWLIAGGPSSGAPPPS-NH₂
Compound 186.YaibEGTFTSDLSIQLEKEAVRLFIEWLIAGGPSK(x18)-NH₂
Compound 187.YaibEGTFTSDLSIYLEKK(x20)AVRLFIEWLLAGGPSSGAPPPS-NH₂
Compound 188.YaibEGTFTSDLSIaibLEKEAVRLFIC(x18)WLIAGGPSSGAPPPS-NH₂
Compound 189.YaibEGTFTSDLSIQLEKEAVRLFIEWLIC(x18)GGPSSG-NH₂
Compound 190.YaibEGTFTSDLSIALEKEA VK(x18)LFIEWLKAGGPSSGA-NH₂
Compound 191.YaibEGTFTSDLSIALEKEAVRLFIEWLIAGGPSSK(x20)-NH₂
Compound 192.YaibEGTFTSDLSIQLEKEAVRLFIEWLKK(x18)GGPSSGAPPPS-NH₂
Compound 193.YaibEGTFTSDLSIALEKEAVK(x18)LFIEWLKAGGPSS-NH₂
Compound 194.YaibEGTFTSDLSIYLEKK(x20)AVRLFIEWLKAGGPSSGAPPPS-NH₂
Compound 195.YaibEGTFTSDLSIQLEKEAVRLFIK(x18)WLKAGGPSS-NH₂
Compound 196.YaibEGTFTSDLSIYLEKK(x18)AVRLFIEWLKAGGPSSGA-NH₂
Compound 197.YaibEGTFTSDLSIYLEKEAVRLFIK(x18)WLLAGGPSSGAPPPS-NH₂
Compound 198.YaibEGTFTSDLSIQLEKEAVRLFIEWLKAGGPSSGAP-NH₂
Compound 199.YaibEGTFTSDLSIALEKEAVK(x20)LFIEWLKAGGPSSGAPPPS-NH₂
Compound 200.YaibEGTFTSDLSIYLEKEAVRLFIEWLIAGGPSSGAPPPC(x20)-NH₂
Compound 20 1.Y aibEGTFTS DLS IaibLEKEA VRLFIK(x18)WLLAGGPSSGAPPPS-NH₂
Compound 202.YaibEGTFTSDLSIYLEKK(x18)AVRLFIEWLKAGGPSS-NH2
Compound 203.YaibEGTFTSDLSIALEKEAVK(x18)LFIEWLIAGGPSSGAPPPS-NH₂
Compound 204.YaibEGTFTSDLSIQLEKEAVRLFIEWLKAGGPSSGA-NH₂
Compound 205.YaibEGTFTSDLSIALEKEAVRLFIEWLLC(x18)GGPSS-NH₂
Compound 206.YaibEGTFTSDLSIALEKEAVK(x20)DFVNWLLAGGPSSGAPPPS-NH₂
Compound 207.YaibEGTFTSDLSIQLEKEAVRLFIEWLIAGGPSK(x20)-NH₂
Compound 208.YaibEGTFTSDLSIYLEKEAVRLFIEWLLK(x18)GGPSSGA-NH₂
Compound 209.YaibEGTFTSDLSIALEKEAVC(x18)LFIEWLIAGGPSSGAPPPS-NH₂
Compound 210.YaibEGTFTSDLSIALEKEAVRLFIC(x18)WLLAGGPSSGAPPPS-NH₂
Compound 211.YaibEGTFTSDLSIQLEKEAVRLFIEWLKAGGPSSG-NH₂
Compound 212.YaibEGTFTSDLSIALEKEAVC(x20)LFIEWLIAGGPSSGAPPPS-NH₂
Compound 213.YaibEGTFTSDLSIaibLEKK(x18)AVRLFIEWLLAGGPSS-NH₂
Compound 214.YaibEGTFTSDLSIALEKEAVRLFIEWLLC(x20)GGPSSGAPPPS-NH₂
Compound 215.YaibEGTFTSDLSIYLEKEAVRLFIEWLLC(x18)GGPSS-NH₂
Compound 216.YaibEGTFTSDLSIALEKC(x20)AVRLFIEWLIAGGPSSGAPPPS-NH₂
Compound 217.YaibEGTFTSDLSIYLEKEAVK(x20)LFIEWLLAGGPSSGAPPPS-NH₂
Compound 218.YaibEGTFTSDLSIQLEKEAVRLFIEWLKAGGPSS-NH₂
Compound 219.YaibEGTFTSDYSIYLEKEAVRLFIC(x20)WLLAGGPSSGAPPPS-NH₂
Compound 220.YaibEGTFTSDLSIYLEKEAVK(x20)LFIEWLKAGGPS-NH₂
Compound 221.YaibEGTFTSDLSIaibLEKEAVRLFIEWLK(x20)AGGPSSGA-NH₂
Compound 222.YaibEGTFTSDLSIaibLEKEAVRLFIK(x18)WLKAGGPSS-NH₂
Compound 223.YaibEGTFTSDLSIALEKC(x18)AVRLFIEWLIAGGPSSGAPPPS-NH₂
Compound 224.YaibEGTFTSDLSIQLEKEAVRLFIEWLKAGGPS-NH₂
Compound 225.YaibEGTFTSDLSIYLEKEAVK(x18)LFIEWLIAGGPSSGAPPPS-NH₂
Compound 226.YaibEGTFTSDLSIaibLEK(x20)EAVRLFIEWLIAGGPSSGAPPPS-NH₂
Compound 227.YaibEGTFTSDLSIYLEKEAVK(x18)LFIEWLLAGGPSSG-NH₂
Compound 228.YaibEGTFTSDLSIYLEKEAVRLFIEWLLC(x20)GGPSS-NH₂
Compound 229.YaibEGTFTSDLSIALEKEAVRLFIK(x20)WLKAGGPSSGA-NH₂
Compound 230.YaibEGTFTSDLSIaibLEKEAVRLFIEWLLC(x20)GGPSS-NH₂
Compound 231.YaibEGTFTSDLSIaibLEKEAVK(x18)LFIEWLKAGGPSSGA-NH₂
Compound 232.YaibEGTFTSDLSIALEKEAVRLFIK(x20)WLKAGGPSS-NH₂
Compound 233.YaibEGTFTSDLSIALEKEAVKEFIAWLK(x20)AGGPSSGAPPPS-NH₂
Compound 234.YaibEGTFTSDLSIYLEKEAVRLFIEWLKAGGPS-NH₂
Compound 235.YaibEGTFTSDLSIQLEKEAVK(x18)LFIEWLLAGGPSSGAPPPS-NH₂
Compound 236.YaibEGTFTSDLSIYLEK(x20)EAVRLFIEWLIAGGPSSGAPPPS-NH₂
Compound 237.YaibEGTFTSDLSIaibLEKEAVRLFIEWLIAGGPSSGAK(x20)-NH₂
Compound 238.YaibEGTFTSDLSIQLEKEAVRLFIEWLK(x20)AGGPSSGAPPPS-NH₂
Compound 239.YaibEGTFTSDLSIALEKEAVRLFIK(x18)WLKAGGPSS-NH₂
Compound 240.Y aibEGTFTS DLS IaibLEKEA VRLFIEWLLC(x18)GGPSSGAPPPS-NH₂
Compound 241.YaibEGTFTSDYSIYLEKEAVRLFIC(x18)WLLAGGPSSG-NH₂
Compound 242.YaibEGTFTSDLSIaibLEKEAVRLFIEWLLC(x20)GGPSSGA-NH₂
Compound 243.YaibEGTFTSDLSIQLEKEAVRLFIC(x20)WLLAGGPSSGAPPPS-NH₂
Compound 244.YaibEGTFTSDLSIaibLEKEAVRLFIEWLIAGGPSSGK(x18)-NH₂
Compound 245.YaibEGTFTSDYSIYLEKEAVRLFIEWLK(x20)AGGPSSGAPPPS-NH₂
Compound 246.YaibEGTFTSDLSIQLEKEAVRLFIK(x18)WLIAGGPS-NH₂
Compound 247.YaibEGTFTSDLSIALEK(x18)EAVRLFIEWLLAGGPSSGAPPPS-NH₂
Compound 248.YaibEGTFTSDLSIaibLEKEAVRLFIEWLLC(x18)GGPSSGA-NH₂
Compound 249.YaibEGTFTSDLSIQLEK(x18)EAVRLFIEWLLAGGPSSG-NH₂
Compound 250.YaibEGTFTSDLSIALEKEAVRLFIK(x18)WLKAGGPSSGA-NH₂
Compound 251.YaibEGTFTSDLSIQLEK(x18)EAVRLFIEWLLAGGPS-NH₂
Compound 252.YaibEGTFTSDYSIYLEKEAVRLFIC(x18)WLLAGGPSSGAPPPS-NH₂
Compound 253.Y aibEGTFTS DLS IaibLEKEA VRLFIEWLKK(x18)GGPSSGAPPPS-NH₂
Compound 254.YaibEGTFTSDLSIYLEKEAVRLFIEWLKAGGPSSGAPPPS-NH₂
Compound 255.YaibEGTFTSDLSIaibLEKEAVRLFIK(x20)WLIAGGPSSGA-NH₂
Compound 256.YaibEGTFTSDLSIQLEKEA VK(x18)LFIEWLLAGGPSSG-NH₂
Compound 257.YaibEGTFTSDLSIaibLEKEAVRLFIK(x18)WLIAGGPSS-NH₂
Compound 258.YaibEGTFTSDLSIYLEKEAVRLFIEWLLC(x20)GGPSSGA-NH₂
Compound 259.YaibEGTFTSDLSIQLEKK(x20)AVRLFIEWLIAGGPSSGAPPPS-NH₂
Compound 260.YaibEGTFTSDLSIaibLEKEAVRLFIK(x20)WLKAGGPSSGAPPPS-NH₂
Compound 261.YaibEGTFTSDLSIQLEKEAVK(x18)LFIEWLIAGGPS-NH₂
Compound 262.YaibEGTFTSDLSIaibLEKEAVRLFIC(x20)WLLAGGPSSGAPPPS-NH₂
Compound 263.YaibEGTFTSDLSIQLEKEAVRLFIEWLIC(x20)GGPSSG-NH₂
Compound 264.YaibEGTFTSDLSIYLEKEAVRLFIEWLKAGGPSSGAPP-NH₂
Compound 265.YaibEGTFTSDLSIQLEKEAVRLFIEWLK(x20)AGGPSSGA-NH₂
Compound 266.YaibEGTFTSDLSIaibLEKEAVRLFIC(x20)WLKAGGPSSGAPPPS-NH₂
Compound 267.YaibEGTFTSDLSIALEKEAVRLFIEWLIK(x20)GGPSSGA-NH₂
Compound 268.YaibEGTFTSDLSIQLEKEA VRLFIC(x18)WLLAGGPSSGAPPPS-NH₂
Compound 269.YaibEGTFTSDLSIALEKEAVRLFIEWLLC(x18)GGPSSGAPPPS-NH₂
Compound 270.YaibEGTFTSDLSIYLEKEAVRLFIEWLKAGGPSSGAP-NH₂
Compound 271.Y aibEGTFTS DLS IaibLEKEA VRLFIC(x18)WLKAGGPSSGAPPPS-NH₂
Compound 272..YaibEGTFTSDLSIQLEKEAVRLFIEWLK(x18)AGGPSS-NH₂
Compound 273.YaibEGTFTSDLSIALEKEAVRLFIEWLKAGGPSSGAK(x20)-NH₂
Compound 274..YaibEGTFTSDLSIQLEKEAVRLFIEWLC(x18)AGGPSSGAPPPS-NH₂
Compound 275.YaibEGTFTSDLSIYLEKEAVRLFIEWLKAGGPSSGA-NH₂
Compound 276.YaibEGTFTSDLSIQLEKEAVRLFIK(x18)WLLAGGPSSGAPPPS-NH₂
Compound 277.YaibEGTFTSDLSIaibLEKEAVK(x18)LFIEWLKAGGPSS-NH₂
Compound 278.YaibEGTFTSDLSIYLEKEAVRLFIEWLLC(x18)GGPSSGAPPPS-NH2
Compound 279.YaibEGTFTSDLSIQLEKEAVRLFIK(x18)WLIAGGPSSG-NH₂
Compound 280.YaibEGTFTSDLSIQLEKEAVRLFIEWLIC(x18)GGPSSGAPPPS-NH₂
Compound 281.YaibEGTFTSDLSIaibLEKEAVRLFIEWLKAGGPSS-NH₂
Compound 282.YaibEGTFTSDLSIQLEKEAVRLFIK(x18)WLKAGGPSSGAPPPS-NH₂
Compound 283.YaibEGTFTSDLSIaibLEKEAVK(x20)LFIEWLLAGGPSSGAPPPS-NH₂
Compound 284.YaibEGTFTSDLSIQLEKEAVRLFIK(x18)WLKAGGPSSGA-NH₂
Compound 285.YaibEGTFTSDLSIALEKEAVRLFIK(x18)WLKAGGPSSGAPPPS-NH₂
Compound 286.YaibEGTFTSDLSIQLEKEAVRLFIEWLC(x20)AGGPSSGA-NH₂
Compound 287.YaibEGTFTSDLSIaibLEKEAVRLFIK(x18)WLKAGGPSSGA-NH₂
Compound 288.Y aibEGTFTSDLSIALEKEA VRLFIEWLC(x 18)AGGPSSGAPPPS-NH₂
Compound 289.YaibEGTFTSDLSIQLEKEAVRLFIEWLIAGGPSSK(x20)-NH₂
Compound 290.YaibEGTFTSDLSIALEKEAVRLFIK(x20)WLLAGGPSSGA-NH₂
Compound 291.YaibEGTFTSDLSIaibLEKEAVRLFIEWLK(x18)AGGPSSGA-NH₂
Compound 292.YaibEGTFTSDLSIQLEKEAVRLFIK(x20)WLKAGGPSSGAPPPS-NH₂
Compound 293.YaibEGTFTSDLSIYLEKEAVRLFIEWLLK(x20)GGPSSGA-NH₂
Compound 294.YaibEGTFTSDLSIALEKEAVRLFIK(x20)WLLAGGPSS-NH₂
Compound 295.YaibEGTFTSDLSIaibLEKEAVRLFIEWLK(x18)AGGPSS-NH₂
Compound 296.YaibEGTFTSDLSIYLEKEAVRLFIEWLLC(xl8)GGPSSGA-NH₂
Compound 297.YaibEGTFTSDLSIaibLEKEAVRLFIEWLKAGGPSSGAPP-NH2
Compound 298.YaibEGTFTSDLSIALEKEAVRLFIEWLLC(x20)GGPSSGA-NH₂
Compound 299.YaibEGTFTSDLSIALEKEAVRLFIK(x18)WLKAGGPS-NH₂
Compound 300.YaibEGTFTSDLSIALEKEAVRLFIC(x18)WLKAGGPSSGAPPPS-NH₂
Compound 301.YaibEGTFTSDLSIALEKEAVRLFIEWLKK(x 18)GGPSSGAPPPS-NH₂
Compound 302.YaibEGTFTSDLSIaibLEKEAVRLFIK(x20)WLKAGGPSSGA-NH₂
Compound 303.YaibEGTFTSDLSIQLEKK(x18)AVRLFIEWLIAGGPSSGA-NH₂
Compound 304.YaibEGTFTSDLSIaibLEKEAVRLFIEWLKK(x20)GGPSSGAPPPS-NH₂
Compound 305.YaibEGTFTSDLSIALEKEAVRLFIC(x20)WLKAGGPSSGAPPPS-NH₂
Compound 306.YaibEGTFTSDLSIYLEKEAVRLFIEWLKAGGPSSG-NH₂
Compound 307.YaibEGTFTSDLSIQLEKEAVRLFIEWLC(x20)AGGPSSGAPPPS-NH₂
Compound 308.YaibEGTFTSDK(x20)SIaibLEKEAVKEFIAWLLAGGPSSGAPPPS-NH₂
Compound 309.YaibEGTFTSDLSIALEKEAVRLFIEWLKAGGPSSGK(x20)-NH₂
Compound 310.YaibEGTFTSDLSK(x18)aibLEKEAVKEFIAWLIAGGPSSGAPPPS-NH₂
Compound 311.YaibEGTFTSDLSIALEKEAVRLFIC(x20)WLLAGGPSSGAPPPS-NH₂
Compound 312.YaibEGTFTSDLSIYLEKEAVRLFIEWLK(x20)AGGPSSGAPPPS-NH₂
Compound 313.YaibEGTFTSDLSIaibLEKEAVK(x18)LFIEWLKAGGPSSGAPPPS-NH₂
Compound 314.YaibEGTFTSDLSIQLEKEAVRLFIEWLIC(x20)GGPSSGAPPPS-NH₂
Compound 315.YaibEGTFTSDLSIaibLEKEAVRLFIK(x20)WLKAGGPSS-NH₂
Compound 316.YaibEGTFTSDLSIQLEKEAVQDFVNWLIK(x18)GGPSSGAPPPS-NH₂
Compound 317.YaibEGTFTSDLSIaibLEKEAVRLFIEWLKAGGPSK(x18)-NH₂
Compound 318.YaibEGTFTSDLSIYLEKEAVRLFIEWLC(x18)AGGPSSGAPPPS-NH₂
Compound 319. YaibEGTFTSDLSIQLEEEAVK(x18)EFIAWLLKGG-NH₂
Compound 320.YaibEGTFTSDLSIaibLEKEAVRLFIEWLIAGGPSSGAPPPK(x18)-NH₂
Compound 321.YaibEGTFTSDLSIQLEKEAVKLFVNWLK(x20)AGGPSSGAPPPS-NH₂
Compound 322.YaibEGTFTSDLSIALEKEAVRLFIEWLKAGGPSSGAP-NH₂
Compound 323.YaibEGTFTSDLSIaibLEKEAVRLFIEWLIAGGPSSGAK(x18)-NH₂
Compound 324.YaibEGTFTSDLSIQLEKEAVQDFVNWLK(x18)AGGPSSGAPPPS-NH₂
Compound 325.YaibEGTFTSDLSIYLEKEAVKLFIEWLIAGGPSK(x18)-NH₂
Compound 326.YaibEGTFTSDLSIaibLEKEAVRLFIK(x18)WLKAGGPSSGAPPPS-NH₂
Compound 327.YaibEGTFTSDLSIQLEKEAVRLFIEWLC(x18)AGGPSSGA-NH₂
Compound 328.YaibEGTFTSDK(x18)SIQLEKEAVRLFIEWLLAGGPSS-NH₂
Compound 329.YaibEGTFTSDLSIALEKEAVRLFIEWLK(x18)AGGPSS-NH₂
Compound 330.Y(aib)EGTFTSDLSIQLEKEAVRLFIEWLIAGGPSSGAK(x18)-NH₂
Compound 331.YaibEGTFTSDLSIALEKEAVRLFIEWLLC(x18)GGPSSGA-NH₂
Compound 332.YaibEGTFTSDLSIQLEKK(x18)AVRLFIEWLIAGGPSS-NH₂
Compound 333.YaibEGTFTSDLSIALEKEAVRLFIEWLC(x20)AGGPSS-NH₂
Compound 334.Y(aib)EGTFTSDLSIaibLEKEAVRLFIEWLKAGGPSSK(x18)-NH₂
Compound 335.YaibEGTFTSDLSIALEKEAVRLFIEWLC(x18)AGGPSS-NH₂
Compound 336.YaibEGTFTSDLSIaibLEKEAVRLFIEWLKAGGPSSGAP-NH₂
Compound 337.YaibEGTFTSDYSIYLEKEAVRLFIEWLLK(x18)GGPSSGAPPPS-NH₂
Compound 338.Y(aib)EGTFTSDLSIQLEKEAVRLFIEWLIAGGPSSGK(x18)-NH₂
Compound 339.YaibEGTFTSDLSIYLEKEAVRLFIEWLLK(x20)GGPSS-NH₂
Compound 340.YaibEGTFTSDK(x18)SIALEKEAVRLFIEWLLAGGPSSGA-NH₂
Compound 341.YaibEGTFTSDLSIQLEKEAVRLFIEWLKK(x20)GGPSSGAPPPS-NH₂
Compound 342.YaibEGTFTSDLSIQLEKEAVRLFIEWLIAGGPSSGAK(x20)-NH₂
Compound 343.YaibEGTFTSDLSIYLEKEAVRLFIC(x20)WLLAGGPSSGAPPPS-NH₂
Compound 344.YaibEGTFTSDLSK(x18)QLEKEAVRLFIEWLLAGGPSSGA-NH₂
Compound 345.YaibEGTFTSDLSIQLEKEAVRLFIEWLIC(x18)GGPS-NH₂
Compound 346. YaibEGTFTSDLSIALEKEAVRLFIEWLKAGGPSSGAPP-NH₂
Compound 347.YaibEGTFTSDLSIYLEKEAVRLFIEWLC(x20)AGGPSSGAPPPS-NH₂
Compound 348.YaibEGTFTSDLSIALEKEAVRLFIEWLIAGGPSSGAPPK(x20)-NH₂
Compound 349. YaibEGTFTSDLSIQLEKEAVRLFIK(x20)WLKAGGPSSGAPP-NH₂
Compound 350.Y(aib)EGTFTSDLSIALEKEAVRLFIEWLKAGGPSK(x18)-NH₂
Compound 351.Y(aib)EGTFTSDLSIaibLEKEAVRLFIEWLIAGGPSSGK(x20)-NH₂
Compound 352.YaibEGTFTSDLSIaibLEKEAVRLFIEWLLC(x18)GGPSS-NH₂
Compound 353.YaibEGTFTSDLSIYLEKEAVRLFIC(x18)WLLAGGPSSGAPPPS-NH₂
Compound 354.YaibEGTFTSDLSSYLEKEAVRLFVQWLKRGGPSSGAPC(x20)-NH₂
Compound 355.YaibEGTFTSDLSIaibLEKEAVRLFIEWLLC(x20)GGPSSGAPPPS-NH₂
Compound 356. YaibEGTFTSDLSIQLEKEAVRLFIK(x20)WLIAGGPSSGAP-NH₂
Compound 357. YaibEGTFTSDLSIYLEEEAAK(x18)EFIAWLLKGGP-NH₂.

7. A pharmaceutical composition comprising the peptide compound or the pharmaceutically acceptable salt or solvate thereof of any one of the preceding claims, and a pharmaceutically acceptable carrier or excipient.

8. The first medical use of the peptide compound or the pharmaceutically acceptable salt or solvate thereof according to any one of claims 1 to 6 or the pharmaceutical composition according to claim 7.

9. Use of the peptide compound or the pharmaceutically acceptable salt or solvate thereof according to any one of claims 1 to 6 or the pharmaceutical composition according to claim 7 for the preparation of a medication for treating or preventing the following diseases or conditions: impaired glucose tolerance (IGT), hyperglycemia, type 1 diabetes, type 2 diabetes, obesity, metabolic syndrome, neurodegenerative diseases, especially for delaying or preventing disease progression in type 2 diabetes, delaying progression from impaired glucose tolerance to type 2 diabetes; delaying progression from type 2 diabetes to insulin-requiring diabetes, treating metabolic syndrome, regulating appetite, inducing satiety, reducing food intake, increasing energy expenditure, treating obesity, preventing overweight; preventing weight rebound after successful weight loss; treating diseases or conditions associated with overweight or obesity; treating bulimia; treating binge eating; treating dyslipidemia, atherosclerosis, hypertension, coronary heart disease, β- blocker poisoning; non-alcoholic fatty liver disease (NAFLD) (can be divided into simple fatty liver (SFL), non-alcoholic fatty hepatitis (NASH) and related cirrhosis); use for inhibition of the motility of the gastrointestinal tract; neurodegenerative diseases including Alzheimer's disease, Parkinson's disease, Huntington's disease, ataxia, Kennedy disease, myotonic dystrophy, Lewy body dementia, multi-systemic atrophy, amyotrophic lateral sclerosis, primary lateralsclerosis, spinal muscular atrophy, prion-associated diseases, multiple sclerosis, capillary expansion, Batten disease, corticobasal degeneration, subacute combined degeneration of the spinal cord, Tabes dorsalis, Tay-Sachs disease, toxic encephalopathy, infantile Refsum disease, Refsum disease, neuroacanthocytosis, Niemann-Pick disease, Lyme disease, Machado-Joseph disease, Sandhoff disease, Shy-Drager syndrome, wobbly hedgehog syndrome, proteopathy, cerebral β-amyloid angiopathy, retinal ganglion cell degeneration in glaucoma, synucleinopathies, tauopathies, frontotemporal lobar degeneration (FTLD), dementia, Cadasil syndrome, hereditary cerebral hemorrhage with amyloidosis, Alexander disease, seipinopathies, familial amyloidotic neuropathy, senile systemic amyloidosis, serpinopathies, AL (light chain) amyloidosis (primary systemic amyloidosis), AH (heavy chain) amyloidosis, AA (secondary) amyloidosis, aortic medial amyloidosis, ApoAI amyloidosis, ApoAII amyloidosis, ApoAIV amyloidosis, familial amyloidosis of the Finnish type (FAF), lysozyme amyloidosis, fibrinogen amyloidosis, dialysis amyloidosis, inclusion body myositis/myopathy, cataract, retinitis pigmentosa with rhodopsin mutation, medullary thyroid carcinoma, cardiac atrial amyloidosis, pituitary prolactinoma, hereditary lattice corneal dystrophy, cutaneous lichen amyloidosis, Mallory bodies, corneal lactoferrin amyloidosis, pulmonary alveolar proteinosis, odontogenic (Pindborg) tumor amyloid, cyst fibrosis, sickle cell disease or critically illness myopathy (CIM), and bone-related disorders.

10. The use according to claim 9, wherein the use is for the preparation of a medication for weight loss.

11. Use of the peptide compound or the pharmaceutically acceptable salt or solvate thereof according to any one of claims 1 to 6 or the pharmaceutical composition according to claim 7 for the preparation of a medication for reducing blood lipid, preferably reducing following blood lipid components: cholesterol, triglycerides, free fatty acids, low-density lipoprotein cholesterol.

12. Use of the peptide compound or the pharmaceutically acceptable salt or solvate thereof according to any one of claims 1 to 6 or the pharmaceutical composition according to claim 7 for the preparation of a medication for reducing blood glucose or treating diabetes.

13. A preparation method of the peptide compound according to any one of claims 1 to 6, wherein the preparation method is chemical synthesis.
